# EUROPEAN PATENT SPECIFICATION

(11) **EP 1 692 109 B1**
(45) Date of publication and mention of the grant of the patent: **18.08.2010**
(21) Application number: 04813392.0
(22) Date of filing: 10.12.2004
(51) Int. Cl.: C07D 213/74, C07D 405/14, C07D 405/12, C07D 277/28, C07D 401/12, A61K 31/506, A61K 31/443, A61K 31/444, A61P 25/00

(54) **PHOSPHODIESTERASE 4 INHIBITORS, INCLUDING N-SUBSTITUTED DIARYLAMINE ANALOGS**
PHOSPHODIESTERASE-4-INHIBITOREN, EINSCHLIESSLICH N-SUBSTITUIERTER DIARYLAMINANALOGA
INHIBITEURS DE LA PHOSPHODIESTERASE 4, NOTAMMENT ANALOGUES DE DIARYLAMINES N-SUBSTITUEES

(30) Priority: 11.12.2003 US 528486 P
(43) Date of publication of application: 23.08.2006
(73) Proprietor: Memory Pharmaceuticals Corporation, Montvale, NJ 07645 (US); F.HOFFMANN-LA ROCHE AG, 4070 Basel (CH)
(72) Inventor: SCHUMACHER, Richard, Monroe, NY 10950 (US); HOPPER, Allen, Glen Rock, NJ 07452 (US); DUNN, Robert, Towaco, NJ 07082 (US); KUESTER, Erik, Maine 55901 (US); TEHIM, Ashok, Ridgewood, NJ 07450 (US); RENAU, Thomas, E., San Carlos, CA 94070 (US); CAROON, Joan, Mountain View, CA 94043 (US); TALAMAS, Francisco, Mountain View, CA 94040 (US); LABADIE, Sharada, Sunnyvale, CA 94087 (US)
(74) Representative: Jungblut, Bernhard Jakob
(86) International application number: PCT/US2004/041068
(87) International publication number: WO 2005/061458

(56) References cited:
- WO-A-95/20578
- WO-A-02/074726
- GB-A- 1 337 389
- US-A- 3 259 623
- US-A- 6 107 301
- DATABASE BEILSTEIN BEILSTEIN CROSSFIRE INSTITUT ZUR FOERDERUNG DER CHEMISCHEN WISSENSCHAFTEN; BRN 9244105 2003, XP002326038 & CHAMBERS R D ET AL: COLLECTION OF CZECHOSLOVAK CHEMICAL COMMUNICATIONS, ACADEMIC PRESS, LONDON, GB, vol. 67, no. 9, 2002, pages 1277-1284, ISSN: 0010-0765
- GERSHON H ET AL: "PYRIMIDINES. 7. A STUDY OF THE CHLORINATION OF PYRIMIDINES WITH PHOSPHORUS OXYCHLORIDE IN THE PRESENCE OF N,N-DIMETHYLANILINE" JOURNAL OF HETEROCYCLIC CHEMISTRY, HETEROCORPORATION. PROVO, US, vol. 21, no. 4, July 1984 (1984-07), pages 1161-1167, XP001068813 ISSN: 0022-152X

## Description

### Field of the Invention

The present invention relates generally to the field of phosphodiesterase 4 (PDE4) enzyme inhibition. More specifically, this invention relates to selective PDE4 inhibition by novel compounds, e.g., *N*-substituted diarylamine analogs, methods of preparing such compounds, compositions containing such compounds, and methods of use thereof.

### Background of the Invention

The cyclic nucleotide specific phosphodiesterases (PDEs) represent a family of enzymes that catalyze the hydrolysis of various cyclic nucleoside monophosphates (including cAMP and cGMP). These cyclic nucleotides act as second messengers within cells, and as messengers, carry impulses from cell surface receptors having bound various hormones and neurotransmitters. PDEs act to regulate the level of cyclic nucleotides within cells and maintain cyclic nucleotide homeostasis by degrading such cyclic mononucleotides resulting in termination of their messenger role.

PDE enzymes can be grouped into eleven families according to their specificity toward hydrolysis of cAMP or cGMP, their sensitivity to regulation by calcium, calmodulin or cGMP, and their selective inhibition by various compounds. For example, PDE1 is stimulated by Ca²⁺/calmodulin. PDE2 is cGMP-dependent, and is found in the heart and adrenals. PDE3 is cGMP-dependent, and inhibition of this enzyme creates positive inotropic activity. PDE4 is cAMP specific, and its inhibition causes airway relaxation, anti-inflammatory, enhanced cognition, and antidepressant activity. PDE5 appears to be important in regulating cGMP content in vascular smooth muscle, and therefore PDE5 inhibitors may have cardiovascular activity. Since the PDEs possess distinct biochemical properties, it is likely that they are subject to a variety of different forms of regulation.

PDE4 is distinguished by various kinetic properties including low Michaelis constant for cAMP and sensitivity to certain drugs. The PDE4 enzyme family consists of four genes, which produce 4 isoforms of the PDE4 enzyme designated PDE4A, PDE4B, PDE4C, and PDE4D [See: Wang et al., Expression, Purification, and Characterization of human cAMP-Specific Phosphodiesterase (PDE4) Subtypes A, B, C, and D, Biochem. Biophys. Res. Comm., 234, 320-324 (1997)]. In addition, various splice variants of each PDE4 isoform have been identified.

PDE4 isoenzymes are localized in the cytosol of cells and specifically inactivate cAMP by catalyzing its hydrolysis to adenosine 5'-monophosphate (AMP). Regulation of cAMP activity is important in many biological processes, including inflammation and memory. Inhibitors of PDE4 isoenzymes such as rolipram, piclamilast, CDP-840 and ariflo are powerful antiinflummatory agents and therefore may be useful in treating diseases where inflammation is problematic such as asthma or arthritis. Further, rolipram improves the cognitive performance of rats and mice in learning paradigms.

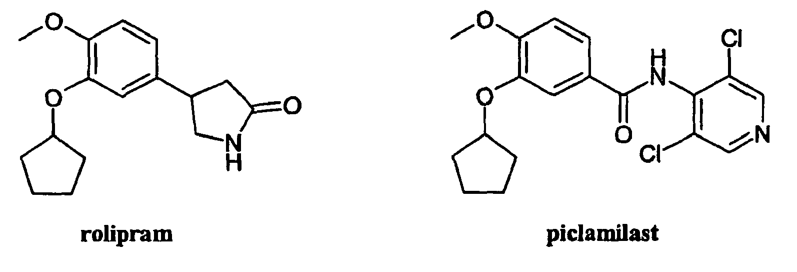

In addition to such compounds as rolipram, xanthine derivatives such as pentoxifylline, denbufylline, and theophylline inhibit PDE4 and have received considerable attention of late for their cognition enhancing effects. cAMP and cGMP are second messengers that mediate cellular responses to many different hormones and neurotransmitters. Thus, therapeutically significant effects may result from PDE inhibition and the resulting increase in intracellular cAMP or cGMP in key cells, such as those located in the nervous system and elsewhere in the body.

Rolipram, previously in development as an anti-depressant, selectively inhibits the PDE4 enzyme and has become a standard agent in the classification of PDE enzyme subtypes. Early work in the PDE4 field focused on depression and inflammation, and has subsequently been extended to include indications such as dementia. [see "The PDE IV Family Of Calcium-Phosphodiesterases Enzymes," John A. Lowe, III, et al., Drugs of the Future 1992, 17(9):799-807 for a general review]. Further clinical developments of rolipram and other first-generation PDE4 inhibitors were terminated due to the side effect profile of these compounds. The primary side effect in primates is emesis, while the primary side effects in rodents are testicular degranulation, weakening of vascular smooth muscle, psychotrophic effects, increased gastric acid secretion and stomach erosion.

WO 95/20578 and WO 02/074726 both describe Phosphodiesterase inhibitors which are, however, structurally different from the compounds according to the present invention.

### Summary of the Invention

The present invention relates to novel compounds, e.g., novel *N*-substituted diarylamine compounds, that inhibit PDE4 enzymes, and especially have improved side effect profiles, e.g., are relatively non-emetic, (e.g., as compared to the previously discussed prior art compounds). Preferably, the compounds selectively inhibit PDE4 enzymes. The compounds of this invention at the same time facilitate entry into cells, especially cells of the nervous system.

Still further, the present invention provides methods for synthesizing compounds with such activity and selectivity as well as methods of (and corresponding pharmaceutical compositions for) treating a patient, e.g., mammals, including humans, requiring PDE inhibition, especially PDE4 inhibition, for a disease state that involves elevated intracellular PDE4 levels or decreased cAMP levels, e.g, involving neurological syndromes, especially those states associated with memory impairment, most especially long term memory impairment, as where such memory impairment is due in part to catabolism of intracellular cAMP levels by PDE4 enzymes, or where such memory impairment may be improved by effectively inhibiting PDE4 enzyme activity.

In a preferred aspect, the compounds of the invention improve such diseases by inhibiting PDE4 enzymes at doses which do not induce emesis.

The present invention includes compounds of Formula I: 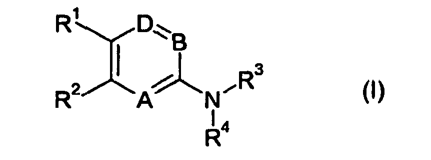 wherein
- A, B and D: are each N or CR⁵ wherein one of A, B and D is N;
- R¹: is halogen, alkyl having 1 to 4 carbon atoms (e.g., methyl, ethyl), halogenated alkyl having 1 to 4 carbon atoms (e.g., CH₂F, CHF₂, CF₃), OR⁶, COR⁶, CONR⁶, or NR⁶COR¹⁰;
- R²: is halogen, alkyl having 1 to 4 carbon atoms, halogenated alkyl having 1 to 4 carbon atoms, OR⁷, COR⁶, CONR⁶, or NR⁶COR¹⁰;
- R³: is alkyl having 1 to 8 which is branched or unbranched and which is unsubstituted or substituted one or more times by halogen, cyano, C₁₋₄-alkoxy, or combinations thereof, a partially unsaturated carbocycle-alkyl group wherein the carbocyclic portion has 5 to 14 carbon atoms and the alkyl portion which is branched or unbranched has 1 to 5 carbon atoms, and which is unsubstituted, substituted in the carbocyclic portion one or more times by halogen, alkyl, alkoxy, nitro, cyano, oxo, or combinations thereof, and/or substituted in the alkyl portion one or more times by halogen, C₁-₄-alkoxy, cyano or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, or heterocycle-alkyl group, wherein the heterocycle portion is unsaturated, partially saturated or fully saturated and has 5 to 10 ring atoms in which at least 1 ring atom is an N, N-O, O or S, the alkyl portion, which is branched or unbranched, has 1 to 5 carbon atoms, the heterocycle-alkyl group is unsubstituted, substituted one or more times in the heterocycle portion by halogen, alkyl, alkoxy, cyano, trifluoromethyl, CF₃O, nitro, oxo, amino, alkylamino, dialkylamino, or combinations thereof and/or substituted in the alkyl portion one or more times by halogen, cyano, alkyl having 1 to 4 carbon atoms, or combinations thereof;
- R⁴: is cycloalkyl having 3 to 10 carbon atoms, which is unsubstituted or substituted one or more times by halogen, hydroxy, oxo, cyano, alkyl having 1 to 4 carbon atoms, alkoxy having 1 to 4 carbon atoms, or combinations thereof, aryl having 6 to 14 carbon atoms and which is unsubstituted or substituted one or more times by halogen, alkyl, alkenyl, alkynyl, hydroxy, alkoxy, alkoxyalkoxy, nitro, methylenedioxy, ethylenedioxy, trifluoromethyl, OCF₃, amino, aminoalkyl, aminoalkoxy, dialkylamino, hydroxyalkyl, hydroxamic acid, pyrrolyl, tetrazole-5-yl, 2(-heterocycle)tetrazole-5-yl, hydroxyalkoxy, carboxy, carboxyalkyl, alkoxycarbonyl, cyano, acyl, alkylthio, alkylsulfinyl, alkylsulfonyl, phenoxy, trialkylsilyloxy, R⁸-L-, or combinations thereof, heteroaryl having 5 to 10 ring atoms in which at least 1 ring atom is a heteroatom, which is unsubstituted or substituted one, two or three times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, carboxyalkyl, alkoxycarbonyl, cyano, acyl, alkylthio, alkylsulfinyl, alkylsulfonyl, phenoxy, trialkylsilyloxy, R⁸-L-, or combinations thereof, a heterocyclic group, which is saturated or partially saturated, having 5 to 10 ring atoms in which at least 1 ring atom is an N, O or S atom, which is unsubstituted or substituted one or more times by halogen, alkyl, hydroxy, alkoxy, alkoxyalkoxy, nitro, oxo, methylenedioxy, ethylenedioxy, trifluoromethyl, OCF₃,amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenylsulfonyl, phenoxy, cycloalkyl, aryl, heteroaryl or combinations thereof, a heterocycle-alkyl group, wherein the heterocyclic portion is saturated, partially saturated or unsaturated, and has 5 to 10 ring atoms in which at least 1 ring atom is an N, O or S atom, and the alkyl portion is branched or unbranched and has 1 to 5 carbon atoms, the heterocycle-alkyl group is unsubstituted, substituted one or more times in the heterocyclic portion by halogen, alkyl, hydroxy, alkoxy, alkoxyalkoxy, nitro, oxo, methylenedioxy, ethylenedioxy, trifluoromethyl, OCF₃, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenylsulfonyl, phenoxy, cycloalkyl, aryl, heteroaryl or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH;
- R⁵: is H, halogen, alkyl having 1 to 4 carbon atoms, halogenated alkyl having 1 to 4 carbon atoms, alkoxy having 1 to 4 carbon atoms, or halogenated alkoxy having 1 to 4 carbon atoms;
- R⁶: is H or alkyl having 1 to 4 carbon atoms, which is branched or unbranched and which is unsubstituted or substituted one or more times by halogen;
- R⁷: is H or alkyl having 1 to 12 which is branched or unbranched and which is unsubstituted or substituted one or more times by halogen, hydroxy, cyano, C₁-₄-alkoxy, oxo or combinations thereof, and wherein optionally one or more -CH₂CH₂- groups is replaced in each case by -CH=CH- or -C≡C-, cycloalkyl having 3 to 10 which is unsubstituted or substituted one or more times by halogen, hydroxy, oxo, cyano, alkyl having 1 to 4 carbon atoms, alkoxy having 1 to 4 carbon atoms, or combinations thereof, cycloalkylalkyl having 4 to 16 which is unsubstituted or substituted in the cycloalkyl portion and/or the alkyl portion one or more times by halogen, oxo, cyano, hydroxy, C₁-₄-alkyl, C₁-₄- alkoxy or combinations thereof, aryl having 6 to 14 carbon atoms which is unsubstituted or substituted one or more times by halogen, CF₃, OCF₃, alkyl, hydroxy, alkoxy, nitro, methylenedioxy, ethylenedioxy, cyano, or combinations thereof, arylalkyl in which the aryl portion has 6 to 14 carbon atoms and the alkyl portion, which is branched or unbranched, has 1 to 5 carbon atoms, wherein the arylalkyl radical is unsubstituted, substituted in the aryl portion one or more times by halogen, CF₃, OCF₃, alkyl, hydroxy, alkoxy, nitro, cyano, methylenedioxy, ethylenedioxy, or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-, a partially unsaturated carbocyclic group having 5 to 14 carbon atoms, which is unsubstituted or substituted one or more times by halogen, alkyl, alkoxy, hydroxy, nitro, cyano, oxo, or combinations thereof , a heterocyclic group, which is saturated, partially saturated or unsaturated, having 5 to 10 ring atoms in which at least 1 ring atom is an N, O or S atom, which is unsubstituted or substituted one or more times by halogen, hydroxy, aryl, alkyl, alkoxy, cyano, trifluoromethyl, nitro, oxo, or combinations thereof, or a heterocycle-alkyl group, wherein the heterocyclic portion is saturated, partially saturated or unsaturated, and has 5 to 10 ring atoms in which at least 1 ring atom is an N, O or S atom, and the alkyl portion is branched or unbranched and has 1 to 5 carbon atoms, the heterocycle-alkyl group is unsubstituted, substituted one or more times in the heterocyclic portion by halogen, OCF₃, hydroxy, aryl, alkyl, alkoxy, cyano, trifluoromethyl, nitro, oxo, or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-;
- R⁸: is H, alkyl having 1 to 8 which is unsubstituted or substituted one or more times by halogen, C₁-₄- alkyl, C₁-₄-alkoxy, oxo, or combinations thereof, alkylamino or dialkylamino wherein each alkyl portion has independently 1 to 8, a partially unsaturated carbocycle-alkyl group wherein the carbocyclic portion has 5 to 14 carbon atoms and the alkyl portion has 1 to 5 carbon atoms, and which is unsubstituted or substituted one or more times by halogen, alkyl, alkoxy, nitro, cyano, oxo, or combinations thereof, cycloalkyl having 3 to 10 which is unsubstituted or substituted one or more times by halogen, hydroxy, oxo, cyano, alkoxy, alkyl having 1 to 4 carbon atoms, or combinations thereof, cycloalkylalkyl having 4 to 16 which is unsubstituted or substituted in the cycloalkyl portion and/or the alkyl portion one or more times by halogen, oxo, cyano, hydroxy, alkyl, alkoxy or combinations thereof, aryl having 6 to 14 carbon atoms which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, cycloalkyl, aryl, heteroaryl or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, a heterocyclic group, which is saturated, partially saturated or unsaturated, having 5 to 10 ring atoms in which at least 1 ring atom is an N, O or S atom, which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, cycloalkyl, aryl, heteroaryl or combinations thereof, or a heterocycle-alkyl group, wherein the heterocyclic portion is saturated, partially saturated or unsaturated, and has 5 to 10 ring atoms in which at least 1 ring atom is an N, O or S atom, and the alkyl portion, which is branched or unbranched, has 1 to 5 carbon atoms, the heterocycle-alkyl group is unsubstituted, substituted one or more times in the heterocyclic portion by halogen, alkyl, alkoxy, cyano, trifluoromethyl, CF₃O, nitro, oxo, amino, alkylamino, dialkylamino, or combinations thereof and/or substituted one or more times in the alkyl portion by halogen, cyano, alkyl having 1 to 4 carbon atoms, or combinations thereof;
- L: is a single bond or a divalent aliphatic radical having 1 to 8 carbon atoms wherein one or more - CH₂- groups are each optionally replaced by -O-, - S-, -SO-, -SO₂-, -NR⁹-, -SO₂NR⁹-, -NR⁹SO₂-, -CO-, -CO₂-, -NR⁹CO-, - CONR⁹-, -NHCONH-, -OCONH, -NHCOO-, -SCONH-, - SCSNH-, -NHCSNH-, -CONHSO₂- or -SO₂NHCO-; and
- R⁹: is H, alkyl having 1 to 8 carbon atoms, which is branched or unbranched and which is unsubstituted or substituted one or more times with halogen, C₁- ₄-alkyl, C₁-₄-alkoxy, oxo, or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, or aryl having 6 to 14 carbon atoms and which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, or combinations thereof; and
- R¹⁰: is H or alkyl having 1 to 4 carbon atoms, which is branched or unbranched and which is unsubstituted or substituted one or more times by halogen; or
a pharmaceutically acceptable salt thereof.

The present invention further includes compounds of Formula III: 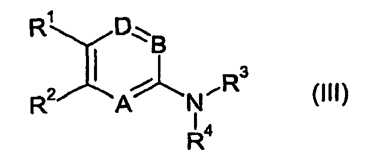 wherein
A, B and D are each N or CR⁵;
wherein
- A, B and D: are each N or CR⁵ and one of A, B and D is N;
- R¹: is halogen, alkyl having 1 to 4 carbon atoms, halogenated alkyl having 1 to 4 carbon atoms, or OR⁶;
- R²: is halogen, alkyl having 1 to 4 carbon atoms, halogenated alkyl having 1 to 4 carbon atoms, or OR⁷;
- R³: is arylalkyl having 7 to 19 carbon atoms wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, or heterocycle-alkyl group, wherein the heterocycle portion is unsaturated, partially saturated or fully saturated and has 5 to 10 ring atoms in which at least 1 ring atom is an N, N-O, O or S, the alkyl portion, which is branched or unbranched, has 1 to 5 carbon atoms, the heterocycle-alkyl group is unsubstituted, substituted one or more times in the heterocycle portion by halogen, alkyl, alkoxy, cyano, trifluoromethyl, CF₃O, nitro, oxo, amino, alkylamino, dialkylamino, or combinations thereof and/or substituted in the alkyl portion one or more times by halogen, cyano, alkyl having 1 to 4 carbon atoms, or combinations thereof;
- R⁴: is cycloalkyl having 3 to 10 carbon atoms which is unsubstituted or substituted one or more times by halogen, hydroxy, oxo, cyano, alkyl having 1 to 4 carbon atoms, alkoxy having 1 to 4 carbon atoms, or combinations thereof, aryl having 6 to 14 carbon atoms and which is unsubstituted or substituted one or more times by halogen, alkyl, alkenyl, alkynyl, hydroxy, alkoxy, alkoxyalkoxy, nitro, methylenedioxy, ethylenedioxy, trifluoromethyl, OCF₃, amino, aminoalkyl, aminoalkoxy, dialkylamino, hydroxyalkyl, hydroxamic acid, pyrrolyl, tetrazole-5-yl, 2(-heterocycle)tetrazole-5-yl, hydroxyalkoxy, carboxy, alkoxycarbonyl, cyano, acyl, alkylthio, alkylsulfinyl, alkylsulfonyl, phenoxy, trialkylsilyloxy, R⁸-L-, or combinations thereof, heteroaryl having 5 to 10 ring atoms in which at least 1 ring atom is a heteroatom, which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, trialkylsilyloxy, R⁸-L-, or combinations thereof, a heterocyclic group, which is saturated or partially saturated, having 5 to 10 ring atoms in which at least 1 ring atom is an N, O or S atom, which is unsubstituted or substituted one or more times by halogen, alkyl, hydroxy, alkoxy, alkoxyalkoxy, nitro, oxo, methylenedioxy, ethylenedioxy, trifluoromethyl, OCF₃,amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenylsulfonyl, phenoxy, cycloalkyl, aryl, heteroaryl or combinations thereof, a heterocycle-alkyl group, wherein the heterocyclic portion is saturated, partially saturated or unsaturated, and has 5 to 10 ring atoms in which at least 1 ring atom is an N, O or S atom, and the alkyl portion is branched or unbranched and has 1 to 5 carbon atoms, the heterocycle-alkyl group is unsubstituted, substituted one or more times in the heterocyclic portion by halogen, alkyl, hydroxy, alkoxy, alkoxyalkoxy, nitro, oxo, methylenedioxy, ethylenedioxy, trifluoromethyl, OCF₃, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenylsulfonyl, phenoxy, cycloalkyl, aryl, heteroaryl or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-;
- R⁵: is H, halogen, alkyl having 1 to 4 carbon atoms, halogenated alkyl having 1 to 4 carbon atoms, alkoxy having 1 to 4 carbon atoms, or halogenated alkoxy having 1 to 4 carbon atoms;
- R⁶: is H or alkyl having 1 to 4 carbon atoms, which is branched or unbranched and which is unsubstituted or substituted one or more times by halogen;
- R⁷: is H or alkyl having 1 to 12 carbon atoms, which is branched or unbranched and which is unsubstituted or substituted one or more times by halogen, hydroxy, cyano, C₁-₄-alkoxy, oxo or combinations thereof, and wherein optionally one or more -CH₂CH₂- groups is replaced in each case by -CH=CH- or -C≡C-, cycloalkyl having 3 to 10 carbon atoms, which is unsubstituted or substituted one or more times by halogen, hydroxy, oxo, cyano, alkyl having 1 to 4 carbon atoms, alkoxy having 1 to 4 carbon atoms, or combinations thereof, cycloalkylalkyl having 4 to 16 carbon atoms, which is unsubstituted or substituted in the cycloalkyl portion and/or the alkyl portion one or more times by halogen, oxo, cyano, hydroxy, C₁-₄-alkyl, C₁-₄- alkoxy or combinations thereof, aryl having 6 to 14 carbon atoms, which is unsubstituted or substituted one or more times by halogen, CF₃, OCF₃, alkyl, hydroxy, alkoxy, nitro, methylenedioxy, ethylenedioxy, cyano, or combinations thereof, arylalkyl in which the aryl portion has 6 to 14 carbon atoms and the alkyl portion, which is branched or unbranched, has 1 to 5 carbon atoms, wherein the arylalkyl radical is unsubstituted, substituted in the aryl portion one or more times by halogen, CF₃, OCF₃, alkyl, hydroxy, alkoxy, nitro, cyano, methylenedioxy, ethylenedioxy, or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-, a partially unsaturated carbocyclic group having 5 to 14 carbon atoms, which is unsubstituted or substituted one or more times by halogen, alkyl, alkoxy, hydroxy, nitro, cyano, oxo, or combinations thereof, a heterocyclic group, which is saturated, partially saturated or unsaturated, having 5 to 10 ring atoms in which at least 1 ring atom is an N, O or S atom, which is unsubstituted or substituted one or more times by halogen, hydroxy, aryl, alkyl, alkoxy, cyano, trifluoromethyl, nitro, oxo, or combinations thereof, or a heterocycle-alkyl group, wherein the heterocyclic portion is saturated, partially saturated or unsaturated, and has 5 to 10 ring atoms in which at least 1 ring atom is an N, O or S atom, and the alkyl portion is branched or unbranched and has 1 to 5 carbon atoms, the heterocycle-alkyl group is unsubstituted, substituted one or more times in the heterocyclic portion by halogen, OCF₃, hydroxy, aryl, alkyl, alkoxy, cyano, trifluoromethyl, nitro, oxo, or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-;
- R⁸: is H, alkyl having 1 to 8 carbon atoms, which is unsubstituted or substituted one or more times by halogen, C₁-₄-alkyl, C₁-₄-alkoxy, oxo, or combinations thereof, alkylamino or dialkylamino wherein each alkyl portion has independently 1 to 8 carbon atoms, a partially unsaturated carbocycle-alkyl group wherein the carbocyclic portion has 5 to 14 carbon atoms and the alkyl portion has 1 to 5 carbon atoms, and which is unsubstituted or substituted one or more times by halogen, alkyl, alkoxy, nitro, cyano, oxo, or combinations thereof, cycloalkyl having 3 to 10 carbon atoms, which is unsubstituted or substituted one or more times by halogen, hydroxy, oxo, cyano, alkoxy, alkyl having 1 to 4 carbon atoms, or combinations thereof, cycloalkylalkyl having 4 to 16 carbon atoms, which is unsubstituted or substituted in the cycloalkyl portion and/or the alkyl portion one or more times by halogen, oxo, cyano, hydroxy, alkyl, alkoxy or combinations thereof, aryl having 6 to 14 carbon atoms which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, cycloalkyl, aryl, heteroaryl or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, a heterocyclic group, which is saturated, partially saturated or unsaturated, having 5 to 10 ring atoms in which at least 1 ring atom is an N, O or S atom, which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, cycloalkyl, aryl, heteroaryl or combinations thereof, or a heterocycle-alkyl group, wherein the heterocyclic portion is saturated, partially saturated or unsaturated, and has 5 to 10 ring atoms in which at least 1 ring atom is an N, O or S atom, and the alkyl portion, which is branched or unbranched, has 1 to 5 carbon atoms, the heterocycle-alkyl group is unsubstituted, substituted one or more times in the heterocyclic portion by halogen, alkyl, alkoxy, cyano, trifluoromethyl, CF₃O, nitro, oxo, amino, alkylamino, dialkylamino, or combinations thereof and/or substituted one or more times in the alkyl portion by halogen, cyano, alkyl having 1 to 4 carbon atoms, or combinations thereof;
- L: is a single bond or a divalent aliphatic radical having 1 to 8 carbon atoms wherein one or more - CH₂- groups are each optionally replaced by -0-, - S-, -SO-, -SO₂-, -NR⁹-, -SO₂NR⁹-, -NR⁹SO₂-, - CO-, -CO₂-, -NR⁹CO-, -CONR⁹-, -NHCONH-, -OCONH, - NHCOO-, -SCONH-, -SCSNH-, -NHCSNH-, -CONHSO₂- or -SO₂NHCO-; and
- R⁹: is H, alkyl having 1 to 8 carbon atoms, which is branched or unbranched and which is unsubstituted or substituted one or more times with halogen, C₁- ₄-alkyl, C₁-₄-alkoxy, oxo, or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, or aryl having 6 to 14 carbon atoms and which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, or combination thereof ; and
wherein R¹ is OR⁶ and/or R² is OR⁷ ;
or a pharmaceutically acceptable salt thereof.

In Formulas I and III, R¹ is preferably halogen (e.g., F) or is preferably OR⁶, e.g., wherein R⁶ is alkyl (e.g., methyl), or halogenated alkyl (e.g., CHF₂), or.

In Formulas I and III, R² is preferably halogen (such as F or Cl) or OR⁷, e.g., wherein R⁷ is alkyl (such as methyl, ethyl, isopropyl), cycloalkyl (such as cyclobutyl or cyclopentyl), cycloalkylalkyl (such as cyclopropylmethyl), a heterocyclic group (such as tetrahydrofuranyl), or halogenated alkyl (e.g., CHF₂).

In Formulas I and III, R³ is preferably arylalkyl, especially benzyl, or heteroarylalkyl, especially pyridylmethyl, thiazolylmethyl or pyrimidinylmethyl, which in each case is substituted or unsubstituted. For example, R³ can be benzyl or pyridylmethyl, which in each case is substituted or unsubstituted.

In Formula I, R⁴ is preferably cycloalkyl, aryl, heteroaryl or a heterocyclic group, which is substituted or unsubstituted, particularly cyclohexyl, piperidinyl, or phenyl, especially phenyl, in each case substituted or unsubstituted. When R⁴ is phenyl, the preferred substituents are halogen, carboxy, cyano, tetrazole, and/or L-R⁸.

In Formula I, R⁴ is also preferably cycloalkyl, aryl, or a heterocyclic group, which is substituted or unsubstituted, particularly cyclohexyl, piperidinyl, or phenyl, especially phenyl, in each case substituted or unsubstituted. When R⁴ is phenyl, the preferred substituents are carboxy, cyano, tetrazole, and/or L-R⁸.

According to further embodiments of Formulas I and III, Mil, R⁴ is at least monosubstituted by R⁸-L- in which L is a single bond or a divalent aliphatic radical having 1 to 8 carbon atoms wherein at least one -CH₂- group is replaced by -SO₂NR⁹, -NR⁹-, - NR⁹CO-, - CONR⁹-, -CO₂-, -CONHSO₂-, -SO₂NHCO-, -SO₂-, or -NR⁹SO₂- (e.g., the replacement may result in the divalent radical having no carbon atoms, i.e., where it is a single -CH₂- group which is replaced by - SO₂NR⁹ or -NR⁹O₂-).

In Formulas I and III, R⁸ is preferably methyl, ethyl, propyl or phenyl, which in each case is unsubstituted or substituted.

In another embodiment, in Formulas I and III, R⁹ is H, alkyl having 1 to 4 carbon atoms, or aryl.

In a further embodiment, in Formulas I and III, R⁵ is preferably H, F or methyl.

According to a further aspect of the invention, in Formula I, R¹ is COR⁶, CONR⁶or NR⁶COR¹⁰.

According to a further aspect of the invention, in Formula I, R² is COR⁶, CONR⁶ or NR⁶COR¹⁰.

According to further aspects, the compounds of Formula I or Formula III are in accordance with the following subformula:

| | |
|---|---|
| Ia, IIIa | A is N, |
| | B and D are each independently CR⁵, |
| | R¹ is OR⁶, |
| | R² is halogen or OR⁷, |
| | R³ is pyridylmethyl, fluorobenzyl, or 2,6-difluorobenzyl, |
| | R⁴ is aryl, cycloalkyl, or a saturated heterocyclic group, in each case substituted or unsubstituted, |
| | R⁵ is H, halogen, or alkyl which is substituted or unsubstituted, |
| | R⁶ is alkyl which is substituted or unsubstituted, and |
| | R⁷ is alkyl, cycloalkyl, cycloalkylalkyl or a saturated heterocyclic group, |
| | in each case substituted or unsubstituted. |
| Ib,IIIb | A is N, |
| | B and D are each independently CR⁵, |
| | R¹ is OR⁶, |
| | R² is halogen or OR⁷, |
| | R³ is pyridylmethyl, fluorobenzyl, or 2,6-difluorobenzyl, |
| | R⁴ is phenyl, which is unsubstituted or substituted, e.g., substituted by carboxy, cyano, tetrazole, and/or L-R⁸, |
| | R⁵ is H, halogen, or alkyl which is substituted or unsubstituted (e.g., CH₃), |
| | R⁶ is alkyl which is substituted or unsubstituted, and |
| | R⁷ is alkyl, cycloalkyl, cycloalkylalkyl, or a saturated heterocyclic group, |
| | in each case substituted or unsubstituted. |
| Ic, IIIc | A is N, |
| | B and D are each independently CR⁵, |
| | R¹ is OR⁶, |
| | R² is halogen or OR⁷, |
| | R³ is pyridylmethyl, fluorobenzyl, 2,6-difluorobenzyl, 5-thiazolylmethyl, or 5-pyrimidinylmethyl, |
| | R⁴ is phenyl, which is unsubstituted or substituted, e.g., substituted by carboxy, cyano, tetrazole, and/or L-R⁸, |
| | R⁵ is H, halogen, or alkyl which is substituted or unsubstituted (e.g., CH₃), |
| | R⁶ is alkyl which is substituted or unsubstituted, and |
| | R⁷ is alkyl, cycloalkyl, cycloalkylalkyl, or a saturated heterocyclic group, in each case substituted or unsubstituted. |
| Id, IIId | A is N, |
| | B and D are each independently CH, |
| | R¹ is OR⁶, |
| | R² is halogen or OR⁷, |
| | R³ is pyridylmethyl, |
| | R⁴ is unsubstituted cycloalkyl, aryl which is substituted or unsubstituted, or piperidinyl which is substituted or unsubstituted, |
| | R⁶ is unsubstituted alkyl or CHF₂, and |
| | R⁷ is alkyl, cycloalkyl, cycloalkylalkyl or tetrahydrofuranyl, in each case substituted or unsubstituted. |
| Ie, IIIe | A is N, |
| | B and D are each independently CH, |
| | R¹ is OR⁶, |
| | R² is halogen or OR⁷, |
| | R³ is pyridylmethyl, 5-thiazolylmethyl, or 5-pyrimidinylmethyl, |
| | R⁴ is unsubstituted cycloalkyl, aryl which is substituted or unsubstituted, or piperidinyl which is substituted or unsubstituted, |
| | R⁶ is unsubstituted alkyl or CHF₂, and |
| | R⁷ is alkyl, cycloalkyl, cycloalkylalkyl or tetrahydrofuranyl, in each case substituted or unsubstituted. |
| If, IIIf | A is N, |
| | B and D are each independently CH, |
| | R¹ is OR⁶, |
| | R² is halogen or OR⁷, |
| | R³ is 3-pyridylmethyl, |
| | R⁴ is cyclohexyl, phenyl which is substituted or unsubstituted, or piperidinyl which is substituted or unsubstituted, |
| | R⁶ is unsubstituted alkyl (e.g. CH₃) or CHF₂, and |
| | R⁷ is alkyl, cycloalkyl, cycloalkylalkyl or tetrahydrofuranyl, in each case substituted or unsubstituted. |
| Ig, IIIg | A is N, |
| | B and D are each independently CH, |
| | R¹ is OR⁶, |
| | R² is halogen or OR⁷, |
| | R³ is 3-pyridylmethyl, 5-thiazolylmethyl, or 5 pyrimidinylmethyl, |
| | R⁴ is cyclohexyl, phenyl which is substituted or unsubstituted, or piperidinyl which is substituted or unsubstituted, |
| | R⁶ is unsubstituted alkyl (e.g. CH₃) or CHF₂, and |
| | R⁷ is alkyl, cycloalkyl, cycloalkylalkyl or tetrahydrofuranyl, in each case substituted or unsubstituted. |
| Ih, IIIh | A is N and B and D are each independently CR⁵, |
| | R¹ is OR⁶, |
| | R² is OR⁷, |
| | R³ is heteroarylalkyl (e.g., pyridylmethyl), |
| | R⁴ is heterocyclic group (e.g. piperidinyl), which is unsubstituted or substituted, e.g., substituted by alkyl, alkylsulphonyl, and/or acyl such as unsubstituted or halogen substituted benzoyl, |
| | R⁵ is H, halogen, or alkyl which is substituted or unsubstituted (e.g., CH₃), |
| | R⁶ is alkyl, |
| | R⁷ is alkyl, cycloalkyl, or cycloalkylalkyl, in each case substituted or unsubstituted. |

In accordance with a further aspect, the compounds of the invention include the following compounds:
4-{*N*-[4-Methoxy-3-(*R*)-(tetrahydrofuran-3-yloxy)phenyl]pyridin-3-ylmethylamino}piperidine-1-carboxylic acid *tert*-butyl ester,
3-[*N*-(6-Cyclopropylmethoxy-5-methoxypyridin-2-yl)-pyridin-3-ylmethylamino]benzoic acid,
3-[*N*-(5,6-Dimethoxypyridin-2-yl)-pyridin-3-ylmethylamino]-benzoic acid,
3-[*N*-(6-Cyclobutyloxy-5-methoxypyridin-2-yl)-pyridin-3-ylmethylamino]benzoic acid,
3-[*N*-(6-Cyclopropylmethoxy-5-difluoromethoxypyridin-2-yl)-pyridin-3-ylmethylamino]benzoic acid,
3-[*N*-(5-Difluoromethoxy-6-methoxypyridin-2-yl)-pyridin-3-ylmethylamino]benzoic acid,
3-[*N*-(6-Ethoxy-5-methoxypyridin-2-yl)-pyridin-3-ylmethylamino]benzoic acid,
3-[*N*-(6-Isopropoxy-5-methoxypyridin-2-yl)-pyridin-3-ylmethylamino]benzoic acid,
3-[*N*-(5-Difluoromethoxy-6-isopropoxypyridin-2-yl)-pyridin-3-ylmethylamino]benzoic acid,
3-[*N*-(6-Cyclobutyloxy-5-difluoromethoxypyridin-2-yl)-pyridin-3-ylmethylamino]benzoic acid,
*N*-(1-Benzenesulfonylpiperidin-3-yl)-*N*-[5-methoxy-6-(*R*)-(tetrahydrofuran-3-yloxy)pyridin-2-yl]-pyridin-3-ylmethylamine,
*N*-(1-Methanesulfonylpiperidin-3-yl)-*N*-[5-methoxy-6-(R)-(tetrahydrofuran-3-yloxy)-pyridin-2-yl]-pyridin-3-ylmethylamine,
*N*-[5-Methoxy-6-(*R*)-(tetrahydrofuran-3-yloxy)-pyridin-2-yl]-*N*-piperidin-3-yl-pyridin-3-ylmethylamine,
*N*-[5-Methoxy-6-(*R*)-(tetrahydrofuran-3-yloxy)-pyridin-2-yl]-*N*-piperidin-4-ylmethyl-pyridin-3-ylmethylamine, 4-(*N*-{[5-Methoxy-6-(*R*)-(tetrahydrofuran-3-yloxy)-pyridin-2-yl]-pyridin-3-ylmethylamino}-methyl)-*N*-piperidine-1-carboxylic acid *tert*-butyl ester,
*N*-(1-Benzenesulfonylpiperidin-4-yl)-*N*-[5-methoxy-6-(*R*)-(tetrahydrofuran-3-yloxy)-pyridin-2-yl]-pyridin-3-ylmethylamine,
1-(4-{*N*-[5-Methoxy-6-(*R*)-(tetrahydrofuran-3-yloxy)-pyridin-2-yl]-pyridin-3-ylmethylamino}-*N*-piperidin-1-yl)ethanone,
*N*-[5-Methoxy-6-(*R*)-(tetrahydrofuran-3-yloxy)-pyridin-2-yl]-piperidin-4-yl-pyridin-3-ylmethylamine,
9-{*N*-[5-Methoxy-6-(*R*)-(tetrahydrofuran-3-yloxy)-pyridin-2-yl]-pyridin-3-ylmethylamino}-piperidine-1-carboxylic acid *tert*-butyl ester,
3-{*N*-[5-Methoxy-6-(*R*)-(tetrahydrofuran-3-yloxy)-pyridin-2-yl]-pyridin-3-ylmethylamino}-benzoic acid,
*N*-[5-Methoxy-6-(*R*)-(tetrahydrofuran-3-yloxy)-pyridin-2-yl]-pyridin-3-ylmethyl-N-[4-(2H-tetrazol-5-yl)phenyl]amine,
*N*-Cyclohexyl-*N*-[5-methoxy-6-(*R*)-(tetrahydrofuran-3-yloxy)-pyridin-2-yl]-pyridin-3-ylmethylamine,
*N*-[5-Methoxy-6-(*R*)-(tetrahydrofuran-3-yloxy)-pyridin-2-yl]-*N*-phenyl-pyridin-3-ylmethylamine,
*N*-(3-Chlorophenyl)-*N*-[5-methoxy-6-(*R*)-(tetrahydrofuran-3-yloxy)pyridin-2-yl]-pyridin-3-ylmethylamine,
3-{*N*-[5-Methoxy-6-(tetrahydrofuran-3-yloxy)-pyridin-2-yl]pyridin-3-ylmethylamino}benzoic acid,
3-[*N*-(6-Cyclopentyloxy-5-methoxypyridin-2-yl)-pyridin-3-ylmethylamino]benzoic acid,
4-[*N*-(5-Cyclopentyloxy-4-methoxy-2-methylphenyl)pyridin-3-ylmethylamino]benzoic acid,
3-[*N*-(5-Cyclopentyloxy-4-methoxy-2-methylphenyl)pyridin-3-ylmethylamino]benzoic acid, and
pharmaceutically acceptable salts thereof,
wherein compounds that are optically active can be in the form of their separate enantiomers or mixtures thereof, including racemic mixtures.

In accordance with a further aspect, the compounds of the invention include the following compounds:
(6-Cyclopentyloxy-5-methoxy-pyridin-2-yl)-piperidin-4-yl-pyridin-3-ylmethyl-amine hydrochloride,
(6-Cyclopentyloxy-5-methoxy-pyridin-2-yl)-piperidin-4-yl-pyridin-4-ylmethyl-amine,
(6-Cyclopropylmethoxy-5-methoxy-pyridin-2-yl)-piperidin-4-yl-pyridin-3-ylmethyl-amine,
{4-[(6-Cyclopentyloxy-5-methoxy-pyridin-2-yl)-pyridin-3-ylmethyl-amino]-piperidin-1-yl}-(4-fluoro-phenyl)-methanone,
(6-Cyclopentyloxy-5-methoxy-pyridin-2-yl)-(1-methanesulfonyl-piperidin-4-yl)-pyridin-3-ylmethyl-amine,
6-(cyclopentyloxy)-5-methoxy-N-piperidin-3-yl-N-(pyridin-3-ylmethyl)pyridin-2-amine,
6-(cyclopentyloxy)-5-methoxy-N-piperidin-3-yl-N-(pyridin-3-ylmethyl)pyridin-2-amine oxalate,
6-isopropoxy-5-methoxy-N-piperidin-4-yl-N-(pyridin-3-ylmethyl)pyridin-2-amine,
6-isopropoxy-5-methoxy-N-piperidin-4-yl-N-(pyridin-3-ylmethyl)pyridin-2-amine oxalate,
6-(cyclopropylmethoxy)-5-(difluoromethoxy)-N-piperidin-4-yl-N-(pyridin-3-ylmethyl)pyridin-2-amine,
6-(cyclopropylmethoxy)-5-(difluoromethoxy)-N-piperidin-4-yl-N-(pyridin-3-ylmethyl)pyridin-2-amine trifluoroacetate,
6-(cyclopentyloxy)-5-methoxy-N-phenyl-N-piperidin-4-ylpyridin-2-amine,
6-(cyclopentyloxy)-5-methoxy-N-piperidin-4-yl-N-(pyrimidin-5-ylmethyl)pyridin-2-amine,
and
pharmaceutically acceptable salts thereof,
wherein compounds that are optically active can be in the form of their separate enantiomers or mixtures thereof, including racemic mixtures.

The following list presents structure and data for compounds in accordance with the invention: 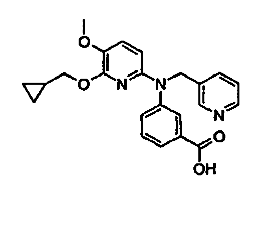
a) 3-[*N*-(6-Cyclogopylmethoxy-5-methoxypyridin-2-yl)-pyridin-3-ylmethylamino]benzoic acid; MS (ES): m/z 406.2 [M + 1] 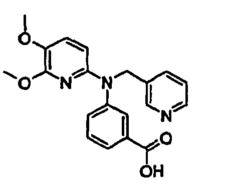
b) 3-[*N*-(5,6-Dmethoxypyridin-2-yl)-pyridin-3-ylmethylamino]benzoic acid; MS (ES): m/z 366.2 [M+1] 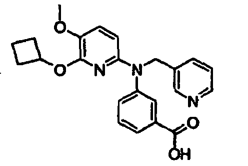
c) 3-[*N-*(6-Cyclobutyloxy-5-methoxypyridin-2-yl)-pyridin-3-ylmethylamino]benzoic acid; MS (ES): m/z 406.2 [M + 1] 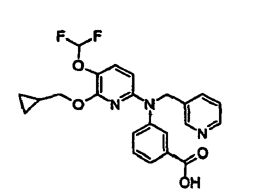
d) 3-[*N*-(6-Cyclopropylmethoxy-5-difluoromethoxypyridin-2-yl)-pyridin-3-ylmethylamino]benzoic acid; MS (ES): m/z 442.1 [M + 1] 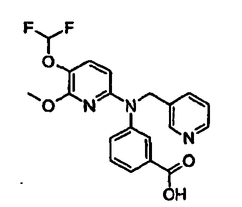
e) 3-[*N*-(5-Difluoromethoxy-6-methoxypyridin-2-yl)-pyridin-3-ylmethylamino]benzoic acid; MS (ES): m/z 402.2 [M + 1] 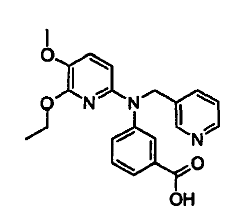
f) 3-[*N*-(6-Ethoxy-5-methoxypyridin-2-yl)-pyridin-3-ylmethylamino]benzoic acid; MS (ES): m/z 380.2 [M + 1] 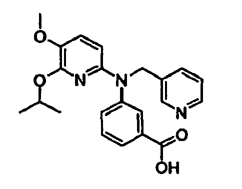
g) 3-[*N*-(6-Isopropoxy-5-methoxypyridin-2-yl)-pyridin-3-ylmethylamino]benzoic acid; MS (ES): m/z 394.2 [M + 1] 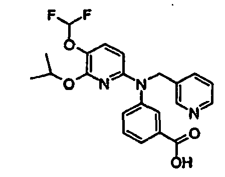
h) 3-[*N*-(5-Difluoromethoxy-6-isopropoxypyridin-2-yl)-pyridin-3-ylmethylamino]benzoic acid; MS (ES): m/z 430.1 [M+ 1] 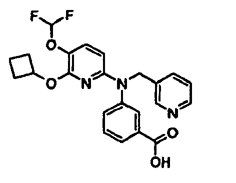
i) 3-[*N*-(6-Cyclobutyloxy-5-difluoromethoxypyridin-2-yl)-pyridin-3-ylmethylamino]benzoic acid; MS (ES): m/z 442.1 [M + 1] 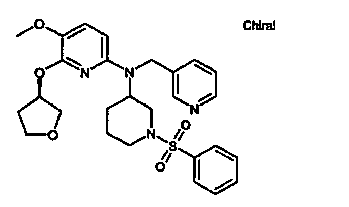 o) *N*-(1-Benzenesulfonylpiperidin-3-yl)-N-[5-methoxy-6-(3*R*)-(tetrahydrofuran-3-yloxy)pyridin-2-yl]-pyridin-3-ylmethylamine; MS (ES): m/z 525 [M + 1] 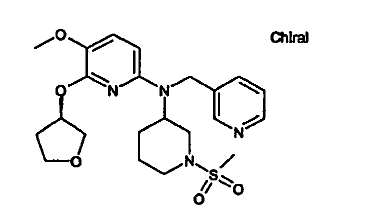
p) *N*-(1-Methanesulfonylpiperidin-3-yl)-*N*-[5-methoxy-6-(3*R*)-(tetrahydrofuran-3-yloxy)-pyridin-2-yl]-pyridin-3-ylmethylamine; MS (ES): m/z 463 [M + 1] 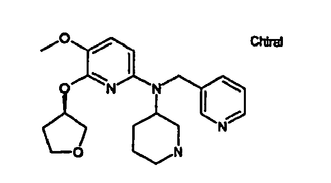
q) *N*-[5-Methoxy-6-(3*R*)-(tetrahydrofuran-3-yloxy)-pyridin-2-yl]-*N*-piperidin-3-yl-pyridin-3-ylmethylamine; MS (ES): m/z 385 [M+ 1] 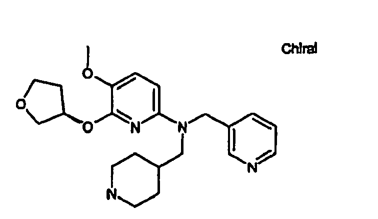
r) *N*-[5-Methoxy-6-(3*R*)-(tetrahydrofuran-3-yloxy)-pyridin-2-yl]-*N*-piperidin-4-ylmethyl-pyridin-3-ylmethylamine; MS (ES): m/z 399 [M + 1] 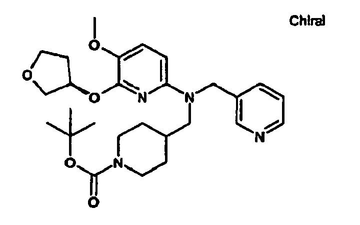
s) 4-(*N*-{[5-Methoxy-6-(3*R*)-(tetrahydrofuran-3-yloxy)-pyridin-2-yl]-pyridin-3-ylmethylamino}-methyl)-*N*-piperidine-1-carboxylic acid *tert*-butyl ester; MS (ES): m/z 499 [M+1] 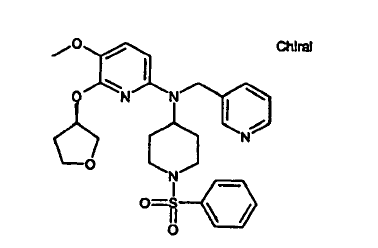
t) *N*-(1-Benzenesulfonylpiperidin-4-yl)-*N*-[5-methoxy-6-(3*R*)-(tetrahydrofuran-3-yloxy)-pyridin-2-yl]-pyridin-3-ylmethylamine; MS (ES): m/z 525 [M + 1] 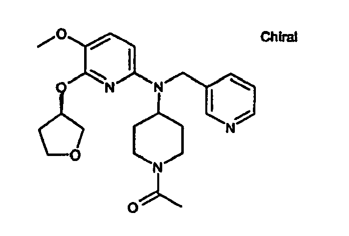
u) 1-(4-{*N*-[5-Methoxy-6-(3*R*)-(tetrahydrofuran-3-yloxy)-pyridin-2-yl]-pyridin-3-ylmethylamino}-*N*-piperidin-1-yl)ethanone; MS (ES): m/z 427 [M + 1] 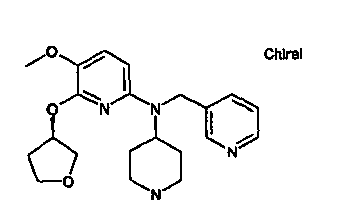
v) *N*-[5-Methoxy-6-(3*R*)-(tetrahydrofuran-3-yloxy)-pyridin-2-yl]-piperidin-4-yl-pyridin-3-ylmethylamine; MS (ES): m/z 385 [M + 1] 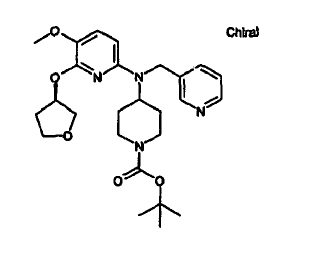
w) 4-{*N*-[5-Methoxy-6-(3*R*)-(tetrahydrofuran-3-yloxy)-pyridin-2-yl]-pyridin-3-ylmethylamino}-piperidine-1-carboxylic acid *tert*-butyl ester; MS (ES): m/z 485 [M + 1] 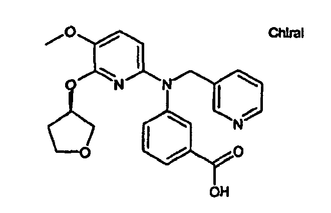
x) 3-{*N*-[5-Methoxy-6-(3*R*)-(tetrahydrofuran-3-yloxy)-pyridin-2-yl]pyridin-3-ylmethylamino}benzoic acid; MS (ES): m/z 422.0 [M + 1] 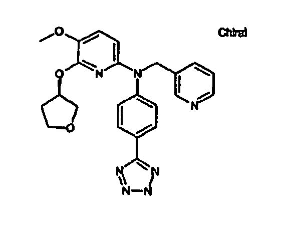
y) *N*-[5-Methoxy-6-(3*R*)-(terahydrofuran-3-yloxy)-pyridin-2-yl]-pyridin-3-ylmethyl-*N*-[4-(2H-tetrazol-5-yl)phenyl]amine; MS (ES): m/z 446 [M + 1] 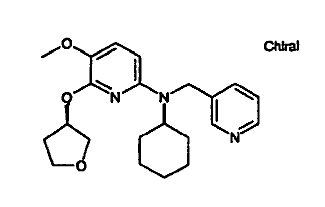
z) *N*-Cyclohexyl-*N*-[5-methoxy-6-(3*R*)-(tetrahydrofuran-3-yloxy)-pyridin-2-yl]pyridin-3-ylmethylamine; MS (ES): m/z 384 [M + 1] 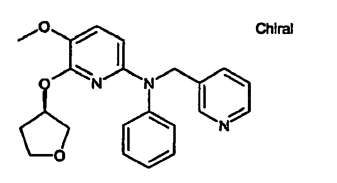
aa) *N*-[5-Methoxy-6-(3*R*)-(tetrahydrofuran-3-yloxy)-pyridin-2-yl]-*N*-phenyl-pyridin-3-ylmethylamine; MS (ES): m/z 378 [M + 1] 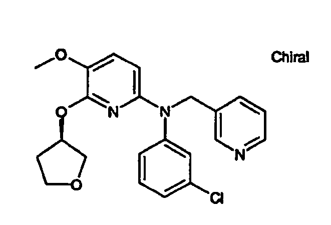
bb) *N*-(3-Chlorophenyl)-*N*-[5-methoxy-6-(3*R*)-(tetrahydrofuran-3-yloxy)pyridin-2-yl]pyridin-3-ylmethylamine; MS (ES): m/z 412,414 [M + 1] 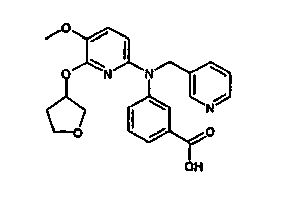 cc) 3-{*N*-[5-Methoxy-6-(tetrahydrofuran-3-yloxy)-pyridin-2-yl]pyridin-3-ylmethylamino}benzoic acid; MS (ES): m/z 422.1 [M + 1] 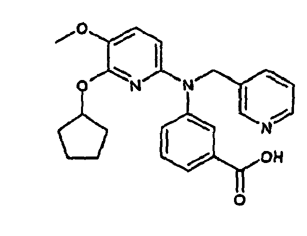
hh) 3-[*N*-(6-Cyclopentyloxy-5-methoxypyridin-2-yl)-pyridin-3-ylmethylamino]benzoic acid; MS (ES): m/z 420.3 [M + 1] 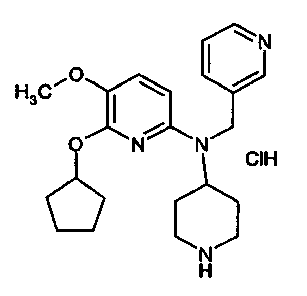
ss) (6-Cyclopentyloxy-5-methoxy-pyridin-2-yl)-piperidin-4-yl-pyridin-3-ylmethyl-amine hydrochloride, 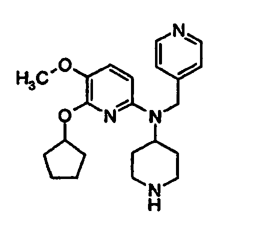
tt) (6-Cyclopentyloxy-5-methoxy-pyridin-2-yl)-piperidin-4-yl-pyridin-4-ylmethyl-amine, 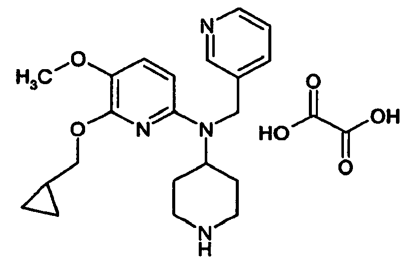
uu) (6-Cyclopopylmethoxy-5-methoxy-pyridin-2-yl)-piperidin-yl-pyridin-3-ylmethylamine, 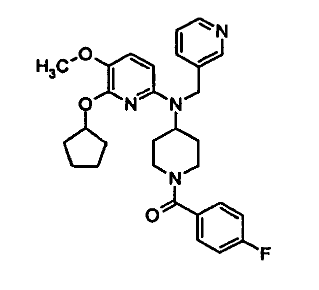
w) {4-[(6-Cyclopentyloxy-5-methoxy-pyridin-2-yl)-pyridin-3-ylmethyl-amino]-piperidin-1-yl}-(4-fluoro-phenyl)-methanone, 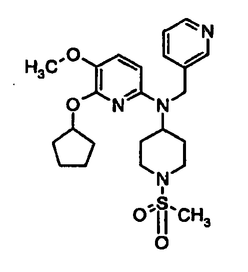
ww) (6-Cyclopentyloxy-5-methoxy-pyridin-2-yl)-(1-methanesulfonyl-piperidin-4-yl)-pyridin-3-ylmethyl-amine, 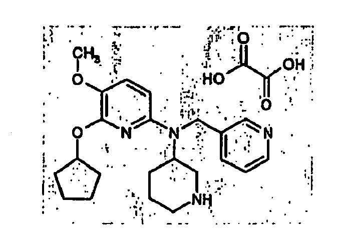
rrr) 6-(cyclopentyloxy)-5-methoxy-N-piperidin-3-yl-N-(pyridin-3-ylmethyl)pyridin-2-amine oxalate, 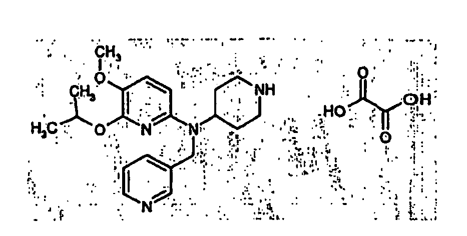
sss) 6-isopropoxy-5-methoxy-N-piperidin-4-yl-N-(pyridin-3-ylmethyl)pyridin-2-amine oxalate, 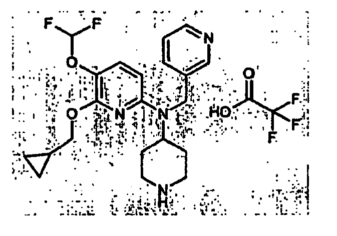
ttt) 6-(cyclopropylmethoxy)-5-(difluoromethoxy)-N-piperidin-4-yl-N-(pyridin-3-ylmethyl)pyridin-2-amine trifluoroacetate, 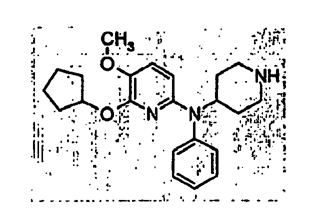
uuu) 6-(cyclopentyloxy)-5-methoxy-N-phenyl-N-piperidin-4-ylpyridin-2-amine, 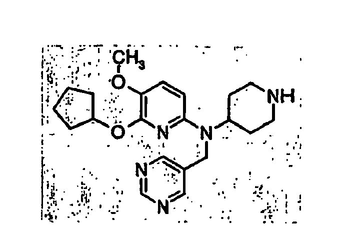
vvv) 6-(cyclopentyloxy)-5-methoxy-N-pipendin-4-yl-N-(pyrimidin-5-ylmethyl)pyridin-2-amine.

The compounds of the present invention are effective in inhibiting, or modulating the activity of PDE4 in animals, e.g., mammals, especially humans. These compounds exhibit neurological activity, especially where such activity affects cognition, including long term memory. These compounds will also be effective in treating diseases where decreased cAMP levels are involved. This includes but is not limited to inflammatory diseases. These compounds may also function as antidepressants, or be useful in treating cognitive and negative symptoms of schizophrenia.

Assays for determining PDE inhibiting activity as well as selectivity of PDE 4 inhibiting activity and selectivity of inhibiting PDE 4 isoenzymes are known within the art. See, e.g., U.S. Patent No. 6,136,821, the disclosure of which is incorporated herein by reference.

According to a further aspect of the invention there are provided compounds useful as intermediates for the production of the PDE4 inhibitors described herein (e.g., PDE4 inhibitors of Formula I) and/or useful for the synthesis of radio-labeled analogs of the PDE4 inhibitors with in this application.

Thus, there are provided intermediate compounds which correspond to compounds of Formula I, wherein R², R³, and R⁴ are as previously defined for Formula I, R¹ is OR⁶, but R⁶ is H, *tert*-butyldimethylsilyl-, or a suitable phenolic protecting group. Suitable phenolic protecting groups are described, for example, in Greene, T.W. and Wuts, P.G.M., Protective Groups in Organic Synthesis, 3rd Edition, John Wiley & Sons, 1999, pp. 246-293. These intermediates are also useful for the synthesis of radio-labeled compounds, such as where R⁶ is ³H₃C-, ¹⁴CH₃- or ¹¹CH₃-, for example, by removing the protecting group and reacting the resultant compound in which R⁶ is H with suitable radio-labelled reagents. Such radio-labeled compounds are useful for determining compound tissue distribution in animals, in PET imaging studies, and for in vivo, ex vivo, and in vitro binding studies.

Also provided are intermediate compounds which correspond to compounds of Formula I, wherein R¹, R³, and R⁴ are as previously defined for Formula I, R² is OR⁷, but R⁷ is H, *tert*-butyldimethylsflyloxy-, or a suitable phenolic protecting group. Suitable phenolic protecting groups are described, for example, in Greene, T.W. and Wuts, P.G.M., Protective Groups in Organic Synthesis, 3rd Edition, John Wiley & Sons, 1999, pp. 246-293. Compounds in which R⁷ is H are useful as intermediates, for example, as scaffolds for parallel or combinatorial chemistry applications. Further, these compounds are useful for the introduction of radio-labels such as ³H, ¹⁴C, or ¹¹C.

As previously described, compounds according to formula II, wherein A, B, D, R¹, R² and R⁴ are as previously described are useful intermediates for the production of compounds according to formula I where in R³ is other than H.

Also, as previously described, compounds according to formula III, wherein A, B, D, R¹, R² and R³ are as previously described are useful intermediates for the production of compounds according to formula I where in R⁴ is other than H.

Halogen herein refers to F, Cl, Br, and I. Preferred halogens are F and Cl.

Alkyl, as a group or substituent per se or as part of a group or substituent (e.g., alkylamino, trialkylsilyloxy, aminoalkyl, hydroxyalkyl), means a straight-chain or branched-chain aliphatic hydrocarbon radical having 1 to 12 carbon atoms, preferably 1 to 8 carbon atoms, especially 1 to 4 carbon atoms. Suitable alkyl groups include methyl, ethyl, propyl, isopropyl, butyl, sec-butyl, *tert*-butyl, pentyl, hexyl, heptyl, octyl, nonyl, decyl, undecyl, and dodecyl. Other examples of suitable alkyl groups include 1-, 2- or 3-methylbutyl, 1,1-, 1,2- or 2,2-dimethylpropyl, 1-ethylpropyl, 1-, 2-, 3- or 4-methylpentyl, 1,1-, 1,2-, 1,3-, 2,2-, 2,3- or 3,3-dimethylbutyl, 1- or 2-ethylbutyl, ethylmethylpropyl, trimethylpropyl, methylhexyl, dimethylpentyl, ethylpentyl, ethylmethylbutyl, dimethylbutyl, and the like.

Substituted alkyl groups are alkyl groups as described above which are substituted in one or more positions by halogens, oxo, hydroxyl, C1-4-alkoxy and/or cyano. Halogens are preferred substituents, especially F and Cl.

Alkoxy means alkyl-O- groups and alkoxyalkoxy means alkyl-O-alkyl-O- groups in which the alkyl portions are in accordance with the previous discussion. Suitable alkoxy and alkoxyalkoxy groups include methoxy, ethoxy, propoxy, butoxy, pentoxy, hexoxy, heptoxy, octoxy, methoxymethoxy, ethoxymethoxy, propoxymethoxy, and methoxyethoxy. Preferred alkoxy groups are methoxy and ethoxy. Similarly, alkoxycarbonyl means alkyl -0-CO- in which the alkyl portion is in accordance with the previous discussion. Examples include methoxycarbonyl, ethoxycarbonyl, propoxycarbonyl, and *tert*-butoxycarbonyl.

Cycloalkyl means a monocyclic, bicyclic or tricyclic nonaromatic saturated hydrocarbon radical having 3 to 10 carbon atoms, preferably 3 to 8 carbon atoms, especially 3 to 6 carbon atoms. Suitable cycloalkyl groups include cyclopropyl, cyclobutyl, cyclopentyl, cyclohexyl, cycloheptyl, cyclooctyl, norbornyl, 1-decalin, adamant-1-yl, and adamant-2-yl. Other suitable cycloalkyl groups include spiropentyl, bicyclo[2.1.0]pentyl, bicyclo[3.1.0]hexyl, spiro[2.4]heptyl, spiro[2.5]octyl, bicyclo[5.1.0]octyl, spiro[2.6]nonyl, bicyclo[2.2.0]hexyl, spiro[3.3]heptyl, bicyclo[4.2.0]octyl, and spiro[3.5]nonyl. Preferred cycloalkyl groups are cyclopropyl, cyclobutyl, cyclopentyl and cyclohexyl. The cycloalkyl group can be substituted, for example, by one or more halogens and/or alkyl groups.

Cycloalkylalkyl refers to cycloalkyl-alkyl radicals in which the cycloalkyl and alkyl portions are in accordance with previous discussions. Suitable examples include cyclopropylmethyl and cyclopentylmethyl.

Aryl, as a group or substituent per se or as part of a group or substituent, refers to an aromatic carbocyclic radical containing 6 to 14 carbon atoms, preferably 6 to 12 carbon atoms, especially 6 to 10 carbon atoms. Suitable aryl groups include phenyl, naphthyl and biphenyl. Substituted aryl groups include the above-described aryl groups which are substituted one or more times by, for example, halogen, alkyl, hydroxy, alkoxy, nitro, methylenedioxy, ethylenedioxy, amino, alkylamino, dialkylamino, hydroxyalkyl, hydroxyalkoxy, carboxy, cyano, acyl, alkoxycarbonyl, alkylthio, alkylsulfinyl, alkylsulfonyl, and phenoxy.

Arylalkyl refers to an aryl-alkyl-radical in which the aryl and alkyl portions are in accordance with the previous descriptions. Suitable examples include benzyl, 1-phenethyl, 2-phenethyl, phenpropyl, phenbutyl, phenpentyl, and napthylmethyl.

Heteroaryl refers to an aromatic heterocyclic group having one or two rings and a total number of 5 to 10 ring atoms wherein at least one of the ring atoms is a heteroatom. Preferably, the heteroaryl group contains 1 to 3, especially 1 or 2, hetero-ring atoms which are selected from N, O and S. Suitable heteroaryl groups include furyl, thienyl, pyrrolyl, pyrazolyl, imidazolyl, triazolyl, tetrazolyl, isoxazolyl, oxazolyl, thiazolyl, isothiazolyl, oxadiazolyl, oxatriazolyl, thiadiazolyl, pyridyl, pyridazinyl, pyrimidinyl, pyrazinyl, triazinyl, benzofuranyl, isobenzofuranyl, thionaphthenyl, isothionaphthenyl, indolyl, isoindolyl, indazolyl, benzisoxazolyl, benzoxazolyl, benzthiazolyl, benzisothiazolyl, purinyl, benzopyranyl, quinolinyl, isoquinolinyl, cinnolinyl, quinazolinyl, naphthyridinyl, and benzoxazinyl, e.g., 2-thienyl, 3-thienyl, 2-, 3- or 4-pyridyl, 2-, 3-, 4-, 5-, 6-, 7- or 8-quinolinyl, and 1-, 3-, 4-, 5-, 6-, 7- or 8-isoquinolinyl.

Substituted heteroaryl refers to the heteroaryl groups described above which are substituted in one or more places by, for example, halogen, aryl, alkyl, alkoxy, carboxy, methylene, cyano, trifluoromethyl, nitro, oxo, amino, alkylamino, and dialkylamino.

Heterocycles include heteroaryl groups as described above as well as non-aromatic cyclic groups containing at least one hetero-ring atom, preferably selected from N, S and O, for example, tetrahydrofuranyl, piperidinyl, dithialyl, oxathialyl, dioxazolyl, oxathiazolyl, oxazinyl, isoxazinyl, oxathiazinyl, oxadiazinyl, and pyrrolidinyl.

Heterocycle-alkyl refers to a heterocycle-alkyl-group wherein the heterocyclic and alkyl portions are in accordance with the previous discussions. Suitable examples are pyridylmethyl, thiazolylmethyl, thienylmethyl, pyrimidinylmethyl, pyrazinylmethyl, and isoquinolinylmethyl.

Partially unsaturated carbocyclic structures are non-aromatic monocyclic or bicyclic structures containing 5 to 14 carbon atoms, preferably 6 to 10 carbon atoms, wherein the ring structure(s) contains at least one C=C bond. Suitable examples are cyclopentenyl, cyclohexenyl, cyclohexadienyl, tetrahydronaphthenyl and indan-2-yl.

Alkenyl refers to straight-chain or branched-chain aliphatic radicals containing 2 to 12 carbon atoms in which one or more -CH₂-CH₂- structures are each replaced by -CH=CH-. Suitable alkenyl groups are ethenyl, 1-propenyl, 2-methylethenyl, 1-butene, 2-butene, 1-pentenyl, and 2-pentenyl.

Alkynyl refers to straight-chain or branched-chain aliphatic radicals containing 2 to 12 carbon atoms in which one or more -CH₂-CH₂- structures are each replaced by -C≡C-. Suitable alkynyl groups are ethynyl, propynyl, 1-butynyl, and 2-butynyl.

Acyl refers to alkanoyl radicals having 1 to 13 carbon atoms in which the alkyl portion can be substituted by halogen, alkyl, aryl and/or alkoxy, or aroyl radicals having 7 to 15 carbon atoms in which the aryl portion can be substituted by, for example, halogen, alkyl and/or alkoxy. Suitable acyl groups include formyl, acetyl, propionyl, butanoyl and benzoyl.

Substituted radicals preferably have 1 to 3 substituents, especially 1 to 2 substituents.

Preferred aspects include pharmaceutical compositions comprising a compound of this invention and a pharmaceutically acceptable carrier and, optionally, another active agent as discussed below; a method of inhibiting a PDE4 enzyme, especially an isoenzyme, e.g., as determined by a conventional assay or one described herein, either in vitro or in vivo (in an animal, e.g., in an animal model, or in a mammal or in a human); a method of treating neurological syndrome, e.g., loss of memory, especially long-term memory, cognitive impairment or decline, memory impairment, etc. a method of treating a disease state modulated by PDE4 activity, in a mammal, e.g., a human, e.g., those mentioned herein.

The compounds of the present invention may be prepared conventionally. Some of the processes which can be used are described below. All starting materials are known or can be conventionally prepared from known starting materials.

The compounds of the present invention may be prepared conventionally. Some of the processes, which can be used, are described below. All starting materials are known or can be conventionally prepared from known starting materials.

The reaction schemes shown below are for illustrative purposes only and should not be viewed as limiting the scope of the synthetic methods available for the production of the compounds described within this application. Note that alternative methods, reagents, solvents, bases, acids etc., which are considered standard in the art, can be utilized in addition or can replace those mentioned here, to prepare many of the compounds described below.

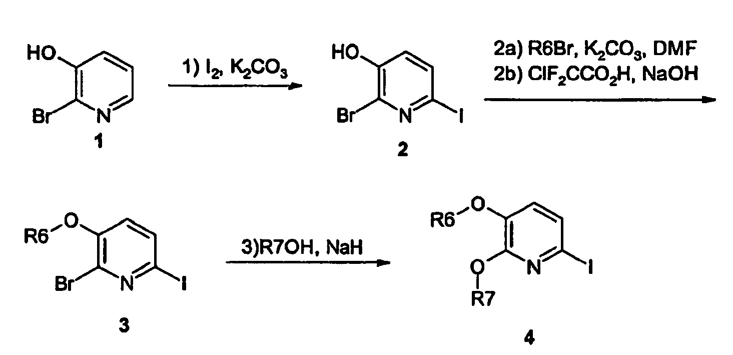

Starting 2,3-diether-6-iodopyridines **4** are prepared in a three step procedure from commercially available 2-bromo-3-hydroxypyridine **1.** Thus, selective 6-iodoination (I₂, K₂CO₃) followed by etherification generates 2-bromo-6-iodopyridines **3** (Koch, V., Schnatter, S., Synthesis, 1990, 497-498). Reaction with a sodium alkoxide (R⁷ONa) provides 2,3-diether-6-iodopyridines **4** (O'Neill, B.T., Yohannes, D., Bundesmann, M.W., Arnold, E.P., Org. Lett., 2000, 2(26), 4201-4204).

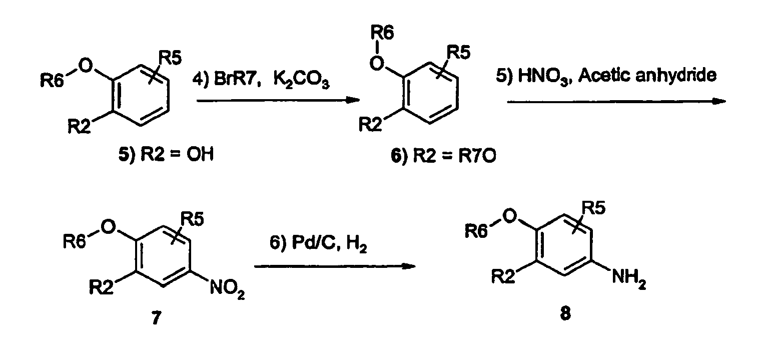

Starting anilines **8** are prepared in a three-step procedure from various 2-alkyloxyphenols **5.** Thus phenol **5** undergoes reaction with an alkylhalide in the presence of a base such as K₂CO₃ to yield substituted dietherbenzenes **6.** Nitration reaction generates nitrocatechols **7,** which are subsequently reduced by catalytic hydrogenation over Pd/C to provide corresponding anilines **8.**

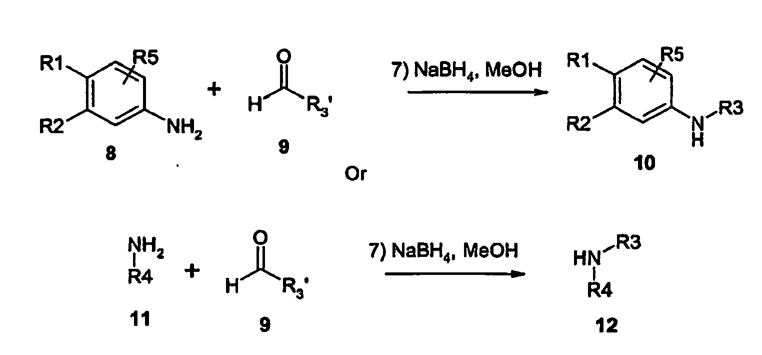

Reductive amination between aniline precursors **8** and aldehydes **9** provide key intermediates **10** in high yield. Alternatively, secondary amines **12** are formed by reductive amination between amines **11** and aldehydes **9.**

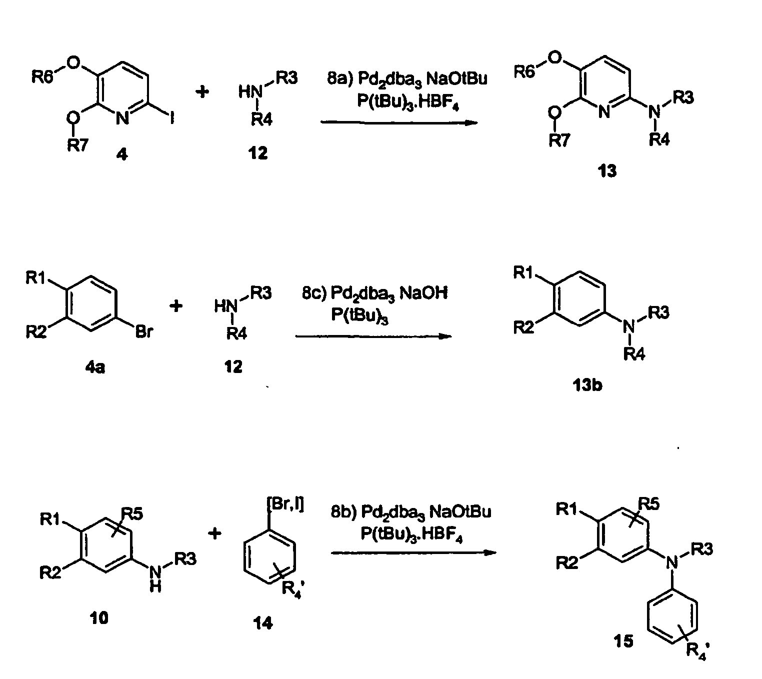

Buchwald *N*-arylation reaction between reductive amination product **12** and 6-iodopyridine **4,** bromobenzene compound 12a, or reductive amination product **10** and an aryl- or heteroaryl-halide **14** provides key targets and intermediates of the general type **13,14** and **15** respectively (Hartwig, J.F., Kawatsura, M., Hauck, S.I., Shaughnessy, K.H., Alcazar-Roman, L.M., J. Org. Chem, 1999, 64, 5575-5580). Compounds of the type **16** where R₄' is CO₂tBu can be converted to the corresponding acid **17** by stirring in a solution of 20% TFA in DCM. When R₄' is a THP-protected tetrazole **18,** the THP group is removed by treating with 3N HCl to provide the tetrazole compounds of type **19** (Greene, T.W., uts, P.G.M., Protective Groups in Organic Synthesis, Third Edition, John Wiley & Sons, Inc. New York, pp. 49-54 and 404-408).

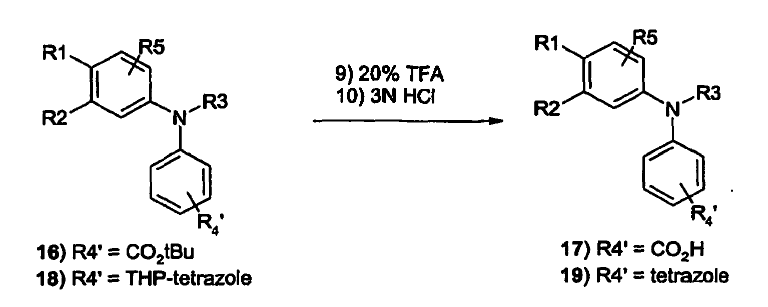

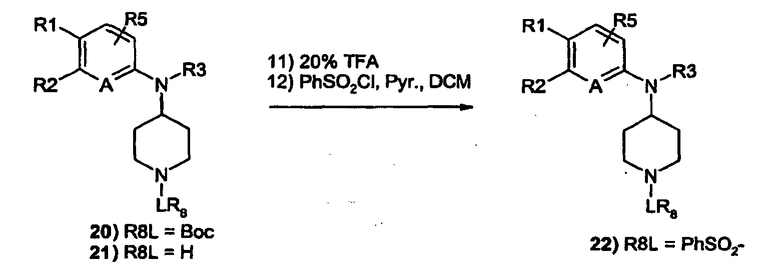

Boc-protected piperidines **20** are unmasked by treating with 20% TFA in DCM to generate piperdine analogs **21.** These piperidines undergo reaction with various acid chlorides and sulfonyl chlorides to provide targets such as **22.**

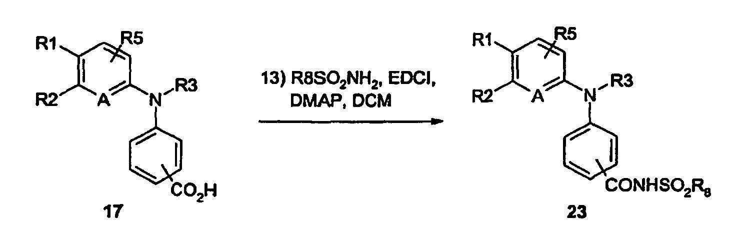

Alternatively, acids **17** undergo reaction with various amine compounds to generate sulfonylaminocarbonyl targets **23** by coupling reaction with a sulfonamide in the presence of EDCI and DMAP.

Esters **24** may be transformed to amides **16** through a three-step procedure via hydrolysis with LiOH in a mixture of THF-MeOH-H₂O to provide acids, which were converted to the corresponding acid chlorides in DCM, and finally to amides **16** by the treatment with various amines.

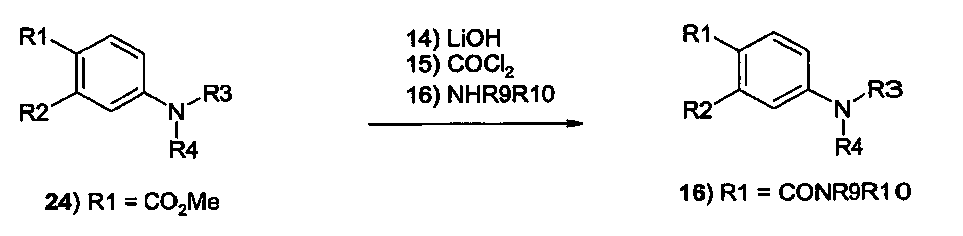

Many of these synthetic procedures are described more fully in the examples below.

One of ordinary skill in the art will recognize that some of the compounds of Formula (I) and the specific compounds listed above can exist in different geometrical isomeric forms. In addition, some of the compounds of the present invention possess one or more asymmetric carbon atoms and are thus capable of existing in the form of optical isomers, as well as in the form of racemic or nonracemic mixtures thereof, and in the form of diastereomers and diastereomeric mixtures inter alia. All of these compounds, including cis isomers, trans isomers, diastereomic mixtures, racemates, nonracemic mixtures of enantiomers, and substantially pure and pure enantiomers, are within the scope of the present invention. Substantially pure enantiomers contain no more than 5% w/w of the corresponding opposite enantiomer, preferably no more than 2%, most preferably no more than 1%.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, dibenzoyltartaric, ditoluoyltartaric and camphorsulfonic acid. Mixtures of diastereoisomers can be separated into their individual diastereomers on the basis of their physical and/or chemical differences by methods known to those skilled in the art, for example, by chromatography or fractional crystallization. The optically active bases or acids are then liberated from the separated diastereomeric salts. A different process for separation of optical isomers involves the use of chiral chromatography (e.g., chiral HPLC columns), with or without conventional derivation, optimally chosen to maximize the separation of the enantiomers. Suitable chiral HPLC columns are manufactured by Diacel, e.g., Chiracel OD and Chiracel OJ among many others, all routinely selectable. Enzymatic separations, with or without derivitization, are also useful. The optically active compounds of Formula I and the specific compounds listed above can likewise be obtained by chiral syntheses utilizing optically active starting materials.

In addition, one of ordinary skill in the art will recognize that the compounds can be used in different enriched isotopic forms, e.g., enriched in the content of ²H, ³H, ¹¹C and/or ¹⁴C.

The present invention also relates to useful forms of the compounds as disclosed herein, such as pharmaceutically acceptable salts and prodrugs of all the compounds of the present invention. Pharmaceutically acceptable salts include those obtained by reacting the main compound, functioning as a base, with an inorganic or organic acid to form a salt, for example, salts of hydrochloric acid, sulfuric acid, phosphoric acid, methane sulfonic acid, camphor sulfonic acid, oxalic acid, maleic acid, succinic acid and citric acid. Pharmaceutically acceptable salts also include those in which the main compound functions as an acid and is reacted with an appropriate base to form, e.g., sodium, potassium, calcium, magnesium, ammonium, and choline salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts are prepared by, for example, reacting a compound of the invention with the appropriate base via a variety of known methods.

The following are further examples of acid salts that can be obtained by reaction with inorganic or organic acids: acetates, adipates, alginates, citrates, aspartates, benzoates, benzenesulfonates, bisulfates, butyrates, camphorates, digluconates, cyclopentanepropionates, dodecylsulfates, ethanesulfonates, glucoheptanoates, glycerophosphates, hemisulfates, heptanoates, hexanoates, fimarates, hydrobromides, hydroiodides, 2-hydroxy-ethanesulfonates, lactates, maleates, methanesulfonates, nicotinates, 2-naphthalenesulfonates, oxalates, palmoates, pectinates, persulfates, 3-phenylpropionates, picrates, pivalates, propionates, succinates, tartrates, thiocyanates, tosylates, mesylates and undecanoates.

Preferably, the salts formed are pharmaceutically acceptable for administration to mammals. However, pharmaceutically unacceptable salts of the compounds are suitable as intermediates, for example, for isolating the compound as a salt and then converting the salt back to the free base compound by treatment with an alkaline reagent. The free base can then, if desired, be converted to a pharmaceutically acceptable acid addition salt.

The compounds of the invention can be administered alone or as an active ingredient of a formulation. Thus, the present invention also includes pharmaceutical compositions of compounds of Formulae I or the compounds specifically mentioned above containing, for example, one or more pharmaceutically acceptable carriers.

Numerous standard references are available that describe procedures for preparing various formulations suitable for administering the compounds according to the invention. Examples of potential formulations and preparations are contained, for example, in the Handbook of Pharmaceutical Excipients, American Pharmaceutical Association (current edition); Pharmaceutical Dosage Forms: Tablets (Lieberman, Lachman and Schwartz, editors) current edition, published by Marcel Dekker, Inc., as well as Remington's Pharmaceutical Sciences (Arthur Osol, editor), 1553-1593 (current edition).

In view of their high degree of PDE4 inhibition, the compounds of the present invention can be administered to anyone requiring or desiring PDE4 inhibition, and/or enhancement of cognition. Administration may be accomplished according to patient needs, for example, orally, nasally, parenterally (subcutaneously, intravenously, intramuscularly, intrasternally and by infusion), by inhalation, rectally, vaginally, topically, locally, transdermally, and by ocular administration.

Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and excipients known in the art, including but not limited to suspending agents, solubilizers, buffering agents, binders, disintegrants, preservatives, colorants, flavorants, lubricants and the like. Time release capsules, tablets and gels are also advantageous in administering the compounds of the present invention.

Various liquid oral dosage forms can also be used for administering compounds of the invention, including aqueous and non-aqueous solutions, emulsions, suspensions, syrups, and elixirs. Such dosage forms can also contain suitable inert diluents known in the art such as water and suitable excipients known in the art such as preservatives, wetting agents, sweeteners, flavorants, as well as agents for emulsifying and/or suspending the compounds of the invention. The compounds of the present invention may be injected, for example, intravenously, in the form of an isotonic sterile solution. Other preparations are also possible.

Suppositories for rectal administration of the compounds of the present invention can be prepared by mixing the compound with a suitable excipient such as cocoa butter, salicylates and polyethylene glycols. Formulations for vaginal administration can be in the form of a pessary, tampon, cream, gel, paste, foam, or spray formula containing, in addition to the active ingredient, such suitable carriers as are known in the art.

For topical administration the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

Aerosol formulations suitable for administering via inhalation also can be made. For example, for treatment of disorders of the respiratory tract, the compounds according to the invention can be administered by inhalation in the form of a powder (e.g., micronized) or in the form of atomized solutions or suspensions. The aerosol formulation can be placed into a pressurized acceptable propellant.

The compounds can be administered as the sole active agent or in combination with other pharmaceutical agents such as other agents used in the treatment of cognitive impairment and/or in the treatment of psychosis, e.g., other PDE4 inhibitors, calcium channel blockers, chloinergic drugs, adenosine receptor modulators, amphakines NMDA-R modulators, mGluR modulators, cholinesterase inhibitors (e.g., donepezil, rivastigimine, and glanthanamine), and selective serotonin reuptake inhibitors (SSRIs). In such combinations, each active ingredient can be administered either in accordance with their usual dosage range or a dose below its usual dosage range.

The present invention further includes methods of treatment that involve inhibition of PDE4 enzymes. Thus, the present invention includes methods of selective inhibition of PDE4 enzymes in animals, e.g., mammals, especially humans, wherein such inhibition has a therapeutic effect, such as where such inhibition may relieve conditions involving neurological syndromes, such as the loss of memory, especially long-term memory. Such methods comprise administering to an animal in need thereof, especially a mammal, most especially a human, an inhibitory amount of a compound, alone or as part of a formulation, as disclosed herein.

The condition of memory impairment is manifested by impairment of the ability to learn new information and/or the inability to recall previously learned information. Memory impairment is a primary symptom of dementia and can also be a symptom associated with such diseases as Alzheimer's disease, schizophrenia, Parkinson's disease, Huntington's disease, Pick's disease, Creutzfeld-Jakob disease, HIV, cardiovascular disease, and head trauma as well as age-related cognitive decline.

Dementias are diseases that include memory loss and additional intellectual impairment separate from memory. The present invention includes methods for treating patients suffering from memory impairment in all forms of dementia. Dementias are classified according to their cause and include: neurodegenerative dementias (e.g., Alzheimer's, Parkinson's disease, Huntington's disease, Pick's disease), vascular (e.g., infarcts, hemorrhage, cardiac disorders), mixed vascular and Alzheimer's, bacterial meningitis, Creutzfeld-Jacob Disease, multiple sclerosis, traumatic (e.g., subdural hematoma or traumatic brain injury), infectious (e.g., HIV), genetic (down syndrome), toxic (e.g., heavy metals, alcohol, some medications), metabolic (e.g., vitamin B12 or folate deficiency), CNS hypoxia, Cushing's disease, psychiatric (e.g., depression and schizophrenia), and hydrocephalus.

The present invention includes methods for dealing with memory loss separate from dementia, including mild cognitive impairment (MCI) and age-related cognitive decline. The present invention includes methods of treatment for memory impairment as a result of disease. In another application, the invention includes methods for dealing with memory loss resulting from the use of general anesthetics, chemotherapy, radiation treatment, post-surgical trauma, and therapeutic intervention.

The compounds may be used to treat psychiatric conditions including schizophrenia, bipolar or manic depression, major depression, and drug addiction and morphine dependence. These compounds may enhance wakefulness. PDE4 inhibitors can be used to raise cAMP levels and prevent neurons from undergoing apoptosis. PDE4 inhibitors are also known to be anti-inflammatory. The combination of anti-apoptotic and anti-inflammatory properties make these compounds useful to treat neurodegeneration resulting from any disease or injury, including stroke, spinal cord injury, Alzheimer's disease, multiple sclerosis, amylolaterosclerosis (ALS), and multiple systems atrophy (MSA).

Thus, in accordance with a preferred embodiment, the present invention includes methods of treating patients suffering from memory impairment due to, for example, Alzheimer's disease, multiple sclerosis, amylolaterosclerosis (ALS), multiple systems atrophy (MSA), schizophrenia, Parkinson's disease, Huntington's disease, Pick's disease, Creutzfeld-Jakob disease, Rubenstein-Taybi syndrome (RSTS), depression, aging, head trauma, stroke, spinal cord injury, CNS hypoxia, cerebral senility, diabetes associated cognitive impairment, memory deficits from early exposure of anesthetic agents, multiinfarct dementia and other neurological conditions including acute neuronal diseases, as well as HIV and cardiovascular diseases, comprising administering an effective amount of a compound according to Formulas I-III or pharmaceutically acceptable salts thereof.

The compounds of the present invention can also be used in a method of treating patients suffering from disease states characterized by decreased NMDA function, such as schizophrenia. The compounds can also be used to treat psychosis characterized by elevated levels of PDE 4, for example, various forms of depression, such as manic depression, major depression, and depression associated with psychiatric and neurological disorders.

The compounds of the present invention can also be used in methods of treating patients suffering from obesity and in treatment methods for neuronal regeneration or neurogenesis.

As mentioned, the compounds of the invention also exhibit anti-inflammatory activity. As a result, the inventive compounds are useful in the treatment of a variety of allergic and inflammatory diseases, particularly disease states characterized by decreased cyclic AMP levels and/or elevated phosphodiesterase 4 levels. Thus, in accordance with a further embodiment of the invention, there is provided a method of treating allergic and inflammatory disease states, comprising administering an effective amount of a compound according to Formula (I), or of the compounds listed above, or a pharmaceutically acceptable salt thereof. Such disease states include: asthma, chronic bronchitis, chronic obstructive pulmonary disease (COPD), atopic dermatitis, urticaria, allergic rhinitis, allergic conjunctivitis, vernal conjunctivitis, esoniophilic granuloma, psoriasis, inflammatory arthritis, rheumatoid arthritis, septic shock, ulcerative colitis, Crobn's disease, reperfusion injury of the myocardium and brain, chronic glomemlonephritis, endotoxic shock, adult respiratory distress syndrome, cystic fibrosis, arterial restenosis, artherosclerosis, keratosis, rheumatoid spondylitis, osteoarthritis, pyresis, diabetes mellitus, pneumoconiosis, chronic obstructive airways disease, chronic obstructive pulmonary disease, toxic and allergic contact eczema, atopic eczema, seborrheic eczema, lichen simplex, sunburn, pruritis in the anogenital area, alopecia areata, hypertrophic scars, discoid lupus erythematosus, systemic lupus erythematosus, follicular and wide-area pyodermias, endogenous and exogenous acne, acne rosacea, Beghet's disease, anaphylactoid purpura nephritis, inflammatory bowel disease, leukemia, multiple sclerosis, gastrointestinal diseases, autoimmune diseases and the like.

PDE4 inhibitors for treating asthma, chronic bronchitis, psoriasis, allergic rhinitis, and other inflammatory diseases, and for inhibiting tumor necrosis factor are known within the art. See, e.g., WO 98/58901, JP11-18957, JP 10-072415, WO 93/25517, WO 94/14742, US 5,814,651, and US 5,935,978. These references also describe assays for determining PDE4 inhibition activity, and methods for synthesizing such compounds. The entire disclosures of these documents are hereby incorporated by reference. PDE4 inhibitors may be used to prevent or ameliorate osteoporosis, as an antibiotic, for treatment of cardiovascular disease by mobilizing cholesterol from atherosclerotic lesions, to treat rheumatoid arthritis (RA), for long-term inhibition of mesenchymal-cell proliferation after transplantation, for treatment of urinary obstruction secondary to benign prostatic hyperplasia, for suppression of chemotaxis and reduction of invasion of colon cancer cells, for treatment of B cell chronic lymphocytic leukemia (B-CLL), for inhibition of uterine contractions, to attenuate pulmonary vascular ischemia-reperfusion injury (IRI), for corneal hydration, for inhibition of IL-2R expression and thereby abolishing HIV-1 DNA nuclear import into memory T cells, for augmentation of glucose-induced insulin secretion, in both the prevention and treatment of colitis, and to inhibit mast cell degranulation.

The compounds of the present invention can be administered as the sole active agent or in combination with other pharmaceutical agents such as other agents used in the treatment of cognitive impairment and/or in the treatment of psychosis, e.g., other PDE4 inhibitors, calcium channel blockers, chloinergic drugs, adenosine receptor modulators, amphakines NMDA-R modulators, mGluR modulators, and cholinesterase inhibitors (e.g., donepezil, rivastigimine, and glanthanamine). In such combinations, each active ingredient can be administered either in accordance with their usual dosage range or a dose below their usual dosage range.

The dosages of the compounds of the present invention depend upon a variety of factors including the particular syndrome to be treated, the severity of the symptoms, the route of administration, the frequency of the dosage interval, the particular compound utilized, the efficacy, toxicology profile, pharmacokinetic profile of the compound, and the presence of any deleterious side-effects, among other considerations.

The compounds of the invention are typically administered at dosage levels and in a manner customary for PDE4 inhibitors such as those known compounds mentioned above. For example, the compounds can be administered, in single or multiple doses, by oral administration at a dosage level of, for example, 0.001-100 mg/kg/day, preferably 0.01-70 mg/kg/day, especially 0.01-10 mg/kg/day. Unit dosage forms can contain, for example, 0.1-50 mg of active compound. For intravenous administration, the compounds can be administered, in single or multiple dosages, at a dosage level of, for example, 0.001-50 mg/kg/day, preferably 0.001-10 mg/kg/day, especially 0.01-1 mg/kg/day. Unit dosage forms can contain, for example, 0.1-10 mg of active compound.

In carrying out the procedures of the present invention it is of course to be understood that reference to particular buffers, media, reagents, cells, culture conditions and the like are not intended to be limiting, but are to be read so as to include all related materials that one of ordinary skill in the art would recognize as being of interest or value in the particular context in which that discussion is presented. For example, it is often possible to substitute one buffer system or culture medium for another and still achieve similar, if not identical, results. Those of skill in the art will have sufficient knowledge of such systems and methodologies so as to be able, without undue experimentation, to make such substitutions as will optimally serve their purposes in using the methods and procedures disclosed herein.

The present invention will now be further described by way of the following non-limiting examples. In applying the disclosure of these examples, it should be kept clearly in mind that other and different embodiments of the methods disclosed according to the present invention will no doubt suggest themselves to those of skill in the relevant art.

In the foregoing and in the following examples, all temperatures are set forth uncorrected in degrees Celsius; and, unless otherwise indicated, all parts and percentages are by weight.

The entire disclosures of all applications, patents and publications, cited above and below, are hereby incorporated by reference.

### EXAMPLES

### Example 1

### 2-Bromo-3-hydroxy-6-iodopyridine

To a mixture of 14g of 2-bromo-3-hydroxypyridine (80.5 mmol), and 22.3g of K₂CO₃ (161 mmol) in 180 mL of water at room temperature was added 21.0g of I₂ (82.7 mmol) in one portion. The mixture was stirred at room temperature for 2h then carefully neutralized with 3N HCl (aq) to pH = 6. The solid was collected by vacuum filtration and washed with water (100 mL), 2M aqueous sodium bisulfite (50 mL), and water (100 mL).
The solid was dried in vacuo to give 16.1g of 2-bromo-3-hydroxy-6-iodopyridine as a tan solid. ¹H NMR (300 MHz, MeOD) δ 7.57 (d, J=8.3Hz, 1H), 6.95 (d, J=8.3Hz, 1H).

### Example 2A

### 2-Bromo-6-iodo-3-methoxypyridine

To a mixture of 16.1 g of 2-bromo-3-hydroxy-6-iodopyridine, and 7.0g of K₂CO₃ in 35 mL DMF was added 11 mL of iodomethane and the mixture was heated to 100 °C for 2h. The mixture was cooled and 150 mL of water was added and the solid was collected by vacuum filtration. The solid was washed with several portions of water and dried in vacuo to give 15.7g of 2-bromo-6-iodo-3-methoxypyridine as a tan solid. ¹H NMR (300 MHz, MeOD) δ 7.70 (d, J=8.3Hz, 1H), 7.14 (d, J=8.3Hz, 1H), 3.91 (s, 3H).

### Example 2B

### 2-Bromo-3-difluoromethoxy-6-iodopyridine

To a solution of 5.0 g (16.7 mmol) of 2-bromo-3-hydroxy-6-iodopyridine in 300 mL of DMF was added 7.6 g (50 mmol) of sodium chlorodifluoroacetate and 0.70 g (17.5 mmol) of NaOH. The light brown solution was warmed to 55 °C with stirring for 16 hours, concentrated in vacuo, diluted with 150 mL of H₂O and extracted with 2 x 150 mL of EtOAc. The combined EtOAc fractions were concentrated to give 4.0 g of crude product which was purified by chromatography over SiO₂ using a gradient elution going from 2% EtOAc in hexanes to 4% EtOAc in hexanes to provide 3.43 g (59% yield) of 2-bromo-3-difluoromethoxy-6-iodopyridine as a pale yellow oil. ¹H NMR (500 MHz, CDCl₃) δ 7.68 (d, J=8.3Hz, 1H), 7.21 (d, J=8.3Hz, 1H), 6.58 (t, J=72.0Hz, 1H), (s, 3H).

### Example 3

### 2-Cyclopentyloxy-6-iodo-3-methoxypyridine

To a mixture of 1.0 g NaH (60% mineral oil dispersion) in 8 mL DMF at room temperature was carefully added 2.2 mL of cyclopentanol and the mixture was allowed to stir for 1h at room temperature. A solution of 4.95 g of 2-bromo-6-iodo-3-methoxypyridine in DMF (2 mL) was added and the mixture was heated to 100 °C for 2h. The mixture was cooled to room temperature and partitioned between Et₂O (100 mL) and water (100 mL). The organic layer was separated, washed with brine (50 mL), dried (MgSO₄), and concentrated in vacuo. The residue was purified by column chromatography eluting with a linear gradient from 0% to 10% EtOAc in hexanes to yield 4.0 g of 2-cyclopentyloxy-6-iodo-3-methoxypyridine as a tan solid. ¹H NMR (300 MHz, MeOD) δ 7.18 (d, J=8.1Hz, 1H), 6.71 (d, J=8.1Hz, 1H), 5.42 (m, 1H), 3.81 (s, 3H), 2.0-1.8 (m, 2H), 1.8-1.7 (m, 4H), 1.7-1.5 (m, 2H).

The following compounds were prepared in a similar manner as described above.
a) 2-Cyclobutyloxy-6-iodo-3-methoxypyridine
b) 2-Cyclopropylmethoxy-6-iodo-3-methoxypyridine
c) 2,3-Dimethoxy-6-iodopyridine
d) 2-Cyclopropylmethoxy-3-difluoromethoxy-6-iodopyridine
e) 3-Difluoromethoxy-6-iodo-2-methoxypyridine
f) 2-Ethoxy-6-iodo-3-methoxypyridine
g) 6-Iodo-2-(2-propyl)oxy-3-methoxypyridine
h) 3-Difluoromethoxy-6-iodo-2-(2-propyl)oxypyridine
i) 2-Cyclobutyloxy-3-difluoromethoxy-6-iodopyridine
j) 6-Iodo-3-methoxy-2-[(3*R*)-tetrahydrofuran-3-yl]oxypyridine
k) 6-Iodo-3-methoxy-2-[tetrahydrofuran-3-yl]oxypyridine

### Example 4

### 2-Cyclopentyloxy-5-fluoroanisole

To a mixture of 4-fluoro-2-methoxyphenol (5.0 g, 35 mmol) in 100 mL acetonitrile was added K ₂CO₃ (10 g, 72 mmol) and bromocyclopentane (10.7 g, 72 mmol). The mixture was heated to 65 °C and stirred for 18 h. The mixture was partitioned between Et₂O (250 mL) and water (250 mL). The ether layer was separated, washed with brine, dried (MgSO₄) and concentrated to give 5.2 g of 2-cyclopentyloxy 5-fluoroanisole as a yellow oil.

The following compounds were prepared in a similar fashion as described above.
a) 2-Cyclopentyloxy-3-fluoroanisole
b) 5-Chloro-2-Cyclopentyloxyanisole
c) 2-Cyclopentyloxy-5-methylanisole

### Example 5

### 1-Cyclopentyloxy4-fluero-2-methoxy-5-nitrobenzene

To a solution of 2-cyclopentyloxy-5-fluoroanisole (2.10 g) in 15 mL of acetic anhydride at 0 °C was added drop-wise 0.90 mL of 70% nitric acid. The mixture was warmed to room temperature and stirred for 0.5h and then carefully neutralized with saturated aqueous Na₂CO₃. The solid was collected by vacuum filtration, washed with several portions of water and dried in vacuo to give 2.3 g of 1-cyclopentyloxy-4-fluoro-2-methoxy-5-nitrobenzene as a tan solid. ¹H NMR (300 MHz, CDCl₃) δ 7.57 (d, J=7.3Hz, 1H), 6.70 (d, J=12.5Hz, 1H), 4.78 (m, 1H), 3.93 (s, 3H), 2.1-1.5 (m, 8H).

The following compounds were prepared in a similar fashion as described above.
a) 2-Cyclopentyloxy-3-fluoro-1-methoxy-4-nitrobenzene
b) 4-Chloro-1-Cyclopentyloxy2-methoxy-5-nitrobenzene
c) 1-Cyclopentyloxy-2-methoxy-4-methyl-5-nitrobenzene

### Example 6

### 5-Cyclopentyloxy-2-fluoro4-methoxyaniline

A solution of 2.3 g of 5-cyclopentyloxy-2-fluoro-4-methoxynitrobenzene in 50 mL of EtOH was added to 100 mg 10% palladium on carbon and the mixture was shaken under 30 psi hydrogen for 3h. The suspension was filtered through celite and the celite pad was washed with several portions of EtOH. The solution was concentrated in vacuo to yield 2.0 g of 5-cyclopentyloxy-2-fluoro-4-methoxyaniline as a yellow oil, which was not purified further.

The following compounds were prepared in a similar fashion as described above.
a) 3-Cyclopentyloxy-2-fluoro-4-methoxyaniline
b) 2-Chloro-5-cyclopentyloxy-4-methoxyaniline
c) 5-Cyclopentyloxy-4-methoxy-2-methylaniline

### Example 7

### 3-Chloro-4-methozy-N-(3-pyridylmethyl)aniline

To a mixture of 3-pyridinecarboxaldehyde (2.2 g, 20 mmol) in ethanol (100 mL) was added 3-chloro-4-methoxyaniline (3.14 g, 20 mmol) and p-toluenesulfonic acid monohydrate (2.0 mg). The reaction mixture was stirred for 16h, cooled to 0°C and sodium borohydride (0.87g, 23 mmol) was added portion wise over 4h. The reaction mixture was slowly warmed to room temperature and stirring continued for 16 hours. The solvent was evaporated and the remaining reaction mixture was diluted with water (50 mL) and extracted with ethyl acetate (2 x 20 mL). The combined organic layers were washed with brine (5 mL), dried (MgSO₄), and concentrated to yield 2.2 g of 3-chloro-4-methoxy-*N*-(3-pyridylmethyl)aniline as a tan solid. ¹H NMR (300 MHz, CDCl₃) δ 8.61 (s, 2H), 8.53 (d, J=4.7Hz, 1H), 7.68 (d, J=7.8Hz, 1H), 7.27 (m, 1H), 6.80 (d, J=8.8Hz, 1H), 6.70 (d, J=2.8Hz, 1H), 6.48 (dd, J=8.8Hz, 2.8Hz, 1H), 4.30 (s, 2H), 3.81 (s, 3H).

The following compounds were prepared in a similar manner as described above.
a) 3-Fluoro-4-difluoromethoxy-*N*-(3-pyridylmethyl)aniline

### Example 8A

### N-(3-Chlorophenyl)-N-[5-methoxy-6-(3R)-(tetrahydrofuran-3-yloxy)pyridin-2-yl]-pyridin-3-ylmethylamine

To a 25 mL oven dried, argon flushed flask was added 180 mg 6-iodo-3-methoxy-2-((3*R*)-3-tetrahydrofuranyl)oxypyridine (0.56 mmol), 111 mg 3-chlorophenyl-*N*-(3-pyridylmethyl)amine (0.50 mmol), 70 mg of NaOtBu (0.70 mmol), 30 mg Pd₂dba₃ (0.033 mmol), 20 mg P(tBu)₃.HBF₄ (0.69 mmol), and 5 mL of toluene. The mixture was stirred for 18 hours at room temperature, filtered through celite and the celite plug was washed with several portions of toluene, concentrated to 5 mL in vacuo and loaded onto a 12 g silica gel column. The product was eluted using a linear gradient from 45% to 55% EtOAc in hexanes to give 83 mg of *N*-(3-Chlorophenyl)-*N*-[5-methoxy-6-(3*R*)-(tetrahydrofuran-3-yloxy)pyridin-2-yl]-pyridin-3-ylmethylamine as a yellow oil. ¹H NMR (300 MHz, CDCl₃) δ 8.56 (s, 1H), 8.48 (d, J=3.4Hz, 1H), 7.59 (d, J=7.8 Hz, 1H), 7.3-7.1 (m, 3H), 7.1-6.9 (m, 2H), 7.00 (d, J=8.4Hz, 1H), 6.31 (d, J=8.4Hz, 1H), 5.28 (m, 1H), 5.14 (s, 2H), 4.0-3.7 (m, 4H), 3.79 (s, 3H), 2.10 (m, 2H).

The following compounds were prepared in a similar manner as described above
a) *tert*-Butyl 3-[*N*-(6-Cyclopropylmethoxy-5-methoxypyridin-2-yl)-pyridin-3-ylmethylamino]benzoate
b) *tert*-Butyl 3-[*N*-(5,6-Diinethoxypyridin-2-yl)-pyridin-3-ylmethylamino]-benzoate
c) *tert*-Butyl 3-[*N*-(6-Cyclobutyloxy-5-methoxypyridin-2-yl)-pyridin-3-ylmethylamino]benzoate
d) *tert*-Butyl 3-[*N*-(6-Cyclopropylmethoxy-5-difluoromethoxypyridin-2-yl)-pyidin-3-ylmethylamino]benzoate
e) *tert*-Butyl 3-[*N*-(5-Difluoromethoxy-6-methoxypyridin-2-yl)-pyridin-3-ylmethylamino]benzoate
f) *tert*-Butyl 3-[*N*-(6-Ethoxy-5-methoxypyridin-2-yl)-pyridin-3-ylmethylamino]benzoate
g) *tert*-Butyl 3-[*N*-(6-Isopropoxy-5-methoxypyridin-2-yl)-pyridin-3-ylmethylamino]benzoate
h) *tert*-Butyl3-[*N*-(5-Difluoromethoxy-6-isopropoxypyridin-2-yl)-pyridin-3-ylmethylamino]benzoate
i) *tert*-Butyl 3-[*N*-(6-Cyclobutyloxy-5-difluoromethoxypyridin-2-yl)-pyridin-3-ylmethylamino]benzoate
j) 4-(*N*-{[5-Methoxy-6-(3*R*)-(tetrahydrofuran-3-yloxy)-pyridin-2-yl]-pyridin-3-ylmethylamino}-methyl)-*N*-piperidine-1-carboxylic acid *tert*-butyl ester
k) 4-{*N*-[5-Methoxy-6-(3*R*)-(tetrahydrofuran-3-yloxy)-pyridin-2-yl]-pyridin-3-ylmethylamino}-piperidine-1-carboxylic acid *tert*-butyl ester
l) *tert*-Butyl 3-{*N*-[5-Methoxy-6-(3*R*)-(tetrahydrofuran-3-yloxy)-pyridin-2-yl]-pyridin-3-ylmethylamino}-benzoate
m) *N*-[5-Methoxy-6-(3*R*)-(tetrahydrofuran-3-yloxy)-pyridin-2-yl]-pyridin-3-ylmethyl-*N-*[4-(2-(tetrahydropyran-2-yl)-2H-tetrazol-5-yl)phenyl]amine
n) *N*-Cyclohexyl-*N*-[5-methoxy-6-(3*R*)-(tetrahydrofuran-3-yloxy)-pyridin-2-yl]-pyridin-3-ylmethylamine
o) *N*-[5-Methoxy-6-(3*R*)-(tetrahydrofuran-3-yloxy)-pyridin-2-yl]-*N*-phenyl-pyridin-3-ylmethylamine
p) *tert*-Butyl 3-{*N*-[5-Methoxy-6-(tetrahydrofuran-3-yloxy)-pyridin-2-yl]pyrdin-3-ylmethylamino}benzoate
q) *tert*-Butyl 3-[*N*-(6-Cyclopentyloxy-5-methoxypyridin-2-yl)-pyridin-3-ylmethylamino]benzoate

### Example 8B

### tert-Butyl 3-[N-(3-Chloro-4-methoxypheoyl)-pyridio-3-ylmethylamioo]beozoate

To a solution of 3-chloro-4-methoxyphenyl-*N*-(3-pyridylmethyl)amine (248 mg, 1 mmol) and t-butyl 3-iodobenzoate (450 mg, 1.5 mmol) in 10 mL toluene was added NaOtBu (150 mg, 1.5 mmol), Pd₂dba₃ (18 mg, 0.02 mmol), and P(tBu)₃HBF₄ (12 mg, 0.04 mmol). The mixture was stirred overnight then filtered through celite and loaded onto a silica column (12g): The product was eluted with a linear gradient from 30% to 45% EtOAc in hexanes to give *tert*-Butyl 3-[*N*-(3-Chloro-4-methoxyphenyl)-pyridin-3-ylmethylamino]benzoate as a yellow oil.

The following compounds were prepared in a similar manner as described above:
a) *tert*-Butyl 4-[N-(3-Chloro-4-methoxyphenyl)-pyridin-3-yhnethylamino]benzoate
b) *tert*-Butyl 3-[N-(3-Fluoro-4-methoxyphenyl)-pyddin-3-ylmethylamino]benzoate
c) *tert*-Butyl 4-[N-(3-Fluoro-4-methoxyphenyl)-pyridin-3-ylmethylamino]benzoate
d) *tert*-Butyl 3-[N-(3-Cyclopentyloxy-2-fluoro-4-methoxyphenyl)-pyridin-3-ylmethylamino]benzoate
e) *tert*-Butyl 3-[N-(2-Chloro-5-cyclopentyloxy-4-methoxyphenyl)pyridin-3-ylmethylamino]benzoate
f) *tert*-Butyl 4-[N-(2-Chloro-5-cyclopentyloxy-4-methoxyphenyl)pyridin-3-ylmethylamino]benzoate
g) *tert*-Butyl 4-[N-(3-Cyclopentyloxy-2-fluoro-4-methoxyphenyl)-pyridin-3-ylmethylamino]benzoate
h) *tert*-Butyl 4-[N-(5-Cyclopentyloxy-4-methoxy-2-methylphenyl)pyridin-3-ylmethylamino]benzoate
i) *tert*-Butyl 3-[N-(5-Cyclopentyloxy-4-methoxy-2-methylphenyl)pyridin-3-ylmethylamino]benzoate
j) N-(5-Cyclopentyloxy-4-methoxy-2-methylphenyl)-N-phenyl-pyridin-3-ylmethylamine
k) *tert*-Butyl 4-[N-(5-Cyclopentyloxy-2-fluoro-4-methoxyphenyl)-pyridin-3-ylmethylamino]benzoate
l) *tert*-Butyl 3-[N-(5-Cyclopentyloxy-2-fluoro-4-methoxyphenyl)-pyridin-3-ylmethylamino]benzoate
m) N-(5-Cyclopentyloxy-2-fluoro-4-methoxyphenyl)-N-phenyl-pyridin-3-ylmethylamine
n) *tert*-Butyl 3-[(4-Difluoromethoxy-3-fluorophenyl)pyridin-3-ylmethylamino]benzoate
o) *tert*-Butyl 3-[(4-Difluoromethoxy-3-fluorophenyl)-(3-fluorobenzyl)amino]benzoate
p) *tert*-Butyl 3-[(2,6-Difluorobemyl)-(4-difluoromethoxy-3-fluorophenyl)amino]benzoate

### Example 9

### 3-[N-(6-Cyclopentyloxy-5-methoxypyridin-2-yl)-pyridin-3-ylmethylamino]benzoic acid

*tert*-Butyl 3-[*N*-(6-cyclopentyloxy-5-methoxypyridin-2-yl)-pyridin-3-ylmethylamino]benzoate (652 mg) was taken up in 10 mL 20% TFA in dichloromethane and allowed to stir overnight. The solvent was removed in vacuo and the residue was partitioned between 50 mL EtOAc and 50 mL water. The aqueous fraction was adjusted to a pH of 5-6 with saturated aqueous sodium bicarbonate and the EtOAc layer was separated, washed with brine, dried and concentrated. The residue was purified by column chromatography eluting with EtOAc to give 440 mg of 3-[*N*-(6-cyclopentyloxy-5-methoxypyridin-2-yl)-pyridin-3-yhnethylamino]benzoic acid as a yellow solid. ¹H NMR (300 MHz, CDCl₃) δ 8.66 (s, 2H), 8.51 (br, 1H), 7.94 (s, 1H), 7.76 (m, 2H), 7.45-7.25 (m, 3H), 6.91 (d, J=8.1Hz, 1H), 6.19 (d, J=8.1Hz, 1H), 5.30-5.10 (m, 3H), 3.75 (s, 3H), 1.70 (m, 6H), 1.45 (m, 2H).

The following compounds were prepared in a similar manner as described above.
a) 3-[*N*-(6-Cyclopropylmethoxy-5-methoxypyridin-2-yl)-pyridin-3-ylmethylamino]benzoic acid
b) 3-[*N*-(5,6-Dimethoxypyridin-2-yl)-pyridin-3-ylmethylamino]-benzoic acid
c) 3-[*N*-(6-Cyclobutyloxy-5-methoxypyridin-2-yl)-pyridin-3-ylmethylamino]benzoic acid
d) 3-[*N*-(6-Cyclopropylmethoxy-5-difluoromethoxypyridin-2-yl)-pyridin-3-ylmethylamino]benzoic acid
e) 3-[*N*-(5-Difluoromethoxy-6-methoxypyridin-2-yl)-pyridin-3-ylmethylamino]benzoic acid
f) 3-[*N*-(6-Ethoxy-5-methoxypyridin-2-yl)-pyridin-3-ylmethylamino]benzoic acid
g) 3-[*N*-(6-Isopropoxy-5-methoxypyridin-2-yl)-pyridin-3-ylmethylamino]benzoic acid
h) 3-[*N*-(5-Difluommethoxy-6-isopropoxypyridin-2-yl)-pyridin-3-ylmethylamino]benzoic acid
i) 3-[*N*-(6-Cyclobutyloxy-5-fluoromethoxypyridin-2-yl)-pyridin-3-ylmethylamino]benzoic acid
j) 4-[*N*-(3-Chloro-4-methoxyphenyl)-pyridin-3-ylmethylamino]benzoic acid
k) 3-[*N*-(3-Chloro-4-methoxyphenyl)-pyridin-3-ylmethylamino]benzoic acid
l) 3-[*N*-(3-Fluoro-4-methoxyphenyl)-pyridin-3-ylmethylamino]benzoic acid
m) 4-[*N*-(3-Fluoro-4-methoxyphenyl)-pyridin-3-ylmethylamino]benzoic acid
n) 3-{*N*-[5-Methoxy-6-(3*R*)-(tetrahydrofuran-3-yloxy)-pyridin-2-yl]-pyridin-3-ylmethylamino}-benzoic acid
o) 3-{*N*-[5-Methoxy-6-(tetrahydrofuran-3-yloxy)-pyridin-2-yl]pyridin-3-ylmethylamino}benzoic acid
p) 3-[*N*-(3-Cyclopentyloxy-2-fluoro-4-methoxyphenyl)-pyridin-3-ylmethylamino]benzoic acid
q) 3-[*N*-(2-Chloro-5-cyclopentyloxy-4-methoxyphenyl)pyridin-3-ylmethylamino]benzoic acid
r) 4-[*N*-(2-Chloro-5-cyclopentyloxy-4-methoxyphenyl)pyridin-3-ylmethylamino]benzoic acid
s) 4-[*N*-(3-Cyclopentyloxy-2-fluoro-4-methoxyphenyl)-pyridin-3-ylmethylamino]benzoic acid
t) 3-[*N*-(6-Cyclopentyloxy-5-methoxypyridin-2-yl)-pyridin-3-ylmethylamino]benzoic acid
u) 4-[*N*-(5-Cyclopentyloxy-4-methoxy-2-methylphenyl)pyridin-3-ylmethylamino]benzoic acid
v) 3-[*N*-(5-Cyclopentyloxy-4-methoxy-2-methylphenyl)pyridin-3-ylmethylamino]benzoic acid
w) 4-[*N*-(5-Cyclopentyloxy-2-fluoro-4-methoxyphenyl)-pyridin-3-ylmethylamino]benzoic acid
x) 3-[*N*-(5-Cyclopentyloxy-2-fluoro-4-methoxyphenyl)-pyridin-3-ylmethylamino]benzoic acid
y) 3-[(4-Difluoromethoxy-3-fluorophenyl)pyridin-3-ylmethylamino]benzoic acid
z) 3-[(4-Difluoromethoxy-3-fluorophenyl)-(3-fluorobenzyl)amino]benzoic acid
aa) 3-[(2,6-Difluorobenzyl)-(4-difluoromethoxy-3-fluorophenyl)amino]benzoic acid

### Example 10

### N-[5-Methoxy-6-(3R)-(tetrahydrofuran-3-yloxy)-pyridin-2-yl]-pyridin-3-ylmethyl-N-[4-(2H-tetrazol-5-yl)phenyl]amine

*N*-[5-Methoxy-6-(3*R*)-(tetrahydrofuran-3-yloxy)-pyridin-2-yl]-pyridin-3-ylmethyl-*N*-[4-(2-(tetrabydropyran-2-yl)-2H-tetrazol-5-yl)phenyl]amine (180 mg, 0.34 mmol) was dissolved in THF (5 mL) and 3 mL of 1N HCl was added. After 6 h at room temperature the mixture was neutralized to pH = 5 with saturated aqueous sodium bicarbonate and extracted with EtOAc (3 x 50 mL). The EtOAc extracts were combined, washed with brine (50 mL), dried (MgSO₄), and concentrated *in vacuo*. The crude residue was loaded onto a RediSep column (10 g, silica gel) and the product was eluted using a linear gradient from 0% to 5% MeOH in EtOAc over 20 min to give 70 mg of *N*-[5-methoxy-6-(3*R*)-(tetrahydrofuran-3-yloxy)-pyridin-2-yl]-pyridin-3-ylmethyl-*N*-[4-(2H-tetrazol-5-yl)phonyl]amine as a yellow solid ¹H NMR (300 MHz, CDCl₃) δ 8.64 (s, 1H), 8.51 (d, J=4.0Hz, 1H), 7.97 (d, J=8.7Hz, 2H), 7.78 (d, J=7.8 Hz, 1H), 7.34 (m, 2H), 7.20 (d, J=8.7Hz, 2H), 7.02 (d, J=8.3Hz, 1H), 6.55 (d, J=8.3Hz, 1H), 5.4-5.2 (m, 3H), 4.0-3.7 (m, 4H), 3.79 (s, 3H), 2.11 (m, 2H).

### Example 11

### N-[5-Methoxy-6-(3R)-(tetrahydrofuran-3-ylogy)-pyridin-2-yl]-piperidin-4-yl-pyridin-3-ylmethylamine

4-{*N*-[5-Methoxy-6-(3*R*)-(tetrahydrofuran-3-yloxy)-pyridin-2-yl]-pyridin-3-ylmethylamino}-piperidine-1-carboxylic acid *tert*-butyl ester (110 mg,) was taken up in 10 mL of 20% TFA in dichloromethane and the mixture was stirred for 18 h, concentrated, and partitioned between EtOAc and sat. aq. NaHCO₃. The EtOAc was separated, washed with brine, dried over magnesium sulfate and concentrated to give 45 mg of *N*-[5-methoxy-6-(3*R*)-(tetrahydrofuran-3-yloxy)-pyridin-2-yl]-piperidin-4-yl-pyridin-3-ylmethylamine as a tan solid. ¹H NMR (300 MHz, CDCl₃) δ 8.51 (s, 1H), 8.47 (d, J=3.4Hz, 1H), 7.54 (d, J=7.9Hz, 1H), 7.21 (m, 1H), 7.02 (d, J=8.5Hz, 1H), 5.83 (d, J=8.5Hz, 1H), 5.30 (m, 1H), 4.57 (s, 2H), 4.6-4.3 (m, 2H), 4.0-3.8 (m, 4H), 3.75 (s, 3H), 3.28 (m, 2H), 2.81 (m, 2H), 2.13 (m, 2H), 1.9-1.6 (m, 4H).

The following compounds were prepared in a similar manner as described above.
a) *N*-[5-Methoxy-6-(3*R*)-(tetrahydrofuran-3-yloxy)-pyridin-2-yl]-*N*-piperidin-3-yl-pyridin-3-ylmethylamine,
b) *N*-[5-Methoxy-6-(3*R*)-(tetrahydrofuran-3-yloxy)-pyridin-2-yl]-*N-*piperidin-4-ylmethyl-pyridin-3-ylmethylamine,
c) (6-Cyclopentyloxy-5-methoxy-pyridin-2-yl)-piperidin-4-yl-pyridin-3-ylmethyl-amine hydrochloride,
d) (6-Cyclopentyloxy-5-methoxy-pyridin-2-yl)-piperidin-4-yl-pyridin-4-ylmethylamine,
e) (6-Cyclopropylmethoxy-5-methoxy-pyridin-2-yl)-piperidin-4-yl-pyridin-3-ylmethylamine,
f) 6-(cyclopentyloxy)-5-methoxy-N-piperidin-3-yl-N-(pyridin-3-ylmethyl)pyridin-2-amine oxalate,
g) 6-isopropoxy-5-methoxy-N-piperidin-4-yl-N-(pyridin-3-ylmethyl)pyridin-2-amine oxalate,
h) 6-(cyclopropylmethoyx)-5-(difluoromethoxy)-N-piperidin4-yl-N-(pyridin-3-ylmethyl)pyridin-2-amine trifluoroacetate,
i) 6-(cyclopentyloxy)-5-methoxy-N-phenyl-N-piperidin-4-ylpyridin-2-amine,
j) 6-(cyclopentyloxy)-5-methoxy-N-piperidin4-yl-N-(pyrimidin-5-ylmethyl)pyridin-2-amine.

### Examples 12A

### N-(1-Benzenesulfonylpiperidin-4-yl)-N-[5-methoxy-6-(3R)-(tetrahydrofuran-3-yloxy)-pyridin-2-yl]-pyridin-3-ylmethylamine

A solution of *N*-[5-methoxy-6-(3*R*)-(tetrahydrofuran-3-yloxy)-pyridin-2-yl]-piperidin4-yl-pyridin-3-ylmethylamine (10 mg) in dichloromethane (1 mL) and pyridine (0.12 mL) was added to a vial containing benzenesulfonyl chloride (10 mg) and the mixture was stirred 18 h. The mixture was partitioned between EtOAc and water. The EtOAc was separated, washed with brine, dried (MgSO₄) and concentrated. The residue was purified by column chromatography to yield 4 mg of *N*-(1-benzenesulfonylpiperidin-4-yl)-*N*-[5-methoxy-6-(3R)-(tetrahydrofuran-3-yloxy)-pyridin-2-yl]-pyridin-3-ylmethylamine as a reddish brown solid. ¹H NMR (300 MHz, CDCl₃) δ 8.49 (br, 2H), 7.78 (d, J=7.0Hz, 2H), 7.7-7.5 (m, 4H), 7.20 (m, 1H), 6.98 (d, J=8.5, 1H), 5.83 (d, J=8.5, 1H), 5.22 (m, 1H), 4.51 (s, 2H), 4.12 (m, 1H), 4.0-3.8 (m, 2H), 3.8-3.7 (m, 2H), 3.73 (s, 3H), 2.37 (m, 2H), 2.2-1.9 (m, 3H), 1.9-1.6 (m, 5H).

The following compounds were prepared in a similar fashion as described above.
a) *N*-(1-Benzenesulfonylpiperidin-3-yl)-N-[5-methoxy-6-(3*R*)-(tetrahydrofuran-3-yloxy)pyridin-2-yl]-pyridin-3-ylmethylamine,
b) *N*-(1-Methanesulfonylpiperidin-3-yl)-*N*-[5-methoxy-6-(3*R*)-(tetrahydrofuran-3-yloxy)-pyridin-2-yl]-pyridin-3-ylmethylamine,
c) 1-(4-{*N*-[5-Methoxy-6-(3*R*)-(tetrahydrofuran-3-yloxy)-pyridin-2-yl]-pyridin-3-ylmethylamino}-*N*-piperidin-1-yl)ethanone,
d) {4-[(6-Cylopentyloxy-5-methoxy-pyridin-2-yl)-pyridin-3-ylmethyl-amino]-piperidin-1-yl}-(4-fluoro-phenyl)-methanone,
e) (6-Cyclopentyloxy-5-methoxy-pyidin-2-yl)-(1-methanesulfonyl-piperidin-4-yl)-pyridin-3-ylmethyl-amine,
f) 6-(cyclopentyloxy)-5-methoxy-N-piperidin-3-yl-N-(pyridin-3-ylmethyl)pyridin-2-amine oxalate,
g) 6-isopropoxy-5-methoxy-N-piperidin4-yl-N-(pyridin-3-ylmethyl)pyridin-2-amine oxalate,
h) 6-(cyclopropylmethoxy)-5-(difluoromethoxy)-N-piperidin-4-yl-N-(pyridin-3-ylmethyl)pyridin-2-amine trifluoroacetate,
i) 6-(cyclopentyloxy)-5-methoxy-N-phenyl-N-piperidin-4-ylpyridin-2-amine,
j) 6-(cyclopentyloxy)-5-methoxy-N-piperidin-4-yl-N-(pyrimidin-5-ylmethyl)pyridin-2-amine.

### Example 12B

### N-methyl-(6-Cyclopentyloxy-5-methoxy-pyridin-2-yl)-piperidin-4-yl-pyridin-3-ylmethyl-amine

To a solution of (6-cyclopentyloxy-5-methoxy-pyridin-2-yl)-piperidin-4-yl-pyridin-3-ylmethylamine (100 mg) in dimethylacetamide (5 mL) and Cs CO₃ (200 mg) was added methyliodide (15 µL). This mixture was heated at 60 °C for one hour as all the starting material was consumed. The mixture was poured into ice water and ethyl acetate. The organic layer was separated, dried (Na₂SO₄) and concentrated. The resulting brown residue was purified by HPLC to give 10 mg of N-methyl-(6-cyclopentyloxy-5-methoxy-pyridin-2-yl)-piperidin-4-yl-pyridin-3-ylmethyl-amine. MS: (ES) *m*/*z* 397 (M+H⁺).

### Example 13

### 4-Fluoro-{N-4-[N-(3-fluoro4-methoxyphenyl)-pyridin-3-ylmethylamino]-benzoyl}benzenesulfonamide

A mixture of 4-[*N*-(3-fluoro-4-methoxyphenyl)-pyridin-3-ylmethylamino]benzoic acid (50 mg, 0.14 mmol), 4-fluorobenzenesulfonamide (49 mg, 0.28 mmol), 1-(3-dimethylaminopropyl)-3-ethylcarbodiimide hydrochloride (54 mg, 0.28 mmol), and dimethylaminopyridine (35 mg, 0.28 mmol) was taken up in dichloromethane (1 mL) and stirred for 18h. The mixture was partitioned between EtOAc (50 mL) and 20% aqueous NH₄OAc. The EtOAc was separated, washed with brine, dried (MgSO₄) and concentrated. The residue was purified by column chromatography (silica gel) eluting with 100% EtOAc to give 38 mg of 4-fluoro-{N-4-[N-(3-fluoro-4-methoxyphenyl)-pyridin-3-ylmethylamino]-benzoyl}benzenesulfonamide as a white solid. ¹H NMR (300 MHz, CDCl₃) δ 8.5-8.4 (m, 2H), 8.11 (m, 2H), 7.7-7.5 (m, 3H), 7.25 (m, 1H), 7.16 (m, 2H), 7.0-6.8 (m, 3H), 6.63 (d, J=9.0, 2H), 4.92 (s, 2H), 3.89 (s, 3H).

### Example 14A

### 3-[{4-Fluoro-3-(R)-(tetrahydrofuran-3-yloxy)-phenyl}-pyridin-3-ylmethyl-amino]-benzoic acid

To a cooled solution of 3-[{4-fluoro-3-methoxy-phenyl}-pyridine-3-methyl-amino]-benzoic acid ethyl ester (0.3g, 0.78 mmol) in dichloromethane (20 mL) at 0 °C was added a solution of BBr₃ (5 mL, 1 M in CH₂Cl₂) and the mixture was then allowed to warm to room temperature and stirred for one hour. The reaction was then quenched cautiously with MeOH and concentrated under reduced pressure. The residue was dissolved in methanol (30 mL) and conc. HCl (1 mL) was added and heated at reflux overnight. The reaction mixture was cooled and concentrated. The residue was basified with aq. NaHCO₃ solution and extracted with EtOAc. The organic layer was washed with water, brine, dried (Na₂SO₄) and concentrated. The residue was purified by flash column chromatography (silica, 20% acetone in CH₂Cl₂) to provide 0.12 g of 3-[{4-fluoro-3-hydroxy-phenyl}-pyridine-3-methyl-amino]-benzoic acid methyl ester.

### Example 14B

### 3-[{4-Fluoro-3-(R)-(tetrahydrofuran-3-yloxy)-phenyl}-pyridin-3-ylmethyl-amino]-benzoic acid methyl ester

To a solution of 3-[{4-fluoro-3-hydroxy-phenyl}pyridine-3-methyl-amino]-benzoic acid methyl ester (0.12g) in dichloromethane (10 mL) at room temperature was added (*S*)-3-hydroxytetrahydrofuran (0.03 g), PPh₋₃ (0.13 g) and followed by di-*tert*-butylazodicarboxylate (0.12 g). The reaction mixture was allowed to stir at room temperature for 15 min before the addition of MP-TsOH resin (0.3 g). The reaction mixture was stirred for 20 min and the resin was collected by filtration, washed with CH₂Cl₂. The product was then released with 10% Et₃N in CH₂Cl₂ and concentrated to give 0.12 g of 3-[{4-fluoro-3-(*R*)-(tetrahydrofuran-3-yloxy)-phenyl}-pyridine-3-methyl-amino]-benzoic acid methyl ester.

### Example 14C

### 3-[{4-Fluoro-3-(R)(tetrahydrofuran-3-yloxy)-phenyl}-pyridin-3-ylmethyl-amino]-benzoic acid

A mixture of 3-[{4-fluoro-3-(*R*)-(tetrahydrofuran-3-yloxy)-phenyl}-pyridine-3-methyl-amino]-benzoic acid methyl ester and LiOH (50 mg) in a mixture of THF (2 mL) and water (2 mL) was heated at 60 °C over night. The mixture was cooled and acidified with dilute aq. HCl before it was extracted with ethyl acetate. The extract was washed with water, brine, dried (Na₂SO₄) and concentrated. The residue was purified by flash column chromatography (3%MeOH and 0.5% AcOH in CH₂Cl₂) to give 3-[{4-fluoro-3-(*R*)-(tetrahydrofuran-3-yloxy)-phenyl}-pyridine-3-methyl-amino]-benzoic acid. MS: (ES) *m*/*z* 409 (M+H⁺).

The following compounds were prepared in a similar manner as described above:
a) 4-[{4-Fluoro-3-(*R*)-(tetrahydrofuran-3-yloxy)-phenyl}-pyridin-3-ylmethyl-amino]-benzoic acid
b) 3-[(3-Cyclopentyloxy-4-fluoro-phenyl)-pyridin-3-ylmethyl-amino]-benzoic acid
c) 4-[(3-Cyclopentyloxy-4-fluoro-phenyl)-pyridin-3-ylmethyl-amino]-benzoic acid
d) 3-[(4-Fluoro-3-methoxy-phenyl)-pyridin-3-ylmethyl-amino]-benzoic acid
e) 4-[(4-Fluoro-3-methoxy-phenyl)-pyridin-3-ylmethyl-amino]-benzoic acid
f) 3-[(3-Fluoro-benzyl)-(4-fluoro-3-methoxy-phenyl)-amino]-benzoic acid
g) 3-[(4-Fluoro-3-methoxy-phenyl)-pyridin-4-ylmethyl-amino]-benzoic acid
h) 3-[(4-Acetyl-3-methoxy-phenyl)-pyridin-3-ylmethyl-amino]-benzoic acid

### Example 15A

2**-Methoxy4[(thiazol-5-ylmethyl)-amino]-benzoic acid methyl ester** To a solution of methyl 4-amino-2-methoxybenzoate (1.50 g, 8.3 mmol) in dichloroethane (30 mL) was added to thiazole-5-carboxaldehyde (0.98 g, 8.7 mmol) and followed by a few drops of acetic acid. The reaction was stirred at room temperature for 1 h and followed by addition of sodium triacetoxyborohydride (3.76 g, 17.7 mmol) in portions. The resulting reaction mixture was stirred at room temperature overnight. The reaction was quenched with water and 1N aq. NaOH solution and extracted with dichloromethane (3x). The combined organic extracts were dried (Na₂SO₄) and concentrated. The crude was purified by flash column chromatography on silica gel to give 2-methoxy-4-[(thiazol-5-ylmethyl)-amino]-benzoic acid methyl ester as a tan colored oil in 90% yield (2.04 g). ¹H NMR (300 MHz, CDCl₃) 3.83 (s, 3H), 3.84 (s, 3H), 4.64 (d, J=1.0Hz, 2H), 6.16 (d, J= 2.2Hz, 1H), 6.23 (dd, J=2.2, 8.6Hz, 1H), 7.78 (d, J=8.6Hz, 1H), 7.83 (d, J=0.8Hz, 1H), 8.75 (d, J=0.7Hz, 1H).

The following compounds were prepared in a similar manner as described above.
a) 3-[(Thiazol-5-ylmethyl)-amino]-benzoic acid *tert*-butyl ester
b) 4-[(Thiazol-5-ylmethyl)-amino]-benzoic acid *tert*-butyl ester
c) (3-Fluoro-4-methoxy-phenyl)-pyridin-3-ylmethyl-amine
d) (3-Fluoro-4-methoxy-phenyl)-pyridin-4-ylmethyl-amine
e) (3-Fluoro-benzyl)-(3-fluoro-4-methoxy-phenyl)-amine
f) 2-Methoxy-4-[(pyridin-3-ylmethyl)-amino]-benzoic acid methyl ester
g) 2-Methoxy-5-[(pyridin-3-ylmethyl)-amino]-benzoic acid methyl ester
h) 1-{2-Methoxy-4-[(pyridin-3-ylmethyl)-amino]-phenyl}-ethanone
i) (4-Methoxy-phenyl)-pyridin-3-ylmethyl-amine
j) N-{2-Methoxy-5-[(pyridin-3-ylemthyl)-amino]-phenyl}-isobutyramide

### Example 15B

### 4-[(4-tert-Butoxycarbonyl-phenyl)-thiazol-5-ylmethyl-amino]-2-methoxy-benzoic acid methyl ester

A round-bottom flask containing 2-methoxy-4-[(thiazol-5-ylmethyl)-amino]-benzoic acid methyl ester (698 mg, 2.51 mmol) and *tert*-Butyl 4-bromobenzoate (1.04 g, 4.05 mmol) were purged with argon for 10 minutes and followed by addition of toluene (5 mL) and DME (5 mL). The resulting solution was then transferred to a schlenk flask containing Pd₂(dba)₃ (117 mg, 0.128 mmol) and powdered sodium hydroxide (200 mg, 5.0 mmol) under argon atmosphere. Tri-*tert-*butyl phosphine (10% wt in hexane solution, 1.41 mL, 0.539 mmol) was then added to the schlenk flask. The resulting mixture was heated at 60 °C overnight before it was cooled and filtered through a plug of celite and concentrated. The crude was purified by flash column chromatography on silica gel to give 710 mg of 4-[(4-*tert*-butoxycarbonyl-phenyl)-thiazol-5-ylmethyl-amino]-2-methoxy-benzoic acid methyl ester as an orange oil (62%). ¹H NMR (300 MHz, CDCl₃) 1.59 (s, 9H), 3.76 (s, 3H), 3.86 (s, 3H), 5.22 (s, 2H), 6.58 (d, J=2.2, 1H), 6.63 (dd, J=2.2, 8.5, 1H), 7.12 (m, 2H), 7.76 (bs, 1H), 7.78 (d, J=8.6, 1H), 7.94 (m, 2H), 8.70 (s, 1H).

The following compounds were prepared in a similar manner as described above:
a) 4-[(3-*tert*-Butoxycarbonyl-phenyl)-thiazol-5-ylmethyl-amino]-2-methoxy-benzoic acid methyl ester
b) 4-[(3-*tert*-Butoxycarbonyl-phenyl)-pyridin-5-ylmethyl-amino]-2-methoxy-benzoic acid methyl ester
c) 4-[(3-Chloro-phenyl)-pyridin-3-ylmethyl-amino]-2-methoxy-benzoic acid methyl ester
d) 5-[(3-Chloro-phenyl)-pyridin-3-ylmethyl-amino]-2-methoxy-benzoic acid methyl ester
e) 1-{4-[(3-Chloro-phenyl)-pyridin-3-ylmethyl-amino]-2-methoxy-phenyl}-ethanone
f) 3-[(4-Acetyl-3-methoxy-phenyl)-pyridin-3-ylmethyl-amino]-benzoic acid *tert*-butyl ester
g) 4-[(3-Fluoro-4-methoxy-phenyl)-thiazol-5-ylmethyl-amino]-benzoic acid *tert*-butyl ester
h) 3-[(3-Fluoro-4-methoxy-phenyl)-thiazol-5-ylmethyl-amino]-benzoic acid *tert*-butyl ester
i) 3-[(4-Fluoro-3-methoxy-phenyl)-pyridin-3-ylmethyl-amino]-benzoic acid ethyl ester
j) 4-[(4-Fluoro-3-methoxy-phenyl)-pyridin-3-ylmethyl-amino]-benzoic acid ethyl ester
k) 3-[(4-Fluoro-3-methoxy-phenyl)-pyridin-4-ylmethyl-amino]-benzoic acid ethyl ester
l) 3-[(3-Fluoro-benzyl)-(4-fluoro-3-methoxy-phenyl)-amino]-benzoic acid ethyl ester
m) 3-[(3-Isobutyrylamino-4-methoxy-phenyl)-pyridin-3-ylmethyl-amino]-benzoic acid *tert*-butyl ester

### Example 15C

### 4-[(4-tert-butoxycarbonyl-phenyl)-thiazol-5-ylmethyl-amino]-2-methoxy-benzoic acid

To a solution of 4-[(4-*tert*-butoxycarbonyl-phenyl)-thiazol-5-ylmethyl-amino]-2-methoxybenzoic acid methyl ester (710 mg, 1.56 mmol) in a mixture of methanol-water-tetrahydrofuran (6 mL, 1:1:1/v:v:v) was added LiOH•H2O (139 mg, 3.31 mmol). The reaction mixture was heated at 60 °C overnight. The organic volatiles were removed under reduced pressure. The aq. layer was washed with ethyl acetate and the layers were separated. The aqueous layer was neutralized with 1N aq. HCl before it was extracted with ethyl acetate (9x). The combined organic extracts were washed with brine, dried (MgSO₄), and concentrated to give 462 mg of 4-[(4-*tert*-butoxycarbonyl-phenyl)-thiazol-5-ylmethyl-amino]-2-methoxy-benzoic acid as a foam (67% yield).

### Example 15D

### 4-[(4-carbamoyl-3-methoxy-phenyl)-thiazol-5-ylmethyl-amino]-benzoic acid tert-butyl ester

To a solution of 4-[(4-*tert*-butoxycarbonyl-phenyl)-thiazol-5-ylmethyl-amino]-2-methoxybenzoic acid (462 mg, 1.05 mmol) in dichloromethane (20 mL) at room temperature was added oxalyl chloride (225 µL, 2.62 mmol) dropwise and followed by DMF (2 drops). The resulting mixture was stirred at room temperature for 1 h before it was concentrated to dryness to give a yellowish foam. This crude was then dissolved in THF (10 mL) and followed by addition of NH₄OH (4 mL) at room temperature. The reaction mixture was stirred overnight before it was diluted with EtOAc. The organic layer was washed with H₂O, brine, dried (MgSO₄) and concentrated. The crude was chromatographed on silica gel to give 371 mg of the desired benzamide as a white foam (80% yield). MS: (ES) *m*/*z* 440.3 (M+H⁺).

The following compounds were prepared in a similar manner as described above.
a) 3-[(4-Carbamoyl-3-methoxy-phenyl)-thiazol-5-ylmethyl-amino]-benzoic acid *tert*-butyl ester
b) 3-[(4-Carbamoyl-3-methoxy-phenyl)-pyridin-3-ylmethyl-amino]-benzoic acid *tert*-butyl ester
c) 4-[(3-Chloro-phenyl)-pyridin-3-ylmethyl-amino]-2-methoxy-benzamide
d) 5-[(3-Chloro-phenyl)-pyridin-3-ylmethyl-amino]-2-methoxy-benzamide

### Example 15E

### 4-[(4-Carbamoyl-3-methoxy-phenyl)-thiazol-5-ylmethyl-amino]-benzoic acid

To a solution of 4-[(4-carbamoyl-3-methoxy-phenyl)-thiazol-5-ylmethyl-amino]-benzoic acid *tert*-butyl ester (371 mg, 0.845 mmol) in CH₂Cl₂ (3 mL) at room temperature was added trifluoroacetic acid (260 µL, 3.375 mmol). The reaction mixture was heated at 110 °C for 1 h in a microwave before it was concentrated in vacuo and chromatographed on silica gel to give 214 mg of 4-[(4-carbamoyl-3-methoxy-phenyl)-thiazol-5-ylmethyl-amino]-benzoic acid as a white solid in 66% yield. MS: (ES) *m*/*z* 384 (M+H⁺). MP 196.7-196.8 °C. ¹H NMR (300 MHz, DMSO-*d*₆) 3.83 (s, 3H), 5.38 (s, 2H), 6.79 (dd, J=2.1, 8.5Hz, 1H), 6.89 (d, J=2.1Hz, 1H), 7.14 (m, 2H), 7.43 (bs, 1H), 7.54 (bs, 1H), 7.78-7.88 (m, 4H), 8.97 (s, 1H), 12.59 (bs, 1H).

The following compounds were prepared in a similar manner as described above.
a) 4-[(4-Carbamoyl-3-methoxy-phenyl)-thiazol-5-ylmethyl-amino]-benzoic acid
b) 3-[(4-Carbamoyl-3-methoxy-phenyl)-pyridin-3-ylmethyl-amino]-benzoic acid
c) 3-[(3-Methoxy-4-methylcarbamoyl-phenyl)-pyridin-3-ylmethyl-amino]-benzoic acid
d) 4-[(3-fluoro-4-methoxy-phenyl)-thiazol-5-ylmethyl-amino]-benzoic acid
e) 3-[(3-Fluoro-4-methoxy-phenyl)-thiazol-5-ylmethyl-amino]-benzoic acid
f) 3-[(3-Isobutyroylamino-4-methoxy-phenyl)-pyridin-3-ylmethyl-amino]-benzoic acid

### Example 16

### In Vitro Measurement of Type 4 Phosphodiesterase Inhibition Activity

Human PDE4 was obtained from baculovirus-infected Sf9 cells that expressed the recombinant enzyme. The cDNA encoding hPDE-4D6 was subcloned into a baculovirus vector.

Insect cells (Sf9) were infected with the baculovirus and cells were cultured until protein was expressed. The baculovirus-infected cells were lysed and the lysate was used as source of hPDE-4D6 enzyme. The enzyme was partially purified using a DEAE ion exchange chromatography. This procedure can be repeated using cDNA encoding other PDE-4 enzymes.

### Assay:

Type 4 phosphodiesterases convert cyclic adenosine monophosphate (cAMP) to 5'-adenosine monophosphate (5'-AMP). Nucleotidase converts 5'-AMP to adenosine. Therefore the combined activity of PDE4 and nucleotidase converts cAMP to adenosine. Adenosine is readily separated from cAMP by neutral alumina columns. Phosphodiesterase inhibitors block the conversion of cAMP to adenosine in this assay; consequently, PDE4 inhibitors cause a decrease in adenosine.

Cell lysates (40 ul) expressing hPDE-4D6 were combined with 50 ul of assay mix and 10 µl of inhibitors and incubated for 12 min at room temperature. Final concentrations of assay components were: 0. 4 ug enzyme, 10mM Tris-HCl (pH 7.5), 10mM MgCl₂, 3 uM cAMP, 0.002 U 5'-nucleotidase, and 3 x 10⁴ cpm of [3H]cAMP. The reaction was stopped by adding 100 µl of boiling 5mN HCl. An aliquot of 75 µl of reaction mixture was transferred from each well to alumina columns (Multiplate; Millipore). Labeled adenosine was eluted into an OptiPlate by spinning at 2000 rpm for 2 min; 150 µl per well of scintillation fluid was added to the OptiPlate. The plate was sealed, shaken for about 30 min, and cpm of [³H]adenosine was determined using a Wallac Triflux^{®}.

All test compounds are dissolved in 100% DMSO and diluted into the assay such that the final concentration of DMSO is 0.1%. DMSO does not affect enzyme activity at this concentration.

A decrease in adenosine concentration is indicative of inhibition of PDE activity. pIC₅₀ values were determined by screening 6 to 12 concentrations of compound ranging from 0.1 nM to 10,000 nM and then plotting drug concentration versus ³H-adenosine concentration. Nonlinear regression software (Assay Explorer^{®}) was used to estimate pIC₅₀ values.

IC₅₀ values for the preferred compounds of the invention are less than 1000 nM, especially less than 100 nM.

### Example 17 (Method A)

### Passive Avoidance in Rats, an in vivo Test for Learning and Memory

The test was performed as previously described (Zhang, H.-T., Crissman, A.M., Dorairaj, N.R, Chandler, L.J., and O'Donnell, J.M., Neuropsychopharmacology, 2000, 23,198-204.). The apparatus (Model E10-16SC, Coulbourn Instruments, Allentown, PA) consisted of a two-compartment chamber with an illuminated compartment connected to a darkened compartment by a guillotine door. The floor of the darkened compartment consisted of stainless steel rods through which an electric foot-shock could be delivered from a constant current source. All experimental groups were first habituated to the apparatus the day before the start of the experiment. During the training, the rat (Male Spraque-Dawley (Harlan) weighing 250 to 350 g) was placed in the illuminated compartment facing away from the closed guillotine door for 1 minute before the door was raised. The latency for entering the darkened compartment was recorded. After the rat entered the darkened compartment, the door was closed and a 0.5 mA electric shock was administered for 3 seconds. Twenty-four hours later, the rat was administered 0.1 mg/kg MK-801 or saline, 30 minutes prior to the injection of saline or test compound (dosed from 0.1 to 2.5 mg/kg, i.p.), which was 30 minutes before the retention test started. The rat was again placed in the illuminated compartment with the guillotine door open. The latency for entering the darkened compartment was recorded for up to 180 seconds, at which time the trial was terminated.

All data were analyzed by analyses of variance (ANOVA); individual comparisons were made using Kewman-Keuls tests. Naive rats required less than 30 seconds, on average, to cross from the illuminated compartment to the darkened compartment. However, 24 hours after the electric shock exposure, most rats pretreated with vehicle did not re-enter the darkened compartment; the average latency was increased up to 175 seconds (p < 0.001). Pretreatment with MK-801 (0.1 mg/kg) markedly reduced this latency when compared to the vehicle (p<0.001). This amnesic effect of MK-801 is reversed in a statistically significant manner by actual test compounds in a dose-dependent fashion.

### Example 17 (Method B)

### Radial arm maze task in Rats, an in vivo Test for Learning and Memory

The test was performed as previously described (Zhang, H.-T., Crissman, A.M., Dorairaj, N.R., Chandler, L.J., and O'Donnell, J.M., Neuropsychopharmacology, 2000, 23, 198-204.). Five days after initial housing, rats (male Spraque-Dawley (Harlan) weighing 250 to 350 g) were placed in the eight-arm radial maze (each arm was 60x10x12 cm high; the maze was elevated 70 cm above the floor) for acclimation for two days. Rats were then placed individually in the center of the maze for 5 minutes with food pellets placed close to the food wells, and then, the next day, in the wells at the end of the arms; 2 sessions a day were conducted. Next, four randomly selected arms were then baited with one pellet of food each. The rat was restricted to the center platform (26 cm in diameter) for 15 seconds and then allowed to move freely throughout the maze until it collected all pellets of food or 10 minutes passed, whichever came first. Four parameters were recorded: 1) working memory errors, i.e., entries into baited arms that had already been visited during the same trial; 2) reference memory errors, i.e., entries into unbaited arms; 3) total arm entries; and 4) the test duration (seconds), i.e., the time spent in the collection of all the pellets in the maze. If the working memory error was zero and the average reference memory error was less than one in five successive trials, the rats began the drug tests. MK-801 or saline was injected 15 minutes prior to vehicle or test agent, which was given 45 minutes before the test. Experiments were performed in a lighted room, which contained several extra-maze visual cues.

All data were analyzed by analyses of variance (ANOVA); individual comparisons were made using Kewman-Keuls tests. Compared to control, MK-801 (0.1 mg/kg, i.p.) increased the frequencies of both working and reference memory errors (p<0.01). This amnesic effect of MK-801 on working memory is reversed in a statistically significant manner by the administration of actual test compounds in a dose-dependent fashion.

The preceding examples can be repeated with similar success by substituting the generically or specifically described reactants and/or operating conditions of this invention for those used in the preceding examples.

While the invention has been illustrated with respect to the production and of particular compounds, it is apparent that variations and modifications of the invention can be made without departing from the spirit or scope of the invention as defined in the claims.

## Claims

1. A compound of Formula I: 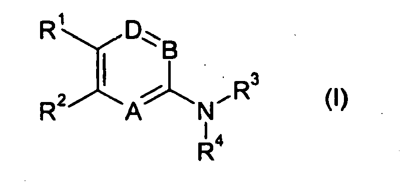 wherein
A, B and D are each N or CR⁵ wherein one of A, B and D is N;
R¹ is halogen, alkyl having 1 to 4 carbon atoms, halogenated alkyl having 1 to 4 carbon atoms, OR⁶, COR⁶, CONR⁶, or NR⁶COR¹⁰;
R² is halogen, alkyl having 1 to 4 carbon atoms, halogenated alkyl having 1 to 4 carbon atoms, OR⁷, COR⁶, CONR⁶, or NR⁶COR¹⁰;
R³ is alkyl having 1 to 8 which is branched or unbranched and which is unsubstituted or substituted one or more times by halogen, cyano, C₁₋₄-alkoxy, or combinations thereof, a partially unsaturated carbocycle-alkyl group wherein the carbocyclic portion has 5 to 14 carbon atoms and the alkyl portion which is branched or unbranched has 1 to 5 carbon atoms, and which is unsubstituted, substituted in the carbocyclic portion one or more times by halogen, alkyl, alkoxy, nitro, cyano, oxo, or combinations thereof, and/or substituted in the alkyl portion one or more times by halogen, C₁₋₄-alkoxy, cyano or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, or heterocycle-alkyl group, wherein the heterocycle portion is unsaturated, partially saturated or fully saturated and has 5 to 10 ring atoms in which at least 1 ring atom is an N, N-O, O or S, the alkyl portion, which is branched or unbranched, has 1 to 5 carbon atoms, the heterocycle-alkyl group is unsubstituted, substituted one or more times in the heterocycle portion by halogen, alkyl, alkoxy, cyano, trifluoromethyl, CF₃O, nitro, oxo, amino, alkylamino, dialkylamino, or combinations thereof and/or substituted in the alkyl portion one or more times by halogen, cyano, alkyl having 1 to 4 carbon atoms, or combinations thereof;
R⁴ is cycloalkyl having 3 to 10 carbon atoms, which is unsubstituted or substituted one or more times by halogen, hydroxy, oxo, cyano, alkyl having 1 to 4 carbon atoms, alkoxy having 1 to 4 carbon atoms, or combinations thereof, aryl having 6 to 14 carbon atoms and which is unsubstituted or substituted one or more times by halogen, alkyl, alkenyl, alkynyl, hydroxy, alkoxy, alkoxyalkoxy, nitro, methylenedioxy, ethylenedioxy, trifluoromethyl, OCF₃, amino, aminoalkyl, aminoalkoxy, dialkylamino, hydroxyalkyl, hydroxamic acid, pyrrolyl, tetrazole-5-yl, 2(-heterocycle)tetrazole-5-yl, hydroxyalkoxy, carboxy, carboxyalkyl, alkoxycarbonyl, cyano, acyl, alkylthio, alkylsulfinyl, alkylsulfonyl, phenoxy, trialkylsilyloxy, R⁸-L-, or combinations thereof, heteroaryl having 5 to 10 ring atoms in which at least 1 ring atom is a heteroatom, which is unsubstituted or substituted one, two or three times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, carboxyalkyl, alkoxycarbonyl, cyano, acyl, alkylthio, alkylsulfinyl, alkylsulfonyl, phenoxy, trialkylsilyloxy, R⁸-L-, or combinations thereof, a heterocyclic group, which is saturated or partially saturated, having 5 to 10 ring atoms in which at least 1 ring atom is an N, O or S atom, which is unsubstituted or substituted one or more times by halogen, alkyl, hydroxy, alkoxy, alkoxyalkoxy, nitro, oxo, methylenedioxy, ethylenedioxy, trifluoromethyl, OCF₃,amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenylsulfonyl, phenoxy, cycloalkyl, aryl, heteroaryl or combinations thereof, a heterocycle-alkyl group, wherein the heterocyclic portion is saturated, partially saturated or unsaturated, and has 5 to 10 ring atoms in which at least 1 ring atom is an N, O or S atom, and the alkyl portion is branched or unbranched and has 1 to 5 carbon atoms, the heterocycle-alkyl group is unsubstituted, substituted one or more times in the heterocyclic portion by halogen, alkyl, hydroxy, alkoxy, alkoxyalkoxy, nitro, oxo, methylenedioxy, ethylenedioxy, trifluoromethyl, OCF₃, amino, aminomethyl, aminoalkyl, aminoalkoxy, dialkylamino, hydroxyalkyl; hydroxamic acid, tetrazole-5-yl, hydroxyalkoxy, carboxy, alkoxycarbonyl, cyano, acyl, alkylthio, alkylsulfinyl, alkylsulfonyl, phenylsulfonyl, phenoxy, cycloalkyl, aryl, heteroaryl or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH;
R⁵ is H, halogen, alkyl having 1 to 4 carbon atoms, halogenated alkyl having 1 to 4 carbon atoms, alkoxy having 1 to 4 carbon atoms, or halogenated alkoxy having 1 to 4 carbon atoms;
R⁶ is H or alkyl having 1 to 4 carbon atoms, which is branched or unbranched and which is unsubstituted or substituted one or more times by halogen;
R⁷ is H or alkyl having 1 to 12 which is branched or unbranched and which is unsubstituted or substituted one or more times by halogen, hydroxy, cyano, C₁₋₄-alkoxy, oxo or combinations thereof, and wherein optionally one or more -CH₂CH₂- groups is replaced in each case by -CH=CH- or -C≡C-, cycloalkyl having 3 to 10 which is unsubstituted or substituted one or more times by halogen, hydroxy, oxo, cyano, alkyl having 1 to 4 carbon atoms, alkoxy having 1 to 4 carbon atoms, or combinations thereof, cycloalkylalkyl having 4 to 16 which is unsubstituted or substituted in the cycloalkyl portion and/or the alkyl portion one or more times by halogen, oxo, cyano, hydroxy, C₁₋₄-alkyl, C₁₋₄- alkoxy or combinations thereof, aryl having 6 to 14 carbon atoms which is unsubstituted or substituted one or more times by halogen, CF₃, OCF₃, alkyl, hydroxy, alkoxy, nitro, methylenedioxy, ethylenedioxy, cyano, or combinations thereof, arylalkyl in which the aryl portion has 6 to 14 carbon atoms and the alkyl portion, which is branched or unbranched, has 1 to 5 carbon atoms, wherein the arylalkyl radical is unsubstituted, substituted in the aryl portion one or more times by halogen, CF₃, OCF₃, alkyl, hydroxy, alkoxy, nitro, cyano, methylenedioxy, ethylenedioxy, or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-, a partially unsaturated carbocyclic group having 5 to 14 carbon atoms, which is unsubstituted or substituted one or more times by halogen, alkyl, alkoxy, hydroxy, nitro, cyano, oxo, or combinations thereof a heterocyclic group, which is saturated, partially saturated or unsaturated, having 5 to 10 ring atoms in which at least 1 ring atom is an N, O or S atom, which is unsubstituted or substituted one or more times by halogen, hydroxy, aryl, alkyl, alkoxy, cyano, trifluoromethyl, nitro, oxo, or combinations thereof, or a heterocycle-alkyl group, wherein the heterocyclic portion is saturated, partially saturated or unsaturated, and has 5 to 10 ring atoms in which at least 1 ring atom is an N, O or S atom, and the alkyl portion is branched or unbranched and has 1 to 5 carbon atoms, the heterocycle-alkyl group is unsubstituted, substituted one or more times in the heterocyclic portion by halogen, OCF₃, hydroxy, aryl, alkyl, alkoxy, cyano, trifluoromethyl, nitro, oxo, or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-;
R⁸ is H, alkyl having 1 to 8 which is unsubstituted or substituted one or more times by halogen, C₁₋₄- alkyl, C₁₋₄-alkoxy, oxo, or combinations thereof, alkylamino or dialkylamino wherein each alkyl portion has independently 1 to 8, a partially unsaturated carbocycle-alkyl group wherein the carbocyclic portion has 5 to 14 carbon atoms and the alkyl portion has 1 to 5 carbon atoms, and which is unsubstituted or substituted one or more times by halogen, alkyl, alkoxy, nitro, cyano, oxo, or combinations thereof, cycloalkyl having 3 to 10 which is unsubstituted or substituted one or more times by halogen, hydroxy, oxo, cyano, alkoxy, alkyl having 1 to 4 carbon atoms, or combinations thereof, cycloalkylalkyl having 4 to 16 which is unsubstituted or substituted in the cycloalkyl portion and/or the alkyl portion one or more times by halogen, oxo, cyano, hydroxy, alkyl, alkoxy or combinations thereof, aryl having 6 to 14 carbon atoms which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboy, alkoxycarbonyl, cyano, acyl, alkylthio, alkylsulfinyl, alkylsulfonyl, phenoxy, cycloalkyl, aryl, heteroaryl or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, a heterocyclic group, which is saturated, partially saturated or unsaturated, having 5 to 10 ring atoms in which at least 1 ring atom is an N, O or S atom, which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, cycloalkyl, aryl, heteroaryl or combinations thereof, or a heterocycle-alkyl group, wherein the heterocyclic portion is saturated, partially saturated or unsaturated, and has 5 to 10 ring atoms in which at least 1 ring atom is an N, O or S atom, and the alkyl portion, which is branched or unbranched, has 1 to 5 carbon atoms, the heterocycle-alkyl group is unsubstituted, substituted one or more times in the heterocyclic portion by halogen, alkyl, alkoxy, cyano, trifluoromethyl, CF₃O, nitro, oxo, amino, alkylamino, dialkylamino, or combinations thereof and/or substituted one or more times in the alkyl portion by halogen, cyano, alkyl having 1 to 4 carbon atoms, or combinations thereof;
L is a single bond or a divalent aliphatic radical having 1 to 8 carbon atoms wherein one or more - CH₂- groups are each optionally replaced by -O-, - S-, -SO-, -SO₂-, -NR⁹-, -SO₂NR⁹-, -NR⁹SO₂-, -CO-, -CO₂-, -NR⁹CO-, - CONR⁹-, -NHCONH-, -OCONH, -NHCOO-, -SCONH-, SCSNH-, -NHCSNH-, -CONHSO₂- or -SO₂NHCO-; and
R⁹ is H, alkyl having 1 to 8 carbon atoms, which is branched or unbranched and which is unsubstituted or substituted one or more times with halogen, C₁- ₄-alkyl, C₁-₄-alkoxy, oxo, or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, or aryl having 6 to 14 carbon atoms and which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, or combinations thereof; and
R¹⁰ is H or alkyl having 1 to 4 carbon atoms, which is branched or unbranched and which is unsubstituted or substituted one or more times by halogen; or
a pharmaceutically acceptable salt thereof.

2. A compound according to Formula III: 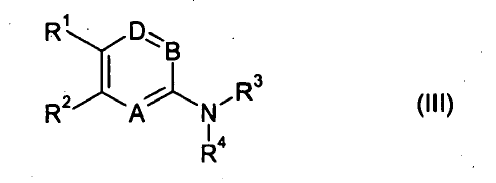 wherein
A, B and D are each N or CR⁵ and one of A, B and D is N;'
R¹ s halogen, alkyl having 1 to 4 carbon atoms, halogenated alkyl having 1 to 4 carbon atoms, or OR⁶;
R² is halogen, alkyl having 1 to 4 carbon atoms, halogenated alkyl having 1 to 4 carbon atoms, or OR⁷
R³ is arylalkyl having 7 to 19 carbon atoms wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, or heterocycle-alkyl group, wherein the heterocycle portion is unsaturated, partially saturated or fully saturated and has 5 to 10 ring atoms in which at least 1 ring atom is an N, N-O, O or S, the alkyl portion, which is branched or unbranched, has 1 to 5 carbon atoms, the heterocycle-alkyl group is unsubstituted, substituted one or more times in the heterocycle portion by halogen, alkyl, alkoxy, cyano, trifluoromethyl, CF₃O, nitro, oxo, amino, alkylamino, dialkylamino, or combinations thereof and/or substituted in the alkyl portion one or more times by halogen, cyano, alkyl having 1 to 4 carbon atoms, or combinations thereof;
R⁴ is cycloalkyl having 3 to 10 carbon atoms which is unsubstituted or substituted one or more times by halogen, hydroxy, oxo, cyano, alkyl having 1 to 4 carbon atoms, alkoxy having 1 to 4 carbon atoms, or combinations thereof, aryl having 6 to 14 carbon atoms and which is unsubstituted or substituted one or more times by halogen, alkyl, alkenyl, alkynyl, hydroxy, alkoxy, alkoxyalkoxy, nitro, methylenedioxy, ethylenedioxy, trifluoromethyl, OCF₃, amino, aminoalkyl, aminoalkoxy, dialkylamino, hydroxyalkyl, hydroxamic acid, pyrrolyl, tetrazole-5-yl, 2(-heterocycle)tetrazole-5-yl, hydroxyalkoxy, carboxy, alkoxycarbonyl, cyano, acyl, alkylthio, alkylsulfinyl, alkylsulfonyl, phenoxy, trialkylsilyloxy, R⁸-L-, or combinations thereof, heteroaryl having 5 to 10 ring atoms in which at least 1 ring atom is a heteroatom, which is unsubstituted or substituted one or more times by halogen, alkyl, hydroxy, alkoxy, alkoxyalkoxy, nitro, methylenedioxy, ethylenedioxy, trifluoromethyl; amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, trialkylsilyloxy, R⁸-L-, or combinations thereof, a heterocyclic group, which is saturated or partially saturated, having 5 to 10 ring atoms in which at least 1 ring atom is an N, O or S atom, which is unsubstituted or substituted one or more times by halogen, alkyl, hydroxy, alkoxy, alkoxyalkoxy, nitro, oxo, methylenedioxy, ethylenedioxy, trifluoromethyl, OCF₃,amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenylsulfonyl, phenoxy, cycloalkyl, aryl, heteroaryl or combinations thereof, a heterocycle-alkyl group, wherein the heterocyclic portion is saturated, partially saturated or unsaturated, and has 5 to 10 ring atoms in which at least 1 ring atom is an N, O or S atom, and the alkyl portion is branched or unbranched and has 1 to 5 carbon atoms, the heterocycle-alkyl group is unsubstituted, substituted one or more times in the heterocyclic portion by halogen, alkyl, hydroxy, alkoxy, alkoxyalkoxy, nitro, oxo, methylenedioxy, ethylenedioxy, trifluoromethyl, OCF₃, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenylsulfonyl, phenoxy, cycloalkyl, aryl, heteroaryl or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-;
R⁵ is H, halogen, alkyl having 1 to 4 carbon atoms, halogenated alkyl having 1 to 4 carbon atoms, alkoxy having 1 to 4 carbon atoms, or halogenated alkoxy having 1 to 4 carbon atoms;
R⁶ is H or alkyl having 1 to 4 carbon atoms, which is branched or unbranched and which is unsubstituted or substituted one or more times by halogen;
R⁷ is H or alkyl having 1 to 12 carbon atoms, which is branched or unbranched and which is unsubstituted or substituted one or more times by halogen, hydroxy, cyano, C₁₋₄-alkoxy, oxo or combinations thereof, and wherein optionally one or more -CH₂CH₂- groups is replaced in each case by -CH=CH- or -C≡C-, cycloalkyl having 3 to 10 carbon atoms, which is unsubstituted or substituted one or more times by halogen, hydroxy, oxo, cyano, alkyl having 1 to 4 carbon atoms, alkoxy having 1 to 4 carbon atoms, or combinations thereof, cycloalkylalkyl having 4 to 16 carbon atoms, which is unsubstituted or substituted in the cycloalkyl portion and/or the alkyl portion one or more times by halogen, oxo, cyano, hydroxy, C₁₋₄-alkyl, C₁₋₄- alkoxy or combinations thereof, aryl having 6 to 14 carbon atoms, which is unsubstituted or substituted one or more times by halogen, CF₃, OCF₃, alkyl, hydroxy, alkoxy, nitro, methylenedioxy, ethylenedioxy, cyano, or combinations thereof, arylalkyl in which the aryl portion has 6 to 14 carbon atoms and the alkyl portion, which is branched or unbranched, has 1 to 5 carbon atoms, wherein the arylalkyl radical is unsubstituted, substituted in the aryl portion one or more times by halogen, CF₃, OCF₃, alkyl, hydroxy, alkoxy, nitro, cyano, methylenedioxy, ethylenedioxy, or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-, a partially unsaturated carbocyclic group having 5 to 14 carbon atoms, which is unsubstituted or substituted one or more times by halogen, alkyl, alkoxy, hydroxy, nitro, cyano, oxo, or combinations thereof, a heterocyclic group, which is saturated, partially saturated or unsaturated, having 5 to 10 ring atoms in which at least 1 ring atom is an N, O or S atom, which is unsubstituted or substituted one or more times by halogen, hydroxy, aryl, alkyl, alkoxy, cyano, trifluoromethyl, nitro, oxo, or combinations thereof, or a heterocycle-alkyl group, wherein the heterocyclic portion is saturated, partially saturated or unsaturated, and has 5 to 10 ring atoms in which at least 1 ring atom is an N, O or S atom, and the alkyl portion is branched or unbranched and has 1 to 5 carbon atoms, then heterocycle-alkyl group is unsubstituted, substituted one or more times in the heterocyclic portion by halogen, OCF₃, hydroxy, aryl, alkyl, alkoxy, cyano, trifluoromethyl, nitro, oxo, or combinations thereof, and/or substituted in the alkyl portion one or more times by halogen, oxo, hydroxy, cyano, or combinations thereof, and wherein in the alkyl portion one or more -CH₂CH₂- groups are each optionally replaced by -CH=CH- or -C≡C-, and one or more -CH₂- groups are each optionally replaced by -O- or -NH-;
R⁸ is H, alkyl having 1 to 8 carbon atoms, which is unsubstituted or substituted one or more times by halogen, C₁₋₄-alkyl, C₁₋₄-alkoxy, oxo, or combinations thereof, alkylamino or dialkylamino wherein each alkyl portion has independently 1 to 8 carbon atoms, a partially unsaturated carbocycle-alkyl group wherein the carbocyclic portion has 5 to 14 carbon atoms and the alkyl portion has 1 to 5 carbon atoms, and which is unsubstituted or substituted one or more times by halogen, alkyl, alkoxy, nitro, cyano, oxo, or combinations thereof, cycloalkyl having 3 to 10 carbon atoms, which is unsubstituted or substituted one or more times by halogen, hydroxy, oxo, cyano, alkoxy, alkyl having 1 to 4 carbon atoms, or combinations thereof, cycloalkylalkyl having 4 to 16 carbon atoms, which is unsubstituted or substituted in the cycloalkyl portion and/or the alkyl portion one or more times by halogen, oxo, cyano, hydroxy, alkyl, alkoxy or combinations thereof, aryl having 6 to 14 carbon atoms which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, cycloalkyl, aryl, heteroaryl or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, a heterocyclic group, which is saturated, partially saturated or unsaturated, having 5 to 10 ring atoms in which at least 1 ring atom is an N, O or S atom, which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy, dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, phenoxy, cycloalkyl, aryl, heteroaryl or combinations thereof, or a heterocycle-alkyl group, wherein the heterocyclic portion is saturated, partially saturated or unsaturated, and has 5 to 10 ring atoms in which at least 1 ring atom is an N, O or S atom, and the alkyl portion, which is branched or unbranched, has 1 to 5 carbon atoms, the heterocycle-alkyl group is unsubstituted, substituted one or more times in the heterocyclic portion by halogen, alkyl, alkoxy, cyano, trifluoromethyl, CF₃O, nitro, oxo, amino, alkylamino, dialkylamino, or combinations thereof and/or substituted one or more times in the alkyl portion by halogen, cyano, alkyl having 1 to 4 carbon atoms, or combinations thereof;
L is a single bond or a divalent aliphatic radical having 1 to 8 carbon atoms wherein one or more - CH₂- groups are each optionally replaced by -O-, - S-, -SO-, -SO₂-, -NR⁹-, -SO₂NR⁹-, -R⁹SO₂-_{,} - CO-, -CO₂-, -NR⁹CO-, -CONR⁹-, -NHCONH-, -OCONH, NHCOO-, -SCONH-, -SCSNH-, -NHCSNH-, -CONHSO₂- or -SO₂NHCO-; and
R⁹ is H, alkyl having 1 to 8 carbon atoms, which is branched or unbranched and which is unsubstituted or substituted one or more times with halogen, C₁- ₄-alkyl, C₁₋₄-alkoxy, oxo, or combinations thereof, arylalkyl having 7 to 19 carbon atoms, wherein the aryl portion has 6 to 14 carbon atoms and the alkyl portion, which is branched or unbranched, has 1 to 5 carbon atoms, wherein the arylalkyl radical is unsubstituted or substituted, in the aryl portion, one or more times by halogen, trifluoromethyl, CF₃O, nitro, amino, alkyl, alkoxy, amino, alkylamino, dialkylamino, or combinations thereof, and/or substituted in the alkyl portion by halogen, cyano, alkyl having 1 to 4 carbon atoms, or combinations thereof, wherein in the alkyl portion one or more -CH₂CH₂- groups are each optionally replaced by -CH=CH- or -C≡C-, and/or one or more -CH₂- groups are each optionally replaced by -O- or -NH-, or aryl having 6 to 14 carbon atoms and which is unsubstituted or substituted one or more times by halogen, alkyl, hydroxy, alkoxy, alkoxyalkoxy, nitro, methylenedioxy, ethylenedioxy, trifluoromethyl, amino, aminomethyl, aminoalkyl, aminoalkoxy dialkylamino, hydroxyalkyl, hydroxamic acid, tetrazole-5-yl, hydroxyalkoxy, carboxy, alkoxycarbonyl, cyano, acyl, alkylthio, alkylsulfinyl, alkylsulfonyl, or combinations thereof; and
wherein R¹ is OR⁶ and/or R² is OR⁷;
or a pharmaceutically acceptable salt thereof.

3. A compound according to claim 1, wherein R¹ is OR⁶ and/or R² is OR⁷.

4. A compound according to claim 1, wherein R¹ and/or R² is COR⁶, CONR⁶, or NR⁶COR¹⁰_{.}

5. A compound according to claims 1 or 2, wherein R¹ is halogen or OR⁶ and R⁶ is alkyl or halogenated alkyl, or
wherein R² is halogen or OR⁷, and R⁷ is alkyl, cycloalkyl, cycloalkylalkyl, a heterocyclic group, or halogenated alkyl.

6. A compound according to claim 1, wherein R³ is arylalkyl or heterocycle-alkyl, which in each case is substituted or unsubstituted.

7. A compound according to claims 1 or 2, wherein R³ is benzyl or pyridylmethyl, which in each case is substituted or unsubstituted.

8. A compound according to claims 1 or 2, wherein R⁴ is cycloalkyl, aryl, heteroaryl or a heterocyclic group, which is substituted or unsubstituted.

9. A compound according to claim 8, wherein R⁴ is cycloalkyl, aryl, or a heterocyclic group, which is substituted or unsubstituted.

10. A compound according to claim 9,
wherein R⁴ is cyclohexyl, piperidinyl, or phenyl, which in each case substituted or unsubstituted, or
wherein R⁴ is phenyl substituted by carboxy, cyano, tetrazole, and/or L-R⁸.

11. A compound according to claims 1 or 2,
wherein R⁴ is at least monosubstituted by R⁸-L- and L is a single bond or a divalent aliphatic radical having 1 to 8 carbon atoms wherein at least one -CH₂- group is replaced by -SO₂NR⁹, -NR⁹-, - NR⁹CO-, - CONR⁹- -CO₂-, -CONHSO₂-, -. SO₂NHCO-, -SO₂-, or -NR⁹SO₂-,
wherein R⁸ is methyl, ethyl, propyl or phenyl, which in each case is unsubstituted or substituted,
wherein R⁹ is H, alkyl having 1 to 4 carbon atoms, or aryl, or
wherein R⁵ is H, F or methyl.

12. A compound according to claims 1 or 2, wherein A is N, B and D are each independently CR⁵, R¹ is OR⁶, R² is halogen or OR⁷, R³ is pyridylmethyl, fluorobenzyl, or 2,6-difluorobenzyl, R⁴ is aryl, cycloalkyl, or a saturated heterocyclic group, in each case substituted or unsubstituted, R⁵ is H, halogen, or alkyl which is substituted or unsubstituted, R⁶ is alkyl which is substituted or unsubstituted, and R⁷ is alkyl, cycloalkyl, cycloalkylalkyl or a saturated heterocyclic group, in each case substituted or unsubstituted.

13. A compound according to claims 1 or 2, wherein A is N, B and D are each independently CR⁵, R¹ is OR⁶, R² is halogen or OR⁷, R³ is pyridylmethyl, fluorobenzyl, 2,6-difluorobenzyl, 5-thiazolylmethyl, or 5-pyrimidinylmethyl, R⁴ is phenyl, which is unsubstituted or substituted, R⁵ is H, halogen, or alkyl which is substituted or unsubstituted, R⁶ is alkyl which is substituted or unsubstituted, and R⁷ is alkyl, cycloalkyl, cycloalkylalkyl, or a saturated heterocyclic group, in each case substituted or unsubstituted.

14. A compound according to claim 13, wherein R³ is pyridylmethyl, fluorobenzyl, 2,6-difluorobenzyl.

15. A compound according to claims 1 or 2, wherein A is N, B and D are each independently CH, R¹ is OR⁶, R² is halogen or OR⁷, R³ is pyridylmethyl, 5-thiazolylmethyl, or 5-pyrimidinylmethyl, R⁴ is unsubstituted cycloalkyl, aryl which is substituted or unsubstituted, or piperidinyl which is substituted or unsubstituted, R⁶ is unsubstituted alkyl or CHF₂, and R⁷ is alkyl, cycloalkyl, cycloalkylalkyl or tetrahydrofuranyl, in each case substituted or unsubstituted.

16. A compound according to claim 15, wherein R³ is pyridylmethyl.

17. A compound according to claims 1 or 2, wherein A is N, B and D are each independently CH, R¹ is OR⁶, R² is halogen or OR⁷, R³ is 3-pyridylmethyl, 5-thiazolylmethyl, or 5-pyrimidinylmethyl, R⁴ is cyclohexyl, phenyl which is substituted or unsubstituted, or piperidinyl which is substituted or unsubstituted, R⁶ is unsubstituted alkyl or CHF₂, and R⁷ is alkyl, cycloalkyl, cycloalkylalkyl or tetrahydrofuranyl, in each case substituted or unsubstituted.

18. A compound according to claim 17, wherein R³ is 3-pyridylmethyl.

19. A compound according to claims 1 or 2, wherein A is N and B and D are each independently CR⁵, R¹ is OR⁶, R² is OR⁷, R³ is heterocycle-alkyl, R⁴ is heterocyclic group, which is unsubstituted or substituted, R⁵ is H, halogen, or alkyl which is substituted or unsubstituted, R⁶ is alkyl, R⁷ is alkyl, cycloalkyl, or cycloalkylalkyl, in each case substituted or unsubstituted.

20. A compound selected from the following:
4-{*N*-[4-Methoxy-3-(*R*)-(tetrahydrofuran-3-yloxy)phenyl]pyridin-3-ylmethylamino}piperidine-1-carboxylic acid *tert*-butyl ester,
3-[*N*-(6-Cyclopropylmethoxy-5-methoxypyridin-2-yl)-pyridin-3-ylmethylamino]benzoic acid,
3-[*N*-(5,6-Dimethoxypyridin-2-yl)-pyridin-3-ylmethylamino]-benzoic acid,
3-[*N*-(6-Cyclobutyloxy-5-methoxypyridin-2-yl)-pyridin-3-ylmethylamino]benzoic acid,
3-[*N*-(6-Cyclopropylmethoxy-5-difluoromethoxypyridin-2-yl)-pyridin-3-ylmethylamino]benzoic acid,
3-[*N*-(5-Difluoromethoxy-6-methoxypyridin-2-yl)-pyridin-3-ylmethylamino]benzoic acid,
3-[*N*-(6-Ethoxy-5-methoxypyridin-2-yl)-pyridin-3-ylmethylamino]benzoic acid,
3-[*N*-(6-Isopropoxy-5-methoxypyridin-2-yl)-pyridin-3-ylmethylamino]benzoic acid,
3-[*N*-(5-Difluoromethoxy-6-isopropoxypyridin-2-yl)-pyridin-3-ylmethylamino]benzoic acid,
3-[*N*-(6-Cyclobutyloxy-5-difluoromethoxypyridin-2-yl)-pyridin-3-ylmethylamino]benzoic acid,
*N*-(1-Benzenesulfonylpiperidin-3-yl)-N-[5-methoxy-6-(R)-(tetrahydrofuran-3-yloxy)pyridin-2-yl]-pyridin-3-ylmethylamine,
*N*-(1-Methanesulfonylpiperidin-3-yl)-*N*-[5-methoxy-6-(*R*)-(tetrahydrofuran-3-yloxy)-pyridin-2-yl]-pyridin-3-ylmethylamine,
*N*-[5-Methoxy-6-(*R*)-(tetrahydrofuran-3-yloxy)-pyridin-2-yl]-*N*-piperidin-3-yl-pyridin-3-ylmethylamine,
*N*-[5-Methoxy-6-(*R*)-(tetrahydrofuran-3-yloxy)-pyridin-2-yl]-*N*-piperidin-4-ylmethyl-pyridin-3-ylmethylamine,
4-(*N*-{[5-Methoxy-6-(*R*)-(tetrahydrofuran-3-yloxy)-pyridin-2-yl]-pyridin-3-ylmethylamino}-methyl)-*N*-piperidine-1-carboxylic acid *tert*-butyl ester,
*N*-(1-Benzenesulfonylpiperidin-4-yl)-*N*-[5-methoxy-6-(*R*)-(tetrahydrofuran-3-yloxy)-pyridin-2-yl]-pyridin-3-ylmethylamine,
1-(4-{*N*-[5-Methoxy-6-(*R*)-(tetrahydrofuran-3-yloxy)-pyridin-2-yl]-pyridin-3-ylmethylamino}-*N*-piperidin-1-yl)ethanone,
*N*-[5-Methoxy-6-(*R*)-(tetrahydrofuran-3-yloxy)-pyridin-2-yl]-piperidin-4-yl-pyridin-3-ylmethylamine,
4-{*N*-[5-Methoxy-6-(*R*)-(tetrahydrofuran-3-yloxy)-pyridin-2-yl]-pyridin-3-ylmethylamino}-piperidine-1-carboxylic acid *tert*-butyl ester,
3-{*N*-[5-Methoxy-6-(*R*)-(tetrahydrofuran-3-yloxy)-pyridin-2-yl]-pyridin-3-ylmethylamino}-benzoic acid,
*N*-[5-Methoxy-6-(*R*)-(tetrahydrofuran-3-yloxy)-pyridin-2-yl]-pyridin-3-ylmethyl-*N*-[4-(2H-tetrazol-5-yl)phenyl]amine,
*N*-Cyclohexyl-*N*-[5'-methoxy-6-(*R*)-(tetrahydrofuran-3-yloxy)-pyridin-2-yl]-pyridin-3-ylmethylamine,
*N*-[5-Methoxy-6-(*R*)-(tetrahydrofuran-3-yloxy)-pyridin-2-yl]-*N*-phenyl-pyridin-3-ylmethylamine,
*N*-(3-Chlorophenyl)-*N*-[5-methoxy-6-(*R*)-(tetrahydrofuran-3-yloxy)pyridin-2-yl]-pyridin-3-ylmethylamine,
3-{*N*-[5-Methoxy-6-(tetrahydrofuran-3-yloxy)-pyridin-2-yl]pyridin-3-ylmethylamino}benzoic acid,
3-[*N*-(6-Cyclopentyloxy-5-methoxypyridin-2-yl)-pyridin-3-ylmethylamino]benzoic acid,
4-[*N*-(5-Cyclopentyloxy-4-methoxy-2-methylphenyl)pyridin-3-ylmethylamino]benzoic acid,
3-[*N*-(5-Cyclopentyloxy-4-methoxy-2-methylphenyl)pyridin-3-ylmethylamino]benzoic acid, and
pharmaceutically acceptable salts thereof,
wherein compounds that are optically active can be in the form of their separate enantiomers or mixtures thereof, including racemic mixtures.

21. A compound selected from the following:
(6-Cyclopentyloxy-5-methoxy-pyridin-2-yl)-piperidin-4-yl-pyridin-3-ylmethyl-amine hydrochloride,
(6-Cyclopentyloxy-5-methoxy-pyridin-2-yl)-piperidin-4-yl-pyridin-4-ylmethyl-amine,
(6-Cyclopropylmethoxy-5-methoxy-pyridin-2-yl)-piperidin-4-yl-pyridin-3-ylmethyl-amine,
{4-[(6-Cyclopentyloxy-5-methoxy-pyridin-2-yl)-pyridin-3-ylmethyl-amino]-piperidin-1-yl}-(4-fluoro-phenyl)-methanone,
(6-Cyclopentyloxy-5-methoxy-pyridin-2-yl)-(1-methanesulfonyl-piperidin-4-yl)-pyridin-3-ylmethyl-amine,
6-(cyclopentyloxy)-5-methoxy-N-piperidin-3-yl-N-(pyridin-3-ylmethyl)pyridin-2-amine,
6-(cyclopentyloxy)-5-methoxy-N-piperidin-3-yl-N-(pyridin-3-ylmethyl)pyridin-2-amine oxalate,
6-isopropoxy-5-methoxy-N-piperidin-4-yl-N-(pyridin-3-ylmethyl)pyridin-2-amine,
6-isopropoxy-5-methoxy-N-piperidin-4-yl-N-(pyridin-3-ylmethyl)pyridin-2-amine oxalate,
6-(cyclopropylmethoxy)-5-(difluoromethoxy)-N-piperidin-4-yl-N-(pyridin-3-ylmethyl)pyridin-2-amine,
6-(cyclopropylmethoxy)-5-(difluoromethoxy)-N-piperidin-4-yl-N-(pyridin-3-ylmethyl)pyridin-2-amine trifluoroacetate,
6-(cyclopentyloxy)-5-methoxy-N-phenyl-N-piperidin-4-ylpyridin-2-amine,
6-(cyclopentyloxy)-5-methoxy-N-piperidin-4-yl-N-(pyrimidin-5-ylmethyl)pyridin-2-amine,
and
pharmaceutically acceptable salts thereof,
wherein compounds that are optically active can be in the form of their separate enantiomers or mixtures thereof, including racemic mixtures.

22. A pharmaceutical composition comprising a compound according to any one of claims 1 to 21, and a pharmaceutically acceptable carrier.

23. A composition according to claim 22, wherein said composition further comprises an additional pharmaceutical agent selected from calcium channel blockers, chloinergic drugs, adenosine receptor modulators, ampakines, NMDA-R modulators, mGluR modulators, cholinesterase inhibitors, or any combination thereof.

24. A composition according to claim 23, wherein said additional pharmaceutical agent is donepezil.

25. Use of a compound according to any one of claims 1 to 21 for the manufacture of a medicament for enhancing cognition in a patient in whom such enhancement is desired.

26. Use of a compound according to any one of claims 1 to 21 for the manufacture of a medicament for treating a patient suffering from cognition impairment or decline.

27. A use according to claim 26, wherein said patient is a human suffering from memory impairment.

28. A use according to claim 27, wherein said patient is suffering from memory impairment due to Alzheimer's disease, schizophrenia, Parkinson's disease, Huntington's disease, Pick's disease, Creutzfeldt-Jakob disease, depression, aging, head trauma, stroke, CNS hypoxia, cerebral senility, multiinfarct dementia, HIV or cardiovascular disease.

29. Use of a compound according to any one of claims 1 to 21 for the manufacture of a medicament for treating a patient having a disease involving decreased cAMP levels.

30. Use of a compound according to any one of claims 1 to 21 for the manufacture of a medicament for inhibiting PDE4 enzyme activity in a patient.

31. A use according to claim 27 wherein said patient is suffering from memory impairment due to a neurodegenerative disease or due to an acute neurodegenerative disorder.

32. Use of a compound according to any one of claims 1 to 21 for the manufacture of a medicament for treating a patient suffering from an allergic or inflammatory disease.

33. Use of a compound according to any one of claims 1 to 21 for the manufacture of a medicament for treating a patient suffering from schizophrenia, bipolar or manic depression, major depression, drug addiction and/or morphine dependence.

34. Use of a compound according to any one of claims 1 to 21 for the manufacture of a medicament for treating a patient suffering from psychosis **characterized by** elevated levels of PDE 4, wherein said psychosis is a form of depression.

## Patentansprüche

1. Verbindung der Formel I: 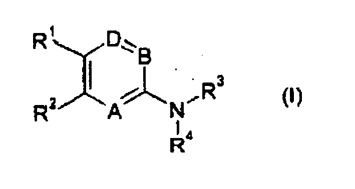 wobei A, B und D jeweils N oder CR⁵ sind, wobei wenigstens eines von A, B und D N ist;
R¹ Halogen, Alkyl mit 1 bis 4 Kohlenstoffatomen, halogeniertes Alkyl mit 1 bis 4 Kohlenstoffatomen, OR⁶, COR⁶, CONR⁶ oder NR⁶COR¹⁰ ist;
R² Halogen, Alkyl mit 1 bis 4 Kohlenstoffatomen, halogeniertes Alkyl mit 1 bis 4 Kohlenstoffatomen, OR⁷, COR⁶, CONR⁶ oder NR⁶COR¹⁰ ist;
R³ Alkyl mit 1 bis 8 Kohlenstoffatomen ist, welches verzweigt oder unverzweigt ist und welches unsubstituiert oder ein oder mehrere Male substituiert ist durch Halogen, Cyan, C1-4-Alkoxy, oder Kombinationen davon,
eine teilweise ungesättigte Carbocyclus-Alkylgruppe ist, wobei der carbocyclische Anteil 5 bis 14 Kohlenstoffatome aufweist und der Alkylanteil, welcher verzweigt oder unverzweigt ist, 1 bis 5 Kohlenstoffatome aufweist, und welche unsubstituiert, im carbocyclischen Anteil ein oder mehrere Male substituiert ist durch Halogen, Alkyl, Alkoxy, Nitro, Cyan, Oxo oder Kombinationen davon, und/oder im Alkylanteil ein oder mehrere Male substituiert ist durch Halogen, C1-4-Alkoxy, Cyan oder Kombinationen davon,
Arylalkyl mit 7 bis 19 Kohlenstoffatomen ist, wobei der Arylanteil 6 bis 14 Kohlenstoffatome aufweist und der Alkylanteil, welcher verzweigt oder unverzweigt ist, 1 bis 5 Kohlenstoffatome aufweist, wobei der Arylalkylrest unsubstituiert oder im Arylanteil ein oder mehrere Male substituiert ist durch Halogen, Trifluormethyl, CF₃O, Nitro, Amino, Alkyl, Alkoxy, Amino, Alkylamino, Dialkylamino oder Kombinationen davon, und/oder im Alkylanteil substituiert ist durch Halogen, Cyan, Alkyl mit 1 bis 4 Kohlenstoffatomen oder Kombinationen davon, wobei im Alkylanteil eine oder mehrere CH₂CH₂-Gruppen jeweils optional durch -CH=CH- oder -C≡C- ersetzt sind, und/oder eine oder mehrere CH₂-Gruppen jeweils optional durch -O- oder -NHersetzt sind, oder
eine Heterocyclus-Alkylgruppe ist, wobei der Heterocyclus-Anteil ungesättigt, teilweise gesättigt oder völlig gesättigt ist, und 5 bis 10 Ringatome aufweist, in welchem wenigstens 1 Ringatom ein N, N-O, O oder S ist, der Alkylanteil, welcher verzweigt oder unverzweigt ist, 1 bis 5 Kohlenstoffatome aufweist, die Heterocyclus-Alkylgruppe unsubstituiert ist, ein oder mehrere Male im Heterocyclus-Anteil substituiert ist durch Halogen, Alkyl, Alkoxy, Cyan, Trifluormethyl, CF₃O, Nitro, Oxo, Amino, Alkylamino, Dialkylamino oder Kombinationen davon, und/oder im Alkylanteil ein oder mehrere Male substituiert ist durch Halogen, Cyan, Alkyl mit 1 bis 4 Kohlenstoffatomen oder Kombinationen davon;
R⁴ Cycloalkyl mit 3 bis 10 Kohlenstoffatomen ist, welches unsubstituiert ist oder ein oder mehrere Male substituiert ist durch Halogen, Hydroxy, Oxo, Cyan, Alkyl mit 1 bis 4 Kohlenstoffatomen, Alkoxy mit 1 bis 4 Kohlenstoffatomen oder Kombinationen davon,
Aryl mit 6 bis 14 Kohlenstoffatomen ist, welches unsubstituiert ist oder ein oder mehrere Male substituiert ist durch Halogen, Alkyl, Alkenyl, Alkynyl, Hydroxy, Alkoxy, Alkoxyalkoxy, Nitro, Methylendioxy, Ethylendioxy, Trifluormethyl, OCF₃, Amino, Aminoalkyl, Aminoalkoxy, Dialkylamino, Hydroxyalkyl, Hydroxamsäure, Pyrrolyl, Tetrazol-5-yl, 2 (-Heterocyclus) Tetrazol-5-yl, Hydroxyalkoxy, Carboxy, Carboxyalkyl, Alkoxycarbonyl, Cyan, Acyl, Alkylthio, Alkylsulfinyl, Alkylsulfonyl, Phenoxy, Trialkylsilyloxy, R⁸-L oder Kombinationen davon,
Heteroaryl mit 5 bis 10 Ringatomen ist, in welchem wenigstens 1 Ringatom ein Heteroatom ist, welches unsubstituiert ist oder ein oder mehrere Male substituiert ist durch Halogen, Alkyl, Hydroxy, Alkoxy, Alkoxyalkoxy, Nitro, Methylendioxy, Ethylendioxy, Trifluormethyl, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Carboxyalkyl, Alkoxycarbonyl, Cyan, Acyl, Alkylthio, Alkylsulfinyl, Alkylsulfonyl, Phenoxy, Trialkylsilyloxy, R⁸-L oder Kombinationen davon,
eine heterocyclische Gruppe ist, welche gesättigt oder teilweise gesättigt ist, mit 5 bis 10 Ringatomen, in welcher wenigstens 1 Ringatom ein N-, O- oder S-Atom ist, welche unsubstituiert ist oder ein oder mehrere Male substituiert ist durch Halogen, Alkyl, Hydroxy, Alkoxy, Alkoxyalkoxy, Nitro, Oxo, Methylendioxy, Ethylendioxy, Trifluormethyl, OCF₃, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenylsulfonyl, Phenoxy, Cycloalkyl, Aryl, Heteroaryl oder Kombinationen davon,
eine Heterocyclus-Alkylgruppe ist, wobei der heterocyclische Anteil gesättigt, teilweise gesättigt oder ungesättigt ist, und 5 bis 10 Ringatome aufweist, in welcher wenigstens 1 Ringatom ein N-, O- oder S-Atom ist, und der Alkylanteil verzweigt oder unverzweigt ist und 1 bis 5 Kohlenstoffatome aufweist, die Heterocyclus-Alkylgruppe unsubstituiert ist, ein oder mehrere Male im heterocyclischen Anteil substituiert ist durch Halogen, Alkyl, Hydroxy, Alkoxy, Alkoxyalkoxy, Nitro, Oxo, Methylendioxy, Ethylendioxy, Trifluormethyl, OCF₃, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenylsulfonyl, Phenoxy, Cycloalkyl, Aryl, Heteroaryl oder Kombinationen davon, und/oder im Alkylanteil ein oder mehrere Male substituiert ist durch Halogen, Oxo, Hydroxy, Cyan oder Kombinationen davon, und wobei im Alkylanteil eine oder mehrere CH₂CH₂-Gruppen jeweils optional ersetzt sind durch -CH=CH- oder -C≡C-, und eine oder mehrere CH₂-Gruppen jeweils optional ersetzt sind durch - 0- oder -NH;
R⁵ H, Halogen, Alkyl mit 1 bis 4 Kohlenstoffatomen, halogeniertes Alkyl mit 1 bis 4 Kohlenstoffatomen, Alkoxy mit 1 bis 4 Kohlenstoffatomen oder halogeniertes Alkoxy mit 1 bis 4 Kohlenstoffatomen ist;
R⁶ H oder Alkyl mit 1 bis 4 Kohlenstoffatomen ist, welches verzweigt oder unverzweigt ist, und welches unsubstituiert ist oder ein oder mehrere Male substituiert ist durch Halogen;
R⁷ H oder Alkyl mit 1 bis 12 Kohlenstoffatomen ist, welches verzweigt oder unverzweigt ist, und welches unsubstituiert ist oder ein oder mehrere Male substituiert ist durch Halogen, Hydroxy, Cyan, C1-4-Alkoxy, Oxo oder Kombinationen davon, und wobei optional eine oder mehrere CH₂CH₂-Gruppen in jedem Fall ersetzt sind durch -CH=CH- oder -C≡C-,
Cycloalkyl mit 3 bis 10 Kohlenstoffatomen ist, welches unsubstituiert ist oder ein oder mehrere Male substituiert ist durch Halogen, Hydroxy, Oxo, Cyan, Alkyl mit 1 bis 4 Kohlenstoffatomen, Alkoxy mit 1 bis 4 Kohlenstoffatomen oder Kombinationen davon,
Cycloalkylalkyl mit 4 bis 16 Kohlenstoffatomen ist, welches unsubstituiert ist oder im Cycloalkylanteil und/oder im Alkylanteil ein oder mehrere Male substituiert ist durch Halogen, Oxo, Cyan, Hydroxy, C1-4-Alkyl, C1-4-Alkoxy oder Kombinationen davon,
Aryl mit 6 bis 14 Kohlenstoffatomen ist, welches unsubstituiert ist oder ein oder mehrere Male substituiert ist durch Halogen, CF₃, OCF₃, Alkyl, Hydroxy, Alkoxy, Nitro, Methylendioxy, Ethylendioxy, Cyan oder Kombinationen davon,
Arylalkyl ist, in welchem der Arylanteil 6 bis 14 Kohlenstoffatome aufweist, und der Alkylanteil, welcher verzweigt oder unverzweigt ist, 1 bis 5 Kohlenstoffatome aufweist, wobei der Arylalkylrest unsubstituiert ist, im Arylanteil ein oder mehrere Male substituiert ist durch Halogen, CF₃, OCF₃, Alkyl, Hydroxy, Alkoxy, Nitro, Cyan, Methylendioxy, Ethylendioxy oder Kombinationen davon, und/oder im Alkylanteil ein oder mehrere Male substituiert ist durch Halogen, Oxo, Hydroxy, Cyan oder Kombinationen davon, und wobei im Alkylanteil eine oder mehrere CH₂CH₂-Gruppen jeweils optional ersetzt sind durch -CH=CH- oder -C≡C-, und eine oder mehrere CH₂-Gruppen jeweils optional ersetzt sind durch -O- oder -NH-,
eine teilweise ungesättigte carbocyclische Gruppe mit 5 bis 14 Kohlenstoffatomen ist, welche unsubstituiert ist oder ein oder mehrere Male substituiert ist durch Halogen, Alkyl, Alkoxy, Hydroxy, Nitro, Cyan, Oxo oder Kombinationen davon,
eine heterocyclische Gruppe ist, welche gesättigt, teilweise gesättigt oder ungesättigt ist, mit 5 bis 10 Ringatomen, in welcher wenigstens 1 Ringatom ein N-, O- oder S-Atom ist, welche unsubstituiert ist oder ein oder mehrere Male substituiert ist durch Halogen, Hydroxy, Aryl, Alkyl, Alkoxy, Cyan, Trifluormethyl, Nitro, Oxo oder Kombinationen davon, oder
eine Heterocyclus-Alkylgruppe ist, wobei der heterocyclische Anteil gesättigt, teilweise gesättigt oder ungesättigt ist, und 5 bis 10 Ringatome aufweist, in welcher wenigstens 1 Ringatom ein N-, O- oder S-Atom ist, und der Alkylanteil verzweigt oder unverzweigt ist und 1 bis 5 Kohlenstoffatome aufweist, die Heterocyclus-Alkylgruppe unsubstituiert ist, ein oder mehrere Male im heterocyclischen Anteil substituiert ist durch Halogen, OCF₃, Hydroxy, Aryl, Alkyl, Alkoxy, Cyan, Trifluormethyl, Nitro, Oxo oder Kombinationen davon, und/oder im Alkylanteil ein oder mehrere Male substituiert ist durch Halogen, Oxo, Hydroxy, Cyan oder Kombinationen davon, und wobei im Alkylanteil eine oder mehrere CH₂CH₂-Gruppen jeweils optional ersetzt sind durch -CH=CH- oder -C≡C-, und eine oder mehrere CH₂-Gruppen jeweils optional ersetzt sind durch -O- oder -NH-;
R⁸ H ist,
Alkyl mit 1 bis 8 Kohlenstoffatomen ist, welches unsubstituiert ist oder ein oder mehrere Male substituiert ist durch Halogen, C1-4-Alkyl, C1-4-Alkoxy, Oxo oder Kombinationen davon,
Alkylamino oder Dialkylamino ist, wobei jeder Alkylanteil unabhängig voneinander 1 bis 8 Kohlenstoffatome aufweist,
eine teilweise ungesättigte Carbocyclus-Alkylgruppe ist, wobei der carbocyclische Anteil 5 bis 14 Kohlenstoffatome aufweist, und der Alkylanteil 1 bis 5 Kohlenstoffatome aufweist, und welche unsubstituiert ist oder ein oder mehrere Male substituiert ist durch Halogen, Alkyl, Alkoxy, Nitro, Cyan, Oxo oder Kombinationen davon,
Cycloalkyl mit 3 bis 10 Kohlenstoffatomen ist, welches unsubstituiert ist oder ein oder mehrere Male substituiert ist durch Halogen, Hydroxy, Oxo, Cyan, Alkoxy, Alkyl mit 1 bis 4 Kohlenstoffatomen oder Kombinationen davon,
Cycloalkylalkyl mit 4 bis 16 Kohlenstoffatomen ist, welches unsubstituiert ist oder im Cycloalkylanteil und/oder im Alkylanteil ein oder mehrere Male substituiert ist durch Halogen, Oxo, Cyan, Hydroxy, Alkyl, Alkoxy oder Kombinationen davon,
Aryl mit 6 bis 14 Kohlenstoffatome ist, welches unsubstituiert ist oder ein oder mehrere Male substituiert ist durch Halogen, Alkyl, Hydroxy, Alkoxy, Alkoxyalkoxy, Nitro, Methylendioxy, Ethylendioxy, Trifluormethyl, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenoxy, Cycloalkyl, Aryl, Heteroaryl oder Kombinationen davon,
Arylalkyl mit 7 bis 19 Kohlenstoffatomen ist, wobei der Arylanteil 6 bis 14 Kohlenstoffatome aufweist, und der Alkylanteil, welcher verzweigt oder unverzweigt ist, 1 bis 5 Kohlenstoffatome aufweist, wobei der Arylalkylrest unsubstituiert oder im Arylanteil ein oder mehrere Male substituiert ist durch Halogen, Trifluormethyl, CF₃O, Nitro, Amino, Alkyl, Alkoxy, Amino, Alkylamino, Dialkylamino oder Kombinationen davon, und/oder im Alkylanteil substituiert ist durch Halogen, Cyan, Alkyl mit 1 bis 4 Kohlenstoffatomen oder Kombinationen davon, wobei im Alkylanteil eine oder mehrere CH₂CH₂-Gruppen jeweils optional ersetzt sind durch -CH=CH- oder -C≡C-, und/oder eine oder mehrere CH₂-Gruppen jeweils optional ersetzt sind durch - O- oder -NH-,
eine heterocyclische Gruppe ist, welche gesättigt, teilweise gesättigt oder ungesättigt ist, mit 5 bis 10 Ringatomen, in welcher wenigstens 1 Ringatom ein N-, O- oder S-Atom ist, welche unsubstituiert ist oder ein oder mehrere Male substituiert ist durch Halogen, Alkyl, Hydroxy, Alkoxy, Alkoxyalkoxy, Nitro, Methylendioxy, Ethylendioxy, Trifluormethyl, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenoxy, Cycloalkyl, Aryl, Heteroaryl oder Kombinationen davon, oder
eine Heterocyclus-Alkylgruppe ist, wobei der heterocyclische Anteil gesättigt, teilweise gesättigt oder ungesättigt ist, und 5 bis 10 Ringatome aufweist, in welcher wenigstens 1 Ringatom ein N-, O- oder S-Atom ist, und der Alkylanteil, welcher verzweigt oder unverzweigt ist, 1 bis 5 Kohlenstoffatome aufweist, die Heterocyclus-Alkylgruppe unsubstituiert ist, ein oder mehrere Male im heterocyclischen Anteil substituiert ist durch Halogen, Alkyl, Alkoxy, Cyan, Trifluormethyl, CF₃O, Nitro, Oxo, Amino, Alkylamino, Dialkylamino oder Kombinationen davon und/oder ein oder mehrere Male im Alkylanteil substituiert ist durch Halogen, Cyan, Alkyl mit 1 bis 4 Kohlenstoffatomen oder Kombinationen davon;
L eine Einzelbindung oder ein zweiwertiger aliphatischer Rest mit 1 bis 8 Kohlenstoffatomen ist, wobei eine oder mehrere CH₂-Gruppen jeweils optional ersetzt sind durch -O-, -S-, -SO-, -SO₂-, - NR⁹-, -SO₂NR⁹-, -NR⁹SO₂-, -CO-, -CO₂-, -NR⁹CO-, - CONR⁹-, -NHCONH-, -OCONH, -NHCOO-, -SCONH-, - SCSNH-, -NHCSNH-, -CONHSO₂- oder -SO₂NHCO-; und
R⁹ H ist,
Alkyl mit 1 bis 8 Kohlenstoffatomen ist, welches verzweigt oder unverzweigt ist und welches unsubstituiert ist oder ein oder mehrere Male substituiert ist durch Halogen, C1-4-Alkyl, C1-4-Alkoxy, Oxo oder Kombinationen davon,
Arylalkyl mit 7 bis 19 Kohlenstoffatomen, wobei der Arylanteil 6 bis 14 Kohlenstoffatome aufweist, und der Alkylanteil, welcher verzweigt oder unverzweigt ist, 1 bis 5 Kohlenstoffatome aufweist, wobei der Arylalkylrest unsubstituiert ist oder im Arylanteil ein oder mehrere Male substituiert ist durch Halogen, Trifluormethyl, CF₃O, Nitro, Amino, Alkyl, Alkoxy, Amino, Alkylamino, Dialkylamino oder Kombinationen davon, und/oder im Alkylanteil substituiert ist durch Halogen, Cyan, Alkyl mit 1 bis 4 Kohlenstoffatomen oder Kombinationen davon, wobei im Alkylanteil eine oder mehrere CH₂CH₂-Gruppen jeweils optional ersetzt sind durch - CH=CH- oder -C≡C-, und/oder eine oder mehrere CH₂-Gruppen jeweils optional ersetzt sind durch -O- oder -NH-, oder
Aryl mit 6 bis 14 Kohlenstoffatomen ist, welches unsubstituiert ist oder ein oder mehrere Male substituiert ist durch Halogen, Alkyl, Hydroxy, Alkoxy, Alkoxyalkoxy, Nitro, Methylendioxy, Ethylendioxy, Trifluormethyl, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl oder Kombinationen davon; und
R¹⁰ H oder Alkyl mit 1 bis 4 Kohlenstoffatomen ist, welches verzweigt oder unverzweigt ist und welches unsubstituiert ist oder ein oder mehrere Male substituiert ist durch Halogen; oder
ein pharmazeutisch akzeptables Salz davon.

2. Verbindung der Formel III: 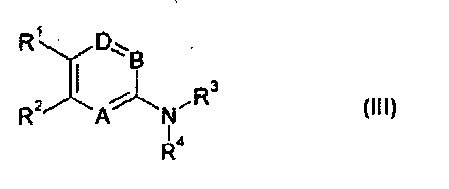 wobei
A, B und D jeweils N oder CR⁵ sind, und eines von A, B und D N ist;
R¹ Halogen, Alkyl mit 1 bis 4 Kohlenstoffatomen, halogeniertes Alkyl mit 1 bis 4 Kohlenstoffatomen oder OR⁶ ist;
R² Halogen, Alkyl mit 1 bis 4 Kohlenstoffatomen, halogeniertes Alkyl mit 1 bis 4 Kohlenstoffatomen oder OR⁷ ist;
R³ Arylalkyl mit 7 bis 19 Kohlenstoffatome ist, wobei der Arylanteil 6 bis 14 Kohlenstoffatome aufweist, und der Alkylanteil, welcher verzweigt oder unverzweigt ist, 1 bis 5 Kohlenstoffatome aufweist, wobei der Arylalkylrest unsubstituiert oder im Arylanteil ein oder mehrere Male substituiert ist durch Halogen, Trifluormethyl, CF₃O, Nitro, Amino, Alkyl, Alkoxy, Amino, Alkylamino, Dialkylamino oder Kombinationen davon, und/oder im Alkylanteil substituiert ist durch Halogen, Cyan, Alkyl mit 1 bis 4 Kohlenstoffatomen oder Kombinationen davon, wobei im Alkylanteil eine oder mehrere CH₂CH₂-Gruppen jeweils optional ersetzt sind durch -CH=CH- oder -C≡C-, und/oder eine oder mehrere CH₂-Gruppen jeweils optional ersetzt sind durch -O- oder -NH-, oder
eine Heterocyclus-Alkylgruppe ist, wobei der Heterocyclus-Anteil ungesättigt, teilweise gesättigt oder völlig gesättigt ist, und 5 bis 10 Ringatomen aufweist, in welchem wenigstens 1 Ringatom ein N, N-O, O oder S ist, der Alkylanteil, welcher verzweigt oder unverzweigt ist, 1 bis 5 Kohlenstoffatome aufweist, die Heterocyclus-Alkylgruppe unsubstituiert ist, ein oder mehrere Male im Heterocyclus-Anteil substituiert ist durch Halogen, Alkyl, Alkoxy, Cyan, Trifluormethyl, CF₃O, Nitro, Oxo, Amino, Alkylamino, Dialkylamino oder Kombinationen davon, und/oder im Alkylanteil ein oder mehrere Male substituiert ist durch Halogen, Cyan, Alkyl mit 1 bis 4 Kohlenstoffatomen oder Kombinationen davon;
R⁴ Cycloalkyl mit 3 bis 10 Kohlenstoffatomen ist, welches unsubstituiert ist oder ein oder mehrere Male substituiert ist durch Halogen, Hydroxy, Oxo, Cyan, Alkyl mit 1 bis 4 Kohlenstoffatomen, Alkoxy mit 1 bis 4 Kohlenstoffatomen oder Kombinationen davon,
Aryl mit 6 bis 14 Kohlenstoffatomen, welches unsubstituiert ist oder ein oder mehrere Male substituiert ist durch Halogen, Alkyl, Alkenyl, Alkynyl, Hydroxy, Alkoxy, Alkoxyalkoxy, Nitro, Methylendioxy, Ethylendioxy, Trifluormethyl, OCF₃, Amino, Aminoalkyl, Aminoalkoxy, Dialkylamino, Hydroxyalkyl, Hydroxamsäure, Pyrrolyl, Tetrazol-5-yl, 2(-Heterocyclus)Tetrazol-5-yl, Hydroxyalkoxy, Carboxy, Alkoxycarbonyl, Cyan, Acyl, Alkylthio, Alkylsulfinyl, Alkylsulfonyl, Phenoxy, Trialkylsilyloxy, R⁸-L oder Kombinationen davon,
Heteroaryl mit 5 bis 10 Ringatomen, in welchem wenigstens 1 Ringatom ein Heteroatom ist, welches unsubstituiert ist oder ein oder mehrere Male substituiert ist durch Halogen, Alkyl, Hydroxy, Alkoxy, Alkoxyalkoxy, Nitro, Methylendioxy, Ethylendioxy, Trifluormethyl, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenoxy, Trialkylsilyloxy, R⁸-L oder Kombinationen davon,
eine heterocyclische Gruppe ist, welche gesättigt oder teilweise gesättigt ist, mit 5 bis 10 Ringatomen, in welcher wenigstens 1 Ringatom ein N-, O- oder S-Atom ist, welches unsubstituiert ist oder ein oder mehrere Male substituiert ist durch Halogen, Alkyl, Hydroxy, Alkoxy, Alkoxyalkoxy, Nitro, Oxo, Methylendioxy, Ethylendioxy, Trifluormethyl, OCF₃, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenylsulfonyl, Phenoxy, Cycloalkyl, Aryl, Heteroaryl oder Kombinationen davon,
eine Heterocyclus-Alkylgruppe ist, wobei der heterocyclische Anteil gesättigt, teilweise gesättigt oder ungesättigt ist, und die 5 bis 10 Ringatome aufweist, in welcher wenigstens 1 Ringatom ein N-, O- oder S-Atom ist, und der Alkylanteil verzweigt oder unverzweigt ist und 1 bis 5 Kohlenstoffatome aufweist, die Heterocyclus-Alkylgruppe unsubstituiert ist, ein oder mehrere Male im heterocyclischen Anteil substituiert ist durch Halogen, Alkyl, Hydroxy, Alkoxy, Alkoxyalkoxy, Nitro, Oxo, Methylendioxy, Ethylendioxy, Trifluormethyl, OCF₃, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenylsulfonyl, Phenoxy, Cycloalkyl, Aryl, Heteroaryl oder Kombinationen davon, und/oder im Alkylanteil ein oder mehrere Male substituiert ist durch Halogen, Oxo, Hydroxy, Cyan oder Kombinationen davon, und wobei im Alkylanteil eine oder mehrere CH₂CH₂-Gruppen jeweils optional ersetzt sind durch -CH=CH- oder -C≡C-, und eine oder mehrere CH₂-Gruppen jeweils optional ersetzt sind durch - O- oder -NH;
R⁵ H, Halogen, Alkyl mit 1 bis 4 Kohlenstoffatomen, halogeniertes Alkyl mit 1 bis 4 Kohlenstoffatomen, Alkoxy mit 1 bis 4 Kohlenstoffatomen oder halogeniertes Alkoxy mit 1 bis 4 Kohlenstoffatomen ist;
R⁶ H oder Alkyl mit 1 bis 4 Kohlenstoffatomen ist, welches verzweigt oder unverzweigt ist und welches unsubstituiert ist oder ein oder mehrere Male substituiert ist durch Halogen;
R⁷ H oder Alkyl mit 1 bis 12 Kohlenstoffatomen ist, welches verzweigt oder unverzweigt ist und welches unsubstituiert ist oder ein oder mehrere Male substituiert ist durch Halogen, Hydroxy, Cyan, C1-4-Alkoxy, Oxo oder Kombinationen davon, und wobei optional eine oder mehrere CH₂CH₂-Gruppen in jedem Fall ersetzt sind durch -CH=CH- oder -C=C-,
Cycloalkyl mit 3 bis 10 Kohlenstoffatomen ist, welches unsubstituiert ist oder ein oder mehrere Male substituiert ist durch Halogen, Hydroxy, Oxo, Cyan, Alkyl mit 1 bis 4 Kohlenstoffatomen, Alkoxy mit 1 bis 4 Kohlenstoffatomen oder Kombinationen davon,
Cycloalkylalkyl mit 4 bis 16 Kohlenstoffatomen ist, welches unsubstituiert ist oder im Cycloalkylanteil und/oder im Alkylanteil ein oder mehrere Male substituiert ist durch Halogen, Oxo, Cyan, Hydroxy, C1-4-Alkyl, C1-4-Alkoxy oder Kombinationen davon,
Aryl mit 6 bis 14 Kohlenstoffatomen ist, welches unsubstituiert ist oder ein oder mehrere Male substituiert ist durch Halogen, CF₃, OCF₃, Alkyl, Hydroxy, Alkoxy, Nitro, Methylendioxy, Ethylendioxy, Cyan oder Kombinationen davon,
Arylalkyl ist, in welchem der Arylanteil 6 bis 14 Kohlenstoffatome aufweist, und der Alkylanteil, welcher verzweigt oder unverzweigt ist, 1 bis 5 Kohlenstoffatome aufweist, wobei der Arylalkylrest unsubstituiert ist, im Arylanteil ein oder mehrere Male substituiert ist durch Halogen, CF₃, OCF₃, Alkyl, Hydroxy, Alkoxy, Nitro, Cyan, Methylendioxy, Ethylendioxy oder Kombinationen davon, und/oder im Alkylanteil ein oder mehrere Male substituiert ist durch Halogen, Oxo, Hydroxy, Cyan oder Kombinationen davon, und wobei im Alkylanteil eine oder mehrere CH₂CH₂-Gruppen jeweils optional ersetzt sind durch -CH=CH- oder -C≡C-, und eine oder mehrere CH₂-Gruppen jeweils optional ersetzt sind durch -O- oder -NH-,
eine teilweise ungesättigte carbocyclische Gruppe mit 5 bis 14 Kohlenstoffatomen ist, welche unsubstituiert ist oder ein oder mehrere Male substituiert ist durch Halogen, Alkyl, Alkoxy, Hydroxy, Nitro, Cyan, Oxo oder Kombinationen davon,
eine heterocyclische Gruppe ist, welche gesättigt, teilweise gesättigt oder ungesättigt ist, mit 5 bis 10 Ringatomen, in welcher wenigstens 1 Ringatom ein N-, 0- oder S-Atom ist, welche unsubstituiert ist oder ein oder mehrere Male substituiert ist durch Halogen, Hydroxy, Aryl, Alkyl, Alkoxy, Cyan, Trifluormethyl, Nitro, Oxo oder Kombinationen davon, oder
eine Heterocyclus-Alkylgruppe, wobei der heterocyclische Anteil gesättigt, teilweise gesättigt oder ungesättigt ist, und 5 bis 10 Ringatomen aufweist, in welcher wenigstens 1 Ringatom ein N-, O- oder S-Atom ist, und der Alkylanteil verzweigt oder unverzweigt ist und 1 bis 5 Kohlenstoffatome aufweist, die Heterocyclus-Alkylgruppe unsubstituiert ist, ein oder mehrere Male im heterocyclischen Anteil substituiert ist durch Halogen, OCF₃, Hydroxy, Aryl, Alkyl, Alkoxy, Cyan, Trifluormethyl, Nitro, Oxo oder Kombinationen davon, und/oder im Alkylanteil ein oder mehrere Male substituiert ist durch Halogen, Oxo, Hydroxy, Cyan oder Kombinationen davon, und wobei im Alkylanteil eine oder mehrere CH₂CH₂-Gruppen jeweils optional ersetzt sind durch -CH=CH- oder -C≡C-, und eine oder mehrere CH₂-Gruppen jeweils optional ersetzt sind durch -O- oder -NH-;
R⁸ H ist,
Alkyl mit 1 bis 8 Kohlenstoffatomen ist, welches unsubstituiert ist oder ein oder mehrere Male substituiert ist durch Halogen, C1-4-Alkyl, C1-4-Alkoxy, Oxo oder Kombinationen davon,
Alkylamino oder Dialkylamino ist, wobei jeder Alkylanteil unabhängig voneinander 1 bis 8 Kohlenstoffatome aufweist,
eine teilweise ungesättigte Carbocyclus-Alkylgruppe ist, wobei der carbocyclische Anteil 5 bis 14 Kohlenstoffatome aufweist, und der Alkylanteil 1 bis 5 Kohlenstoffatome aufweist, und welche unsubstituiert ist oder ein oder mehrere Male substituiert ist durch Halogen, Alkyl, Alkoxy, Nitro, Cyan, Oxo oder Kombinationen davon,
Cycloalkyl mit 3 bis 10 Kohlenstoffatomen ist, welches unsubstituiert ist oder ein oder mehrere Male substituiert ist durch Halogen, Hydroxy, Oxo, Cyan, Alkoxy, Alkyl mit 1 bis 4 Kohlenstoffatomen oder Kombinationen davon,
Cycloalkylalkyl mit 4 bis 16 Kohlenstoffatomen ist, welches unsubstituiert ist oder im Cycloalkylanteil und/oder im Alkylanteil ein oder mehrere Male substituiert ist durch Halogen, Oxo, Cyan, Hydroxy, Alkyl, Alkoxy oder Kombinationen davon,
Aryl mit 6 bis 14 Kohlenstoffatomen ist, welches unsubstituiert ist oder ein oder mehrere Male substituiert ist durch Halogen, Alkyl, Hydroxy, Alkoxy, Alkoxyalkoxy, Nitro, Methylendioxy, Ethylendioxy, Trifluormethyl, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenoxy, Cycloalkyl, Aryl, Heteroaryl oder Kombinationen davon,
Arylalkyl mit 7 bis 19 Kohlenstoffatomen ist, wobei der Arylanteil 6 bis 14 Kohlenstoffatome aufweist, und der Alkylanteil, welcher verzweigt oder unverzweigt ist, 1 bis 5 Kohlenstoffatome aufweist, wobei der Arylalkylrest unsubstituiert oder im Arylanteil ein oder mehrere Male substituiert ist durch Halogen, Trifluormethyl, CF₃O, Nitro, Amino, Alkyl, Alkoxy, Amino, Alkylamino, Dialkylamino oder Kombinationen davon, und/oder im Alkylanteil substituiert ist durch Halogen, Cyan, Alkyl mit 1 bis 4 Kohlenstoffatomen oder Kombinationen davon, wobei im Alkylanteil eine oder mehrere CH₂CH₂-Gruppen jeweils optional ersetzt sind durch -CH=CH- oder -C≡C-, und/oder eine oder mehrere CH₂-Gruppen jeweils optional ersetzt sind durch - O- oder -NH-,
eine heterocyclische Gruppe ist, welche gesättigt, teilweise gesättigt oder ungesättigt ist, mit 5 bis 10 Ringatomen, in welcher wenigstens 1 Ringatom ein N-, O- oder S-Atom ist, welche unsubstituiert ist oder ein oder mehrere Male substituiert ist durch Halogen, Alkyl, Hydroxy, Alkoxy, Alkoxyalkoxy, Nitro, Methylendioxy, Ethylendioxy, Trifluormethyl, Amino, Aminomethyl, Aminoalkyl, Aminoalkoxy, Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl, Phenoxy, Cycloalkyl, Aryl, Heteroaryl oder Kombinationen davon, oder
eine Heterocyclus-Alkylgruppe ist, wobei der heterocyclische Anteil gesättigt, teilweise gesättigt oder ungesättigt ist, und 5 bis 10 Ringatomen aufweist, in welcher wenigstens 1 Ringatom ein N-, O- oder S-Atom ist, und der Alkylanteil, welcher verzweigt oder unverzweigt ist, 1 bis 5 Kohlenstoffatome aufweist, die Heterocyclus-Alkylgruppe unsubstituiert ist, ein oder mehrere Male im heterocyclischen Anteil substituiert ist durch Halogen, Alkyl, Alkoxy, Cyan, Trifluormethyl, CF₃O, Nitro, Oxo, Amino, Alkylamino, Dialkylamino oder Kombinationen davon und/oder ein oder mehrere Male im Alkylanteil substituiert ist durch Halogen, Cyan, Alkyl mit 1 bis 4 Kohlenstoffatomen oder Kombinationen davon;
L eine Einzelbindung oder ein zweiwertiger aliphatischer Rest mit 1 bis 8 Kohlenstoffatomen ist, wobei eine oder mehrere CH₂-Gruppen jeweils optional ersetzt sind durch -O-, -S-, -SO-, -SO₂-, -N R⁹-, -SO₂NR⁹-, -NR⁹SO₂-, -CO-, -CO₂-, -NR⁹CO-, - CONR⁹-, -NHCONH-, -OCONH, -NHCOO-, -SCONH-, - SCSNH-, -NHCSNH-, -CONHSO₂- oder -SO₂NHCO-; und
R⁹ H ist,
Alkyl mit 1 bis 8 Kohlenstoffatomen ist, welches verzweigt oder unverzweigt ist und welches unsubstituiert ist oder ein oder mehrere Male substituiert ist durch Halogen, C1-4-Alkyl, C1-4-Alkoxy, Oxo oder Kombinationen davon,
Arylalkyl mit 7 bis 19 Kohlenstoffatomen ist, wobei der Arylanteil 6 bis 14 Kohlenstoffatome aufweist, und der Alkylanteil, welcher verzweigt oder unverzweigt ist, 1 bis 5 Kohlenstoffatome aufweist, wobei der Arylalkylrest unsubstituiert ist oder im Arylanteil ein oder mehrere Male substituiert ist durch Halogen, Trifluormethyl, CF₃O, Nitro, Amino, Alkyl, Alkoxy, Amino, Alkylamino, Dialkylamino oder Kombinationen davon, und/oder im Alkylanteil substituiert ist durch Halogen, Cyan, Alkyl mit 1 bis 4 Kohlenstoffatomen oder Kombinationen davon, wobei im Alkylanteil eine oder mehrere CH₂CH₂-Gruppen jeweils optional ersetzt sind durch -CH=CH- oder -C≡C-, und/oder eine oder mehrere CH₂-Gruppen jeweils optional ersetzt sind durch -O- oder -NH-, oder
Aryl mit 6 bis 14 Kohlenstoffatomen ist, welches unsubstituiert ist oder ein oder mehrere Male substituiert ist durch Halogen, Alkyl, Hydroxy, Alkoxy, Alkoxyalkoxy, Nitro, Methylendioxy, Ethylendioxy, Trifluormethyl, Amino, Aminomethyl, Aminoalkyl, Aminoalkoxy Dialkylamino, Hydroxyalkyl, Hydroxamsäure, Tetrazol-5-yl, Hydroxyalkoxy, Carboxy, Alkoxycarbonyl, Cyan, Acyl, Alkylthio, Alkylsulfinyl, Alkylsulfonyl oder Kombinationen davon; und
wobei R¹ OR⁶ ist und/oder R² OR⁷ ist;
oder ein pharmazeutisch akzeptables Salz davon.

3. Verbindung nach Anspruch 1, wobei R¹ OR⁶ ist und/oder R² OR⁷ ist.

4. Verbindung nach Anspruch 1, wobei R¹ und/oder R² COR⁶, CONR⁶ oder NR⁶COR¹⁰ ist.

5. Verbindung nach Anspruch 1 oder 2, wobei R¹ Halogen oder OR⁶ und R⁶ Alkyl oder halogeniertes Alkyl ist, oder
wobei R² Halogen oder OR⁷ und R⁷ Alkyl, Cycloalkyl, Cycloalkylalkyl, eine heterocyclische Gruppe oder halogeniertes Alkyl ist.

6. Verbindung nach Anspruch 1, wobei R³ Arylalkyl oder Heterocyclus-Alkyl ist, welches in jedem Fall substituiert oder unsubstituiert ist.

7. Verbindung nach den Ansprüchen 1 oder 2, wobei R³ Benzyl oder Pyridylmethyl ist, welches in jedem Fall substituiert oder unsubstituiert ist.

8. Verbindung nach den Ansprüchen 1 oder 2, wobei R⁴ Cycloalkyl, Aryl, Heteroaryl oder eine heterocyclische Gruppe ist, welche substituiert oder unsubstituiert ist.

9. Verbindung nach Anspruch 8, wobei R⁴ Cycloalkyl, Aryl, oder eine heterocyclische Gruppe ist, welche substituiert oder unsubstituiert ist.

10. Verbindung nach Anspruch 9, wobei R⁴ Cyclohexyl, Piperidinyl oder Phenyl ist, welches in jedem Fall substituiert oder unsubstituiert ist, oder wobei R⁴ Phenyl durch Carboxy, Cyan, Tetrazol und/oder L- R⁸ substituiert ist.

11. Verbindung nach den Ansprüchen 1 oder 2,
wobei R⁴ durch R⁸-L wenigstens monosubstituiert ist, und L eine Einzelbindung oder ein zweiwertiger aliphatischer Rest mit 1 bis 8 Kohlenstoffatomen ist, wobei wenigstens eine CH₂-Gruppe durch - SO₂NR⁹, -NR⁹-, -NR⁹CO-, -CONR⁹-, -CO₂-, -CONHSO₂-,-SO₂NHCO-, -SO₂- oder -NR⁹SO₂- ersetzt ist,
wobei R⁸ Methyl, Ethyl, Propyl oder Phenyl ist, welches in jedem Fall unsubstituiert oder substituiert ist,
wobei R⁹ H, Alkyl mit 1 bis 4 Kohlenstoffatomen oder Aryl ist, oder
wobei R⁵ H, F oder Methyl ist.

12. Verbindung nach den Ansprüchen 1 oder 2, wobei A N ist, B und D jeweils unabhängig CR⁵ sind, R¹ OR⁶ ist, R² Halogen oder OR⁷ ist, R³ Pyridylmethyl, Fluorbenzyl oder 2,6-Difluorbenzyl ist, R⁴ Aryl, Cycloalkyl oder eine gesättigte heterocyclische Gruppe ist, in jedem Fall substituiert oder unsubstituiert, R⁵ H, Halogen oder Alkyl ist, welches substituiert oder unsubstituiert ist, R⁶ Alkyl ist, welches substituiert oder unsubstituiert ist, und R⁷ Alkyl, Cycloalkyl, Cycloalkylalkyl oder eine gesättigte heterocyclische Gruppe ist, in jedem Fall substituiert oder unsubstituiert.

13. Verbindung nach den Ansprüchen 1 oder 2, wobei A N ist, B und D jeweils unabhängig CR⁵ sind, R¹ OR⁶ ist, R² Halogen oder OR⁷ ist, R³ Pyridylmethyl, Fluorbenzyl, 2,6-Difluorbenzyl, 5-Thiazolylmethyl oder 5-Pyrimidinylmethyl ist, R⁴ Phenyl ist, welches unsubstituiert oder substituiert ist, R⁵ H, Halogen oder Alkyl ist, welches substituiert oder unsubstituiert ist, R⁶ Alkyl ist, welches substituiert oder unsubstituiert ist, und R⁷ Alkyl, Cycloalkyl, Cycloalkylalkyl oder eine gesättigte heterocyclische Gruppe ist, in jedem Fall substituiert oder unsubstituiert.

14. Verbindung nach Anspruch 13, wobei R³ Pyridylmethyl, Fluorbenzyl, 2,6-Difluorbenzyl ist.

15. Verbindung nach den Ansprüchen 1 oder 2, wobei A N ist, B und D jeweils unabhängig CH ist, R¹ OR⁶ ist, R² Halogen oder OR⁷ ist, R³ Pyridylmethyl, 5-Thiazolylmethyl oder 5-Pyrimidinylmethyl ist, R⁴ unsubstituiertes Cycloalkyl, Aryl, welches substituiert oder unsubstituiert ist, oder Piperidinyl ist, welches substituiert oder unsubstituiert ist, R⁶ unsubstituiertes Alkyl oder CHF² ist und R⁷ Alkyl, Cycloalkyl, Cycloalkylalkyl oder Tetrahydrofuranyl ist, in jedem Fall substituiert oder unsubstituiert.

16. Verbindung nach Anspruch 15, wobei R³ Pyridylmethyl ist.

17. Verbindung nach den Ansprüchen 1 oder 2, wobei A N ist, B und D jeweils unabhängig CH sind, R¹ OR⁶ ist, R² Halogen oder OR⁷ ist, R³ 3-Pyridylmethyl, 5-Thiazolylmethyl oder 5-Pyrimidinylmethyl ist, R⁴ Cyclohexyl, Phenyl, welches substituiert oder unsubstituiert ist, oder Piperidinyl ist, welches substituiert oder unsubstituiert ist, R⁶ unsubstituiertes Alkyl oder CHF² ist und R⁷ Alkyl, Cycloalkyl, Cycloalkylalkyl oder Tetrahydrofuranyl ist, in jedem Fall substituiert oder unsubstituiert.

18. Verbindung nach Anspruch 17, wobei R³ 3-Pyridylmethyl ist.

19. Verbindung nach den Ansprüchen 1 oder 2, wobei A N ist und B und D jeweils unabhängig C5 sind, R¹ OR⁶ ist, R² OR⁷ ist, R³ Heterozyklus-Alkyl ist, R⁴ eine heterocyclische Gruppe ist, welche unsubstituiert oder substituiert ist, R⁵ H, Halogen oder Alkyl ist, welches substituiert oder unsubstituiert ist, R⁶ Alkyl ist, R⁷ Alkyl, Cycloalkyl oder Cycloalkylalkyl ist, in jedem Fall substituiert oder unsubstituiert.

20. Verbindung ausgewählt aus den folgenden:
4-{N-[4-Methoxy-3-(R)-(Tetrahydrofuran-3-yloxy)Phenyl]Pyridin-3-ylmethylamino}Piperidin-1-Carbonsäure-tert-butyl-Ester,
3-[N-(6-Cyclopropylmethoxy-5-Methoxypyridin-2-yl)-Pyridin-3-ylmethylamino]-Benzoesäure,
3-[N-(5,6-Dimethoxypyridin-2-yl)-Pyridin-3-ylmethylamino]-Benzoesäure,
3-[N-(6-Cyclobutyloxy-5-Methoxypyridin-2-yl)-Pyridin-3-ylmethylamino]-Benzoesäure,
3-[N-(6-Cyclopropylmethoxy-5-Difluormethoxypyridin-2-yl)-Pyridin-3-ylmethylamino]-Benzoesäure,
3-[N-(5-Difluormethoxy-6-Methoxypyridin-2-yl)-Pyridin-3-ylmethylamino]-Benzoesäure,
3-[N-(6-Ethoxy-5-Methoxypyridin-2-yl)-Pyridin-3-ylmethylamino]-Benzoesäure,
3-[N-(6-Isopropoxy-5-Methoxypyridin-2-yl)-Pyridin-3-ylmethylamino]-Benzoesäure,
3-[N-(5-Difluormethoxy-6-Isopropoxypyridin-2-yl)-Pyridin-3-ylmethylamino]-Benzoesäure,
3-[N-(6-Cyclobutyloxy-5-Difluormethoxypyridin-2-yl)-Pyridin-3-ylmethylamino]-Benzoesäure,
N-(1-Benzolsulfonylpiperidin-3-yl)-N-[5-Methoxy-6-(R)-(Tetrahydrofuran-3-yloxy)-Pyridin-2-yl]-Pyridin-3-ylmethylamin,
N-(1-Methanesulfonylpiperidin-3-yl)-N-[5-Methoxy-6-(R)-(Tetrahydrofuran-3-yloxy)-Pyridin-2-yl]-Pyridin-3-ylmethylamin,
N-[5-Methoxy-6-(R)-(Tetrahydrofuran-3-yloxy)-Pyridin-2-yl]-N-Piperidin-3-yl-Pyridin-3-ylmethylamin,
N-[5-Methoxy-6-(R)-(Tetrahydrofuran-3-yloxy)-Pyridin-2-yl]-N-Piperidin-4-ylmethyl-Pyridin-3-ylmethylamin,
4-(N-{[5-Methoxy-6-(R)-(Tetrahydrofuran-3-yloxy)-Pyridin-2-yl]-Pyridin-3-ylmethylamino}-Methyl)-N-Piperidin-1-Carbonsäure-tert-butyl-Ester
N-(1-Benzolsulfonylpiperidin-4-yl)-N-[5-Methoxy-6-(R)-(Tetrahydrofuran-3-yloxy)-Pyridin-2-yl]-Pyridin-3-ylmethylamin
1-(4-{N-[5-Methoxy-6-(R)-(Tetrahydrofuran-3-yloxy)-Pyridin-2-yl]-Pyridin-3-ylmethylamino}-N-Piperidin-1-yl)-Ethanon
N-[5-Methoxy-6-(R)-(Tetrahydrofuran-3-yloxy)-Pyridin-2-yl]-Piperidin-4-yl-Pyridin-3-ylmethylamin
4-{N-[5-Methoxy-6-(R)-(Tetrahydrofuran-3-yloxy)-Pyridin-2-yl]-Pyridin-3-ylmethylamino}-Methyl)-Piperidin-1-Carbonsäure-tert-butyl-Ester
3-{N-[5-Methoxy-6-(R)-(Tetrahydrofuran-3-yloxy)-Pyridin-2-yl]-Pyridin-3-ylmethylamino}-Benzoesäure
N-[5-Methoxy-6-(R)-(Tetrahydrofuran-3-yloxy)-Pyridin-2-yl]-Pyridin-3-ylmethyl-N-[4-(2H-tetrazol-5-yl)Phenyl]-Amin
N-Cyclohexyl-N-[5-Methoxy-6-(R)-(Tetrahydrofuran-3-yloxy)-Pyridin-2-yl]-Pyridin-3-ylmethylamin
N-[5-Methoxy-6-(R)-(Tetrahydrofuran-3-yloxy)-Pyridin-2-yl]-N-phenyl-Piperidin-3-ylmethylamin
N-(3-Chlorophenyl)-N-[5-Methoxy-6-(R)-(Tetrahydrofuran-3-yloxy) Pyridin-2-yl]-Pyridin-3-ylmethylamin
3-{N-[5-Methoxy-6-(Tetrahydrofuran-3-yloxy)-Pyridin-2-yl]-Pyridin-3-ylmethylamino}-Benzoesäure,
3-[N-(6-Cyclopentyloxy-5-Methoxypyridin-2-yl)-Pyridin-3-ylmethylamino]-Benzoesäure,
4-[N-(5-Cyclopentyloxy-4-Methoxy-2-Methylphenyl)Pyridin-3-ylmethylamino]-Benzoesäure,
3-[N-(5-Cyclopentyloxy-4-Methoxy-2-Methylphenyl)Pyridin-3-ylmethylamino]-Benzoesäure, und
pharmazeutisch akzeptable Salze davon,
wobei Verbindungen, die optisch aktiv sind, in der Form ihrer separaten Enantiomere oder Mischungen davon vorliegen können, einschließlich racemischen Mischungen.

21. Verbindung ausgewählt aus den folgenden:
(6-Cyclopentyloxy-5-Methoxy-Pyridin-2-yl)-Piperidin-4-yl-Pyridin-3-ylmethyl-Aminhydrochlorid,
(6-Cyclopentyloxy-5-Methoxy-Pyridin-2-yl)-Piperidin-4-yl-Pyridin-4-ylmethyl-Amin,
(6-Cyclopropylmethoxy-5-Methoxy-Pyridin-2-yl)-Piperidin-4-yl-Pyridin-3-ylmethyl-Amin,
{4-[(6-Cyclopentyloxy-5-Methoxy-Pyridin-2-yl)-Pyridin-3-ylmethyl-amino]-Piperidin-1-yl}-(4-Fluorphenyl)-Methanon,
(6-Cyclopentyloxy-5-Methoxy-Pyridin-2-yl)-(1-Methansulfonyl-Piperidin-4-yl)-Pyridin-3-ylmethyl-Amin,
6-(Cyclopentyloxy)-5-Methoxy-N-Piperidin-3-yl-N-(Pyridin-3-ylmethyl) Pyridin-2-Amin,
6-(Cyclopentyloxy)-5-Methoxy-N-Piperidin-3-yl-N-(Pyridin-3-ylmethyl)Pyridin-2-Aminoxalat,
6-Isopropoxy-5-Methoxy-N-Piperidin-4-yl-N-(Pyridin-3-ylmethyl)Pyridin-2-Amin,
6-Isopropoxy-5-Methoxy-N-Piperidin-4-yl-N-(Pyridin-3-ylmethyl)Pyridin-2-Aminoxalat,
6-(Cyclopropylmethoxy)-5-(Difluormethoxy)-N-Piperidin-4-yl-N-(Pyridin-3-ylmethyl)Pyridin-2-Amin,
6-(Cyclopropylmethoxy)-5-(Difluormethoxy)-N-Piperidin-4-yl-N-(Pyridin-3-ylmethyl)Pyridin-2-Amintrifluoracetat,
6-(Cyclopentyloxy)-5-Methoxy-N-phenyl-N-Piperidin-4-ylpyridin-2-Amin,
6-(Cyclopentyloxy)-5-Methoxy-N-Piperidin-4-yl-N-(Pyrimidin-5-ylmethyl)Pyridin-2-Amin,
und pharmazeutisch akzeptable Salze davon,
wobei Verbindungen, die optisch aktiv sind, in der Form ihrer separaten Enantiomere oder Mischungen davon vorliegen können, einschließlich racemischen Mischungen.

22. Pharmazeutische Zusammensetzung mit einer Verbindung nach einem der Ansprüche 1 bis 21, und ein pharmazeutisch akzeptabler Träger.

23. Zusammensetzung nach Anspruch 22, wobei die Zusammensetzung weiterhin ein zusätzlichen pharmazeutisches Mittel ausgewählt aus Calciumkanalblockern, cholinergen Medikamenten, Adenosin-Rezeptormodulatoren, Amphakinen, NMDA-R-Modulatoren, mGluR-Modulatoren, Cholinesterasehemmern oder Kombinationen davon aufweist.

24. Zusammensetzung nach Anspruch 23, wobei das zusätzliche pharmazeutische Mittel Donepezil ist.

25. Verwendung einer Verbindung nach einem der Ansprüche 1 bis 21 für die Herstellung eines Medikaments zur Verbesserung der Kognition in einem Patienten, bei dem eine solche Verbesserung gewünscht ist.

26. Verwendung einer Verbindung nach einem der Ansprüche 1 bis 21 für die Herstellung eines Medikaments zur Behandlung eines an cognitiven Störungen oder Einbußen leidenden Patienten.

27. Verwendung nach Anspruch 26, wobei der Patient ein an Gedächtnisstörungen leidender Mensch ist.

28. Verwendung nach Anspruch 27, wobei der Patient aufgrund von Alzheimer-Krankheit, Schizophrenie, Parkinson-Krankheit, Huntington-Krankheit, Pick-Krankheit, Creutzfeldt-Jakob-Krankheit, Depression, Alterung, Kopftrauma, Schlaganfall, CNS-Hypoxie, cerebraler Senilität, Multiinfarkt-Demenz, HIV oder cardiovasculärer Krankheit an Gedächtnisstörungen leidet.

29. Verwendung einer Verbindung nach einem der Ansprüche 1 bis 21 für die Herstellung eines Medikaments zur Behandlung eines Patienten mit einer Krankheit mit verringerter cAMP-Konzentration.

30. Verwendung einer Verbindung nach einem der Ansprüche 1 bis 21 für die Herstellung eines Medikaments zur Hemmung von PDE4-Enzym-Aktivität in einem Patienten.

31. Verwendung nach Anspruch 27, wobei der Patient aufgrund einer neurodegenerativen Krankheit oder einer akuten neurodegenerativen Störung an Gedächtnisstörungen leidet.

32. Verwendung einer Verbindung nach einem der Ansprüche 1 bis 21 für die Herstellung eines Medikaments zur Behandlung eines an einer allergischen oder entzündlichen Krankheit leidenden Patienten.

33. Verwendung einer Verbindung nach einem der Ansprüche 1 bis 21 für die Herstellung eines Medikaments zur Behandlung eines an Schizophrenie, bipolarer oder manischer Depression, schwerer Depression, Drogenabhängigkeit und/oder morphiner Abhängigkeit leidenden Patienten.

34. Verwendung einer Verbindung nach einem der Ansprüche 1 bis 21 für die Herstellung eines Medikaments zur Behandlung eines an durch erhöhte Konzentration von PDE 4 **gekennzeichnet**en Psychose leidenden Patienten, wobei die Psychose eine Form von Depression ist.

## Revendications

1. Composé selon la formule I: 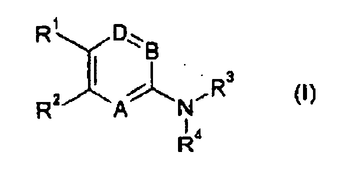 dans lequel A, B et D sont chacun N ou CR⁵, au moins un d'A, B et D étant N;
R¹ est halogène, alkyle ayant 1 à 4 atomes de carbone, alkyle halogéné ayant 1 à 4 atomes de carbone, OR⁶, COR⁶, CONR⁶ ou NR⁶COR¹⁰;
R² est halogène, alkyle ayant 1 à 4 atomes de carbone, alkyle halogéné ayant 1 à 4 atomes de carbone, OR⁷, COR⁶, CONR⁶ ou NR⁶COR¹⁰;
R³ est alkyle ayant 1 à 8 atomes de carbone, qui est ramifié ou non ramifié et qui n'est pas substitué ou est substitué une ou plusieurs fois par halogène, cyano, alcoxy C1-4 ou des combinaisons de ceux-ci,
un groupe carbocycle-alkyle partiellement non saturé dans lequel la partie carbocyclique a 5 à 14 atomes de carbone et la partie d'alkyle, qui est ramifiée ou non ramifiée, a 1 à 5 atomes de carbone, et qui n'est pas substitué, est substitué dans la partie carbocyclique une ou plusieurs fois par halogène, alkyle, alcoxy, nitro, cyano, oxo ou des combinaisons de ceux-ci, et/ou substitué dans la partie d'alkyle une ou plusieurs fois par halogène, alcoxy C1-4, cyano ou des combinaisons de ceux-ci,
arylalkyle ayant 7 à 19 atomes de carbone, dans lequel la partie d'aryle a 6 à 14 atomes de carbone et la partie d'alkyle, qui est ramifiée ou non ramifiée, a 1 à 5 atomes de carbone, dans lequel le radical arylalkyle n'est pas substitué ou est substitué, dans la partie d'aryle, une ou plusieurs fois par halogène, trifluorométhyle, CF₃O, nitro, amino, alkyle, alcoxy, amino, alkylamino, dialkylamino ou des combinaisons de ceux-ci, et/ou substitué dans la partie d'alkyle par halogène, cyano, alkyle ayant 1 à 4 atomes de carbone ou des combinaisons de ceux-ci, dans lequel dans la partie d'alkyle un ou plusieurs groupes -CH₂CH₂- sont chacun optionnellement remplacés par -CH=CH- ou - C≡C-, et/ou un ou plusieurs groupes -CH₂- sont chacun optionnellement remplacés par -O- ou -NH-, ou
un groupe hétérocycle-alkyle, dans lequel la partie hétérocyclique n'est pas saturée, est partiellement saturée ou complètement saturée et a 5 à 10 atomes cycliques, dans lequel au moins 1 atome cyclique est un N, N-O, O ou S, la partie d'alkyle, qui est ramifiée ou non ramifiée et a 1 à 5 atomes de carbone, le groupe hétérocycle-alkyle n'est pas substitué, est substitué une ou plusieurs fois dans la partie hétérocyclique par halogène, alkyle, alcoxy, cyano, trifluorométhyle, CF₃O, nitro, oxo, amino, alkylamino, dialkylamino ou des combinaisons de ceux-ci, et/ou substitué dans la partie d'alkyle une ou plusieurs fois par halogène, cyano, alkyle ayant 1 à 4 atomes de carbone ou des combinaisons de ceux-ci;
R⁴ est cycloalkyle ayant 3 à 10 atomes de carbone, qui n'est pas substitué ou est substitué une ou plusieurs fois par halogène, hydroxy, oxo, cyano, alkyle ayant 1 à 4 atomes de carbone, alcoxy ayant 1 à 4 atomes de carbone ou des combinaisons de ceux-ci,
aryle ayant 6 à 14 atomes de carbone et qui n'est pas substitué ou est substitué une ou plusieurs fois par halogène, alkyle, alcényle, alkynyle, hydroxy, alcoxy, alcoxyalcoxy, nitro, méthylènedioxy, éthylènedioxy, trifluorométhyle, OCF₃, amino, aminoalkyle, aminoalcoxy, dialkylamino, hydroxyalkyle, acide hydroxamique, pyrrolyle, tétrazole-5-yle, 2(-hétérocycle)tétrazole-5-yle, hydroxyalcoxy, carboxy, carboxyalkyle, alcoxycarbonyle, cyano, acyle, alkylthio, alkylsulfinyle, alkylsulfonyle, phénoxy, trialkylsilyloxy, R⁸-L ou des combinaisons de ceux-ci,
hétéroaryle ayant 5 à 10 atomes cycliques dans lequel au moins 1 atome cyclique est un hétéroatome, qui n'est pas substitué ou est substitué une ou plusieurs fois par halogène, alkyle, hydroxy, alcoxy, alcoxyalcoxy, nitro, méthylènedioxy, éthylènedioxy, trifluorométhyle, amino, aminométhyle, aminoalkyle, aminoalcoxy, dialkylamino, hydroxyalkyle, acide hydroxamique, tétrazole-5-yle, hydroxyalcoxy, carboxy, carboxyalkyle, alcoxycarbonyle, cyano, acyle, alkylthio, alkylsulfinyle, alkylsulfonyle, phénoxy, trialkylsilyloxy, R⁸-L ou des combinaisons de ceux-ci,
un groupe hétérocyclique, qui est saturé ou partiellement saturé, ayant 5 à 10 atomes cycliques dans lequel au moins 1 atome cyclique est un atome N, O ou S, qui n'est pas substitué ou est substitué une ou plusieurs fois par halogène, alkyle, hydroxy, alcoxy, alcoxyalcoxy, nitro, oxo, méthylènedioxy, éthylènedioxy, trifluorométhyle, OCF₃, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénylsulfonyle, phénoxy, cycloalkyle, aryle, hétéroaryle ou des combinaisons de ceux-ci,
un groupe hétérocycle-alkyle, dans lequel la partie hétérocyclique est saturée, partiellement saturée ou non saturée, et a 5 à 10 atomes cycliques dans lequel au moins 1 atome cyclique est un atome N, O ou S, et la partie d'alkyle est ramifiée ou non ramifiée et a 1 à 5 atomes de carbone, le groupe hétérocycle-alkyle n'est pas substitué, est substitué une ou plusieurs fois dans la partie hétérocyclique par halogène, alkyle, hydroxy, alcoxy, alcoxyalcoxy, nitro, oxo, méthylènedioxy, éthylènedioxy, trifluorométhyle, OCF₃, amino, aminométhyle, aminoalkyle, aminoalcoxy, dialkylamino, hydoxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénylsulfonyle, phénoxy, cycloalkyle, aryle, hétéroaryle ou des combinaisons de ceux-ci, et/ou substitué dans la partie d'alkyle une ou plusieurs fois par halogène, oxo, hydroxy, cyano ou des combinaisons de ceux-ci, et dans lequel dans la partie d'alkyle un ou plusieurs groupes -CH₂CH₂- sont chacun optionnellement remplacés par -CH=CH- ou - C=C-, et un ou plusieurs groupes -CH₂- sont chacun optionnellement remplacés par -O- ou -NH-;
R⁵ est H, halogène, alkyle ayant 1 à 4 atomes de carbone, alkyle halogéné ayant 1 à 4 atomes de carbone, alcoxy ayant 1 à 4 atomes de carbone ou alcoxy halogéné ayant 1 à 4 atomes de carbone;
R⁶ est H ou alkyle ayant 1 à 4 atomes de carbone, qui est ramifié ou non ramifié et qui n'est pas substitué ou est substitué une ou plusieurs fois par halogène;
R⁷ est H ou alkyle ayant 1 à 12 atomes de carbone, qui est ramifié ou non ramifié et qui n'est pas substitué ou est substitué une ou plusieurs fois par halogène, hydroxy, cyano, alcoxy C1-4, oxo ou des combinaisons de ceux-ci, et dans lequel optionnellement un ou plusieurs groupes -CH₂CH₂- sont remplacés dans chaque cas par -CH=CH- ou - C=C-,
cycloalkyle ayant 3 à 10 atomes de carbone, qui n'est pas substitué ou est substitué une ou plusieurs fois par halogène, hydroxy, oxo, cyano, alkyle ayant 1 à 4 atomes de carbone, alcoxy ayant 1 à 4 atomes de carbone ou des combinaisons de ceux-ci,
cycloalkylalkyle ayant 4 à 16 atomes de carbone, qui n'est pas substitué ou est substitué dans la partie de cycloalkyle et/ou la partie d'alkyle une ou plusieurs fois par halogène, oxo, cyano, hydroxy, alkyle C1-4, alcoxy C1-4 ou des combinaisons de ceux-ci,
aryle ayant 6 à 14 atomes de carbone qui n'est pas substitué ou est substitué une ou plusieurs fois par halogène, CF₃, OCF₃, alkyle, hydroxy, alcoxy, nitro, méthylènedioxy, éthylènedioxy, cyano ou des combinaisons de ceux-ci,
arylalkyle dans lequel la partie d'aryle a 6 à 14 atomes de carbone et la partie d'alkyle, qui est ramifiée ou non ramifiée, a 1 à 5 atomes de carbone, dans lequel le radical arylalkyle n'est pas substitué, est substitué dans la partie d'aryle une ou plusieurs fois par halogène, CF₃, OCF₃, alkyle, hydroxy, alcoxy, nitro, cyano, méthylènedioxy, éthylènedioxy ou des combinaisons de ceux-ci, et/ou substitué dans la partie d'alkyle une ou plusieurs fois par halogène, oxo, hydroxy, cyano ou des combinaisons de ceux-ci, et dans lequel dans la partie d'alkyle un ou plusieurs groupes -
CH₂CH₂- sont chacun optionnellement remplacés par -CH=CH- ou -C≡C-, et un ou plusieurs groupes -CH₂- sont chacun optionnellement remplacés par -O- ou - NH-,
un groupe carbocyclique partiellement non saturée ayant 5 à 14 atomes de carbone, qui n'est pas substitué ou est substitué une ou plusieurs fois par halogène, alkyle, alcoxy, hydroxy, nitro, cyano, oxo ou des combinaisons de ceux-ci,
un groupe hétérocyclique, qui est saturé, partiellement saturé ou non saturé, ayant 5 à 10 atomes cycliques dans lequel au moins 1 atome cyclique est un atome N, O ou S, qui n'est pas substitué ou est substitué une ou plusieurs fois par halogène, hydroxy, aryle, alkyle, alcoxy, cyano, trifluorométhyle, nitro, oxo ou des combinaisons de ceux-ci, ou
un groupe hétérocycle-alkyle, dans lequel la partie hétérocyclique est saturée, partiellement saturée ou non saturée, et a 5 à 10 atomes cycliques dans lequel au moins 1 atome cyclique est un atome N, O ou S, et la partie d'alkyle est ramifiée ou non ramifiée et a 1 à 5 atomes de carbone, le groupe hétérocycle-alkyle n'est pas substitué, est substitué une ou plusieurs fois dans la partie hétérocyclique par halogène, OCF₃, hydroxy, aryle, alkyle, alcoxy, cyano, trifluorométhyle, nitro, oxo ou des combinaisons de ceux-ci, et/ou substitué dans la partie d'alkyle une ou plusieurs fois par halogène, oxo, hydroxy, cyano ou des combinaisons de ceux-ci, et dans lequel dans la partie d'alkyle un ou plusieurs groupes -CH₂CH₂- sont chacun optionnellement remplacés par -CH=CH- ou - C=C-, et un ou plusieurs groupes -CH₂- sont chacun optionnellement remplacés par -O- ou -NH-;
R⁸ est H,
alkyle ayant 1 à 8 atomes de carbone, qui n'est pas substitué ou est substitué une ou plusieurs fois par halogène, alkyle C1-4, alcoxy C1-4, oxo ou des combinaisons de ceux-ci,
alkylamino ou dialkylamino dans lequel chaque partie d'alkyle a indépendamment 1 à 8 atomes de carbone,
un groupe carbocycle-alkyle partiellement non saturé dans lequel la partie carbocyclique a 5 à 14 atomes de carbone et la partie d'alkyle a 1 à 5 atomes de carbone, et qui n'est pas substitué ou est substitué une ou plusieurs fois par halogène, alkyle, alcoxy, nitro, cyano, oxo ou des combinaisons de ceux-ci,
cycloalkyle ayant 3 à 10 atomes de carbone, qui n'est pas substitué ou est substitué une ou plusieurs fois par halogène, hydroxy, oxo, cyano, alcoxy, alkyle ayant 1 à 4 atomes de carbone ou des combinaisons de ceux-ci,
cycloalkylalkyle ayant 4 à 16 atomes de carbone, qui n'est pas substitué ou est substitué dans la partie de cycloalkyle et/ou la partie d'alkyle une ou plusieurs fois par halogène, oxo, cyano, hydroxy, alkyle, alcoxy ou des combinaisons de ceux-ci,
aryle ayant 6 à 14 atomes de carbone qui n'est pas substitué ou est substitué une ou plusieurs fois par halogène, alkyle, hydroxy, alcoxy, alcoxyalcoxy, nitro, méthylènedioxy, éthylènedioxy, trifluorométhyle, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénoxy, cycloalkyle, aryle, hétéroaryle ou des combinaisons de ceux-ci,
arylalkyle ayant 7 à 19 atomes de carbone, dans lequel la partie d'aryle a 6 à 14 atomes de carbone et la partie d'alkyle, qui est ramifiée ou non ramifiée, a 1 à 5 atomes de carbone, dans lequel le radical arylalkyle n'est pas substitué ou est substitué, dans la partie d'aryle, une ou plusieurs fois par halogène, trifluorométhyle, CF₃O, nitro, amino, alkyle, alcoxy, amino, alkylamino, dialkylamino ou des combinaisons de ceux-ci, et/ou substitué dans la partie d'alkyle par halogène, cyano, alkyle ayant 1 à 4 atomes de carbone ou des combinaisons de ceux-ci, dans lequel dans la partie d'alkyle un ou plusieurs groupes -CH₂CH₂- sont chacun optionnellement remplacés par -CH=CH- ou - C=C-, et/ou un ou plusieurs groupes -CH₂- sont chacun optionnellement remplacés par -O- ou -NH-,
un groupe hétérocyclique, qui est saturé, partiellement saturé ou non saturé, ayant 5 à 10 atomes cycliques dans lequel au moins 1 atome cyclique est un atome N, O ou S, qui n'est pas substitué ou est substitué une ou plusieurs fois par halogène, alkyle, hydroxy, alcoxy, alcoxyalcoxy, nitro, méthylènedioxy, éthylènedioxy, trifluorométhyle, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénoxy, cycloalkyle, aryle, hétéroaryle ou des combinaisons de ceux-ci, ou
un groupe hétérocycle-alkyle, dans lequel la partie hétérocyclique est saturée, partiellement saturée ou non saturée, et a 5 à 10 atomes cycliques dans lequel au moins 1 atome cyclique est un atome N, O ou S, et la partie d'alkyle, qui est ramifiée ou non ramifiée, a 1 à 5 atomes de carbone, le groupe hétérocycle-alkyle n'est pas substitué, est substitué une ou plusieurs fois dans la partie hétérocyclique par halogène, alkyle, alcoxy, cyano, trifluorométhyle, CF₃O, nitro, oxo, amino, alkylamino, dialkylamino ou des combinaisons de ceux-ci et/ou substitué une ou plusieurs fois dans la partie d'alkyle par halogène, cyano, alkyle ayant 1 à 4 atomes de carbone ou des combinaisons de ceux-ci;
L est un liage simple ou un radical aliphatique divalent ayant 1 à 8 atomes de carbone dans lequel un ou plusieurs groupes -CH₂- sont chacun optionnellement remplacés par -O-, -S-, -SO-, -SO₂-, - NR⁹-, -SO₂NR⁹-, -NR⁹SO₂-, -CO-, -CO₂-, -NR⁹CO-, - CONR⁹-, -NHCONH-, -OCONH, -NHCOO-, -SCONH-, - SCSNH-, -NHCSNH-, -CONHSO₂- ou -SO₂NHCO-; et
R⁹ est H,
alkyle ayant 1 à 8 atomes de carbone, qui est ramifié ou non ramifié et qui n'est pas substitué ou est substitué une ou plusieurs fois par halogène, alkyle C1-4, alcoxy C1-4, oxo ou des combinaisons de ceux-ci,
arylalkyle ayant 7 à 19 atomes de carbone, dans lequel la partie d'aryle a 6 à 14 atomes de carbone et la partie d'alkyle, qui est ramifiée ou non ramifiée, a 1 à 5 atomes de carbone, dans lequel le radical arylalkyle n'est pas substitué ou est substitué, dans la partie d'aryle, une ou plusieurs fois par halogène, trifluorométhyle, CF₃O, nitro, amino, alkyle, alcoxy, amino, alkylamino, dialkylamino ou des combinaisons de ceux-ci, et/ou substitué dans la partie d'alkyle par halogène, cyano, alkyle ayant 1 à 4 atomes de carbone ou des combinaisons de ceux-ci, dans lequel dans la partie d'alkyle un ou plusieurs groupes -CH₂CH₂- sont chacun optionnellement remplacés par -CH=CH- ou - C=C-, et/ou un ou plusieurs groupes -CH₂- sont chacun optionnellement remplacés par -O- ou -NH-, ou
aryle ayant 6 à 14 atomes de carbone et qui n'est pas substitué ou est substitué une ou plusieurs fois par halogène, alkyle, hydroxy, alcoxy, alcoxyalcoxy, nitro, méthylènedioxy, éthylènedioxy, trifluorométhyle, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylethio, alkylsulfinyle, alkylsulfonyle ou des combinaisons de ceux-ci; et
R¹⁰ est H ou alkyle ayant 1 à 4 atomes de carbone, qui est ramifié ou non ramifié et qui n'est pas substitué ou est substitué une ou plusieurs fois par halogène; ou
un sel pharmaceutiquement acceptable de ceux-ci.

2. Composé selon la formule III: 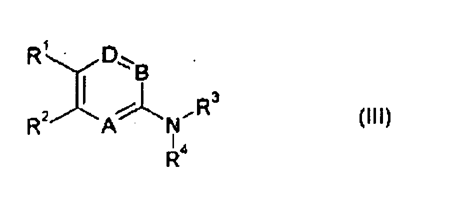 dans lequel
A, B et D sont chacun N ou CR⁵ et un d'A, B et D est N;
R¹ est halogène, alkyle ayant 1 à 4 atomes de carbone, alkyle halogéné ayant 1 à 4 atomes de carbone ou OR⁶;
R² est halogène, alkyle ayant 1 à 4 atomes de carbone, alkyle halogéné ayant 1 à 4 atomes de carbone ou OR⁷;
R³ est arylalkyle ayant 7 à 19 atomes de carbone, dans lequel la partie d'aryle a 6 à 14 atomes de carbone et la partie d'alkyle, qui est ramifiée ou non ramifiée, a 1 à 5 atomes de carbone, dans lequel le radical arylalkyle n'est pas substitué ou est substitué, dans la partie d'aryle, une ou plusieurs fois par halogène, trifluorométhyle, CF₃O, nitro, amino, alkyle, alcoxy, amino, alkylamino, dialkylamino ou des combinaisons de ceux-ci, et/ou substitué dans la partie d'alkyle par halogène, cyano, alkyle ayant 1 à 4 atomes de carbone ou des combinaisons de ceux-ci, dans lequel dans la partie d'alkyle un ou plusieurs groupes -CH₂CH₂- sont chacun optionnellement remplacés par -CH=CH- ou - C≡C-, et/ou un ou plusieurs groupes -CH₂- sont chacun optionnellement remplacés par -O- ou -NH-, ou
un groupe hétérocycle-alkyle, dans lequel la partie hétérocyclique n'est pas saturée, est partiellement saturée ou complètement saturée, et a 5 à 10 atomes cycliques dans lequel au moins 1 atome cyclique est un N, N-O, O ou S, la partie d'alkyle, qui est ramifiée ou non ramifiée, a 1 à 5 atomes de carbone, le groupe hétérocycle-alkyle n'est pas substitué, est substitué une ou plusieurs fois dans la partie hétérocyclique par halogène, alkyle, alcoxy, cyano, trifluorométhyle, CF₃O, nitro, oxo, amino, alkylamino, dialkylamino ou des combinaisons de ceux-ci, et/ou substitué dans la partie d'alkyle une ou plusieurs fois par halogène, cyano, alkyle ayant 1 à 4 atomes de carbone ou des combinaisons de ceux-ci;
R⁴ est cycloalkyle ayant 3 à 10 atomes de carbone, qui n'est pas substitué ou est substitué une ou plusieurs fois par halogène, hydroxy, oxo, cyano, alkyle ayant 1 à 4 atomes de carbone, alcoxy ayant 1 à 4 atomes de carbone ou des combinaisons de ceux-ci,
aryle ayant 6 à 14 atomes de carbone et qui n'est pas substitué ou est substitué une ou plusieurs fois par halogène, alkyle, alcényle, alkynyle, hydroxy, alcoxy, alcoxyalcoxy, nitro, méthylènedioxy, éthylènedioxy, trifluorométhyle, OCF₃, amino, aminoalkyle, aminoalcoxy, dialkylamino, hydroxyalkyle, acide hydroxamique, pyrrolyle, tétrazole-5-yle, 2(-hétérocycle)tétrazole-5-yle, hydroxyalcoxy, carboxy, alcoxycarbonyle, cyano, acyle, alkylthio, alkylsulfinyle, alkylsulfonyle, phénoxy, trialkylsilyloxy, R⁸-L ou des combinaisons de ceux-ci,
hétéroaryle ayant 5 à 10 atomes cycliques dans lequel au moins 1 atome cyclique est un hétéroatome, qui n'est pas substitué ou est substitué une ou plusieurs fois par halogène, alkyle, hydroxy, alcoxy, alcoxyalcoxy, nitro, méthylènedioxy, éthylènedioxy, trifluorométhyle, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénoxy, trialkylsilyloxy, R⁸-L ou des combinaisons de ceux-ci,
un groupe hétérocyclique, qui est saturé ou partiellement saturé, ayant 5 à 10 atomes cycliques dans lequel au moins 1 atome cyclique est un atome N, 0 ou S, qui n'est pas substitué ou est substitué une ou plusieurs fois par halogène, alkyle, hydroxy, alcoxy, alcoxyalcoxy, nitro, oxo, méthylènedioxy, éthylènedioxy, trifluorométhyle, OCF₃,
amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénylsulfonyle, phénoxy, cycloalkyle, aryle, hétéroaryle ou des combinaisons de ceux-ci,
un groupe hétérocycle-alkyle, dans lequel la partie hétérocyclique est saturée, partiellement saturée ou non saturée, et a 5 à 10 atomes cycliques dans lequel au moins 1 atome cyclique est un atome N, O ou S, et la partie d'alkyle est ramifiée ou non ramifiée et a 1 à 5 atomes de carbone, le groupe hétérocycle-alkyle n'est pas substitué, est substitué une ou plusieurs fois dans la partie hétérocyclique par halogène, alkyle, hydroxy, alcoxy, alcoxyalcoxy, nitro, oxo, méthylènedioxy, éthylènedioxy, trifluorométhyle, OCF₃, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénylsulfonyle, phénoxy, cycloalkyle, aryle, hétéroaryle ou des combinaisons de ceux-ci, et/ou substitué dans la partie d'alkyle une ou plusieurs fois par halogène, oxo, hydroxy, cyano ou des combinaisons de ceux-ci, et dans lequel dans la partie d'alkyle un ou plusieurs groupes - CH₂CH₂- sont chacun optionnellement remplacés par -CH=CH- ou -C≡C-, et un ou plusieurs groupes -CH₂- sont chacun optionnellement remplacés par -O- ou - NH;
R⁵ est H, halogène, alkyle ayant 1 à 4 atomes de carbone, alkyle halogéné ayant 1 à 4 atomes de carbone, alcoxy ayant 1 à 4 atomes de carbone ou alcoxy halogéné ayant 1 à 4 atomes de carbone;
R⁶ est H ou alkyle ayant 1 à 4 atomes de carbone, qui est ramifié ou non ramifié et qui n'est pas substitué ou est substitué une ou plusieurs fois par halogène;
R⁷ est H ou alkyle ayant 1 à 12 atomes de carbone, qui est ramifié ou non ramifié et qui n'est pas substitué ou est substitué une ou plusieurs fois par halogène, hydroxy, cyano, alcoxy C1-4, oxo ou des combinaisons de ceux-ci, et dans lequel optionnellement un ou plusieurs groupes -CH₂CH₂- sont remplacés dans chaque cas par -CH=CH- ou - C≡C-,
cycloalkyle ayant 3 à 10 atomes de carbone, qui n'est pas substitué ou est substitué une ou plusieurs fois par halogène, hydroxy, oxo, cyano, alkyle ayant 1 à 4 atomes de carbone, alcoxy ayant 1 à 4 atomes de carbone ou des combinaisons de ceux-ci,
cycloalkylalkyle ayant 4 à 16 atomes de carbone, qui n'est pas substitué ou est substitué dans la partie de cycloalkyle et/ou la partie d'alkyle une ou plusieurs fois par halogène, oxo, cyano, hydroxy, alkyle C1-4, alcoxy C1-4 ou des combinaisons de ceux-ci,
aryle ayant 6 à 14 atomes de carbone qui n'est pas substitué ou est substitué une ou plusieurs fois par halogène, CF₃, OCF₃, alkyle, hydroxy, alcoxy, nitro, méthylènedioxy, éthylènedioxy, cyano ou des combinaisons de ceux-ci,
arylalkyle dans lequel la partie d'aryle a 6 à 14 atomes de carbone et la partie d'alkyle, qui est ramifiée ou non ramifiée, a 1 à 5 atomes de carbone, dans lequel le radical arylalkyle n'est pas substitué, est substitué dans la partie d'aryle une ou plusieurs fois par halogène, CF₃, OCF₃, alkyle, hydroxy, alcoxy, nitro, cyano, méthylènedioxy, éthylènedioxy ou des combinaisons de ceux-ci, et/ou substitué dans la partie d'alkyle une ou plusieurs fois par halogène, oxo, hydroxy, cyano ou des combinaisons de ceux-ci, et dans lequel dans la partie d'alkyle un ou plusieurs groupes - CH₂CH₂- sont chacun optionnellement remplacés par -CH=CH- ou -C≡C-, et un ou plusieurs groupes -CH₂- sont chacun optionnellement remplacés par -O- ou - NH-,
un groupe carbocyclique partiellement non saturée ayant 5 à 14 atomes de carbone, qui n'est pas substitué ou est substitué une ou plusieurs fois par halogène, alkyle, alcoxy, hydroxy, nitro, cyano, oxo ou des combinaisons de ceux-ci,
un groupe hétérocyclique, qui est saturé, partiellement saturé ou non saturé, ayant 5 à 10 atomes cycliques dans lequel au moins 1 atome cyclique est un atome N, O ou S, qui n'est pas substitué ou est substitué une ou plusieurs fois par halogène, hydroxy, aryle, alkyle, alcoxy, cyano, trifluorométhyle, nitro, oxo ou des combinaisons de ceux-ci, ou
un groupe hétérocycle-alkyle, dans lequel la partie hétérocyclique est saturée, partiellement saturée ou non saturée, et a 5 à 10 atomes cycliques dans lequel au moins 1 atome cyclique est un atome N, O ou S, et la partie d'alkyle est ramifiée ou non ramifiée et a 1 à 5 atomes de carbone, le groupe hétérocycle-alkyle n'est pas substitué, est substitué une ou plusieurs fois dans la partie hétérocyclique par halogène, OCF₃, hydroxy, aryle, alkyle, alcoxy, cyano, trifluorométhyle, nitro, oxo ou des combinaisons de ceux-ci, et/ou substitué dans la partie d'alkyle une ou plusieurs fois par halogène, oxo, hydroxy, cyano ou des combinaisons de ceux-ci, et dans lequel dans la partie d'alkyle un ou plusieurs groupes -CH₂CH₂- sont chacun optionnellement remplacés par -CH=CH- ou - C=C-, et un ou plusieurs groupes -CH₂- sont chacun optionnellement remplacés par -O- ou -NH-;
R⁸ est H,
alkyle ayant 1 à 8 atomes de carbone, qui n'est pas substitué ou est substitué une ou plusieurs fois par halogène, alkyle C1-4, alcoxy C1-4, oxo ou des combinaisons de ceux-ci,
alkylamino ou dialkylamino dans lequel chaque partie d'alkyle a indépendamment 1 à 8 atomes de carbone,
un groupe carbocycle-alkyle partiellement non saturé dans lequel la partie carbocyclique a 5 à 14 atomes de carbone et la partie d'alkyle a 1 à 5 atomes de carbone, et qui n'est pas substitué ou est substitué une ou plusieurs fois par halogène, alkyle, alcoxy, nitro, cyano, oxo ou des combinaisons de ceux-ci,
cycloalkyle ayant 3 à 10 atomes de carbone, qui n'est pas substitué ou est substitué une ou plusieurs fois par halogène, hydroxy, oxo, cyano, alcoxy, alkyle ayant 1 à 4 atomes de carbone ou des combinaisons de ceux-ci,
cycloalkylalkyle ayant 4 à 16 atomes de carbone, qui n'est pas substitué ou est substitué dans la partie de cycloalkyle et/ou la partie d'alkyle une ou plusieurs fois par halogène, oxo, cyano, hydroxy, alkyle, alcoxy ou des combinaisons de ceux-ci,
aryle ayant 6 à 14 atomes de carbone qui n'est pas substitué ou est substitué une ou plusieurs fois par halogène, alkyle, hydroxy, alcoxy, alcoxyalcoxy, nitro, méthylènedioxy, éthylènedioxy, trifluorométhyle, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénoxy, cycloalkyle, aryle, hétéroaryle ou des combinaisons de ceux-ci,
arylalkyle ayant 7 à 19 atomes de carbone, dans lequel la partie d'aryle a 6 à 14 atomes de carbone et la partie d'alkyle, qui est ramifiée ou non ramifiée, a 1 à 5 atomes de carbone, dans lequel le radical arylalkyle n'est pas substitué ou est substitué, dans la partie d'aryle, une ou plusieurs fois par halogène, trifluorométhyle, CF₃O, nitro, amino, alkyle, alcoxy, amino, alkylamino, dialkylamino ou des combinaisons de ceux-ci, et/ou substitué dans la partie d'alkyle par halogène, cyano, alkyle ayant 1 à 4 atomes de carbone ou des combinaisons de ceux-ci, dans lequel dans la partie d'alkyle un ou plusieurs groupes -CH₂CH₂- sont chacun optionnellement remplacés par -CH=CH- ou - C=C-, et/ou un ou plusieurs groupes -CH₂- sont chacun optionnellement remplacés par -O- ou -NH-,
un groupe hétérocyclique, qui est saturé, partiellement saturé ou non saturé, ayant 5 à 10 atomes cycliques dans lequel au moins 1 atome cyclique est un atome N, O ou S, qui n'est pas substitué ou est substitué une ou plusieurs fois par halogène, alkyle, hydroxy, alcoxy, alcoxyalcoxy, nitro, méthylènedioxy, éthylènedioxy, trifluorométhyle, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle, phénoxy, cycloalkyle, aryle, hétéroaryle ou des combinaisons de ceux-ci, ou
un groupe hétérocycle-alkyle, dans lequel la partie hétérocyclique est saturée, partiellement saturée ou non saturée, et a 5 à 10 atomes cycliques dans lequel au moins 1 atome cyclique est un atome N, O ou S, et la partie d'alkyle, qui est ramifiée ou non ramifiée, a 1 à 5 atomes de carbone, le groupe hétérocycle-alkyle n'est pas substitué, est substitué une ou plusieurs fois dans la partie hétérocyclique par halogène, alkyle, alcoxy, cyano, trifluorométhyle, CF₃O, nitro, oxo, amino, alkylamino, dialkylamino ou des combinaisons de ceux-ci et/ou substitué une ou plusieurs fois dans la partie d'alkyle par halogène, cyano, alkyle ayant 1 à 4 atomes de carbone ou des combinaisons de ceux-ci;
L est un liage simple ou un radical aliphatique divalent ayant 1 à 8 atomes de carbone dans lequel un ou plusieurs groupes -CH₂- sont chacun optionnellement remplacés par -O-, -S-, -SO-, -SO₂-, - NR⁹-, -SO₂NR⁹-, -NR⁹SO₂-, -CO-, -CO₂-, -NR⁹CO-, - CONR⁹-, -NHCONH-, -OCONH, -NHCOO-, -SCONH-, - SCSNH-, -NHCSNH-, -CONHSO₂- ou -SO₂NHCO-; et
R⁹ est H,
alkyle ayant 1 à 8 atomes de carbone, qui est ramifié ou non ramifié et qui n'est pas substitué ou est substitué une ou plusieurs fois par halogène, alkyle C1-4, alcoxy C1-4, oxo ou des combinaisons de ceux-ci,
arylalkyle ayant 7 à 19 atomes de carbone, dans lequel la partie d'aryle a 6 à 14 atomes de carbone et la partie d'alkyle, qui est ramifiée ou non ramifiée, a 1 à 5 atomes de carbone, dans lequel le radical arylalkyle n'est pas substitué ou est substitué, dans la partie d'aryle, une ou plusieurs fois par halogène, trifluorométhyle, CF₃O, nitro, amino, alkyle, alcoxy, amino, alkylamino, dialkylamino ou des combinaisons de ceux-ci, et/ou substitué dans la partie d'alkyle par halogène, cyano, alkyle ayant 1 à 4 atomes de carbone ou des combinaisons de ceux-ci, dans lequel dans la partie d'alkyle un ou plusieurs groupes -CH₂CH₂- sont chacun optionnellement remplacés par -CH=CH- ou - C=C-, et/ou un ou plusieurs groupes -CH₂- sont chacun optionnellement remplacés par -O- ou -NH-, ou
aryle ayant 6 à 14 atomes de carbone et qui n'est pas substitué ou est substitué une ou plusieurs fois par halogène, alkyle, hydroxy, alcoxy, alcoxyalcoxy, nitro, méthylènedioxy, éthylènedioxy, trifluorométhyle, amino, aminométhyle, aminoalkyle, aminoalcoxy, dialkylamino, hydroxyalkyle, acide hydroxamique, tétrazole-5-yle, hydroxyalcoxy, carboxy, alcoxycarbonyle, cyano, acyle, alkylthio, alkylsulfinyle, alkylsulfonyle ou des combinaisons de ceux-ci; et
dans lequel R¹ est OR⁶ et/ou R² est OR⁷;
ou un sel pharmaceutiquement acceptable de ceux-ci.

3. Composé selon la revendication 1, dans lequel R¹ est OR⁶ et/ou R² est OR⁷.

4. Composé selon la revendication 1, dans lequel R¹ et/ou R² est COR⁶, CONR⁶ ou NR⁶COR¹⁰.

5. Composé selon la revendication 1 ou 2,
dans lequel R¹ est halogène ou OR⁶ et R⁶ est alkyle ou alkyle halogéné, ou
dans lequel R² est halogène ou OR⁷, et R⁷ est alkyle, cycloalkyle, cycloalkylalkyle, un groupe hétérocyclique ou alkyle halogéné.

6. Composé selon la revendication 1, dans lequel R³ est arylalkyle ou hétérocycle-alkyle, qui dans chaque cas est substitué ou non substitué.

7. Composé selon la revendication 1 ou 2, dans lequel R³ est benzyle ou pyridylméthyle, qui dans chaque cas est substitué ou non substitué.

8. Composé selon la revendication 1 ou 2, dans lequel R⁴ est cycloalkyle, aryle, hétéroaryle ou un groupe hétérocyclique, qui est substitué ou non substitué.

9. Composé selon la revendication 8, dans lequel R⁴ est cycloalkyle, aryle ou un groupe hétérocyclique, qui est substitué ou non substitué.

10. Composé selon la revendication 9,
dans lequel R⁴ est cyclohexyle, pipéridinyle ou phényle, qui dans chaque cas est substitué ou non substitué, ou
dans lequel R⁴ est phényle substitué par carboxy, cyano, tétrazole et/ou L-R⁸.

11. Composé selon la revendication 1 ou 2,
dans lequel R⁴ est au moins monosubstitué par R⁸-L et L est un liage simple ou un radical aliphatique divalent ayant 1 à 8 atomes de carbone dans lequel au moins un groupe -CH₂- est remplacé par -SO₂NR⁹, -NR⁹-, -NR⁹CO-, -CONR⁹-, -CO₂-, -CONHSO₂-, - SO₂NHCO-, -SO₂- ou -NR⁹SO₂-_{,}
dans lequel R⁸ est méthyle, éthyle, propyle ou phényle, qui dans chaque cas n'est pas substitué ou est substitué,
dans lequel R9 est H, alkyle ayant 1 à 4 atomes de carbone ou aryle, ou
dans lequel R⁵ est H, F ou méthyle.

12. Composé selon la revendication 1 ou 2, dans lequel A est N, B et D sont chacun indépendamment CR⁵, R¹ est OR⁶, R² est halogène ou OR⁷, R³ est pyridylméthyle, fluorobenzyle ou 2,6-difluorobenzyle, R⁴ est aryle, cycloalkyle ou un groupe hétérocyclique saturé, dans chaque cas substitué ou non substitué, R⁵ est H, halogène ou alkyle qui est substitué ou non substitué, R⁶ est alkyle qui est substitué ou non substitué et R⁷ est alkyle, cycloalkyle, cycloalkylalkyle ou un groupe hétérocyclique saturé, dans chaque cas substitué ou non substitué.

13. Composé selon la revendication 1 ou 2, dans lequel A est N, B et D sont chacun indépendamment CR⁵, R¹ est OR⁶, R² est halogène ou OR⁷, R³ est pyridylméthyle, fluorobenzyle, 2,6-difluorobenzyle, 5-thiazolylméthyle ou 5-pyrimidinylméthyle, R⁴ est phényle, qui n'est pas substitué ou est substitué, R⁵ est H, halogène ou alkyle qui est substitué ou non substitué, R⁶ est alkyle qui est substitué ou non substitué et R⁷ est alkyle, cycloalkyle, cycloalkylalkyle ou un groupe hétérocyclique saturé, dans chaque cas substitué ou non substitué.

14. Composé selon la revendication 13, dans lequel R³ est pyridylméthyle, fluorobenzyle, 2,6-difluorobenzyle.

15. Composé selon la revendication 1 ou 2, dans lequel A est N, B et D sont chacun indépendamment CH, R¹ est OR⁶, R² est halogène ou OR⁷, R³ est pyridylméthyle, 5-thiazolylméthyle ou 5-pyrimidinylméthyle, R⁴ cycloalkyle non substitué, aryle qui est substitué ou non substitué ou pipéridinyle qui est substitué ou non substitué, R⁶ alkyle ou CHF₂ non substitué et R⁷ est alkyle, cycloalkyle, cycloalkylalkyle ou tétrahydrofuranyle, dans chaque cas substitué ou non substitué.

16. Composé selon la revendication 15, dans lequel R³ est pyridylméthyle.

17. Composé selon la revendication 1 ou 2, dans lequel A est N, B et D sont chacun indépendamment CH, R¹ est OR⁶, R² est halogène ou OR⁷, R³ est 3-pyridylméthyle, 5-thiazolylméthyle ou 5-pyrimidinylméthyle, R⁴ est cyclohexyle, phényle qui est substitué ou non substitué ou pipéridinyle qui est substitué ou non substitué, R⁶ alkyle ou CHF2 non substitué et R⁷ est alkyle, cycloalkyle, cycloalkylalkyle ou tétrahydrofuranyle, dans chaque cas substitué ou non substitué.

18. Composé selon la revendication 17, dans lequel R³ est 3-pyridylméthyle.

19. Composé selon la revendication 1 ou 2, dans lequel A est N et B et D sont chacun indépendamment CR⁵, R¹ est OR⁶, R² est OR⁷, R³ est hétérocycle-alkyle, R⁴ est un groupe hétérocyclique, qui n'est pas substitué ou est substitué, R⁵ est H, halogène ou alkyle qui est substitué ou non substitué, R⁶ est alkyle, R⁷ est alkyle, cycloalkyle ou cycloalkylalkyle, dans chaque cas substitué ou non substitué.

20. Composé choisi à partir du suivant:
4-{N-[4-méthoxy-3-(R)-(tétrahydrofurane-3-yloxy)-phényle]pyridine-3-yleméthylamino}pipéridine-1-acide carboxylique-tert-butyl-ester,
3-[N-(6-cyclopropyleméthoxy-5-méthoxypyridine-2-yle)-pyridine-3-yleméthylamino]-acide benzoïque, 3-[N-(5,6-diméthoxypyridine-2-yl)-pyridine-3-yle-méthylamino]-acide benzoïque,
3-[N-(6-cyclobutyloxy-5-méthoxypyridine-2-yle)-pyridine-3-yleméthylamino]-acide benzoïque,
3-[N-(6-cyclopropyleméthoxy-5-difluorométhoxypyridine-2-yle)-pyridine-3-ylméthylamino]-acide benzoïque,
3-[N-(5-difluorométhoxy-6-méthoxypyridine-2-yle)-pyridine-3-ylméthylamino]-acide benzoïque,
3-[N-(6-éthoxy-5-méthoxypyridine-2-yle)-pyridine-3-yleméthylamino]-acide benzoïque,
3-[N-(6-isopropoxy-5-méthoxypyridine-2-yle)-pyridine-3-yleméthylamino]-acide benzoïque,
3-[N-(5-difluorométhoxy-6-isopropoxypyridine-2-yle)-pyridine-3-yleméthylamino]-acide benzoïque,
3-[N-(6-cyclobutyloxy-5-difluorométhoxypyridine-2-yle)-pyridine-3-yleméthylamino]-acide benzoïque, N-(1-benzènesulfonylepipéridine-3-yle)-N-[5-méthoxy-6-(R)-(tétrahydrofurane-3-yleoxy)-pyridine-2-yle]-pyridine-3-yleméthyleamine,
N-(1-méthanesulfonylepipéridine-3-yle)-N-[5-méthoxy-6-(R)-(tétrahydrofurane-3-yleoxy)-pyridine-2-yle]-pyridine-3-yleméthyleamine,
N-[5-méthoxy-6-(R)-(tétrahydrofurane-3-yleoxy)-pyridine-2-yle]-N-pipéridine-3-yle-pyridine-3-yleméthyleamine,
N-[5-méthoxy-6-(R)-(tétrahydrofurane-3-yleoxy)-pyridine-2-yle]-N-pipéridine-4-yleméthyle-pyridine-3-yleméthyleamine,
4-(N-{[5-méthoxy-6-(R)-(tétrahydrofurane-3-yle-oxy)-pyridine-2-yle]-pyridine-3-yleméthylamino}-méthyle)-N-pipéridine-1-acide carboxylique-tert-butyl-ester
N-(1-benzènesulfonylepipéridine-4-yle)-N-[5-méthoxy-6-(R)-(tétrahydrofurane-3-yleoxy)-pyridine-2-yle]-pyridine-3-yleméthyleamine
1-(4-{N-[5-méthoxy-6-(R)-(tétrahydrofurane-3-yle-oxy)-pyridine-2-yle]-pyridine-3-yleméthylamino}-N-pipéridine-1-yle)-éthanone
N-[5-méthoxy-6-(R)-(tétrahydrofurane-3-yleoxy)-pyridine-2-yle]-pipéridine-4-yle-pyridine-3-yleméthyleamine
4-{N-[5-méthoxy-6-(R)-(tétrahydrofurane-3-yleoxy)-pyridine-2-yle]-pyridine-3-yleméthylamino}-pipéridine-1-acide carboxylique-tert-butyl-ester 3-{N-[5-méthoxy-6-(R)-(tétrahydrofurane-3-yleoxy)-pyridine-2-yle]-pyridine-3-yleméthylamino}-acide benzoïque
N-[5-méthoxy-6-(R)-(tétrahydrofurane-3-yleoxy)-pyridine-2-yle]-pyridine-3-yleméthyle-N-[4-(2H-tétrazol-5-yle)-phényle]-amine
N-cyclohexyl-N-[5-méthoxy-6-(R)-(tétrahydrofurane-3-yleoxy)-pyridine-2-yle]-pyridine-3-yleméthyleamine
N-[5-méthoxy-6-(R)-(tétrahydrofurane-3-yleoxy)-pyridine-2-yle]-N-phényle-pipéridine-3-yleméthyleamine
N-(3-Chlorophényle)-N-[5-méthoxy-6-(R)-(tétrahydrofurane-3-yleoxy)-pyridine-2-yle]-pyridine-3-yleméthyleamine
3-{N-[5-méthoxy-6-(tétrahydrofurane-3-yleoxy)-pyridine-2-yle]-pyridine-3-yleméthylamino}-acide benzoïque,
3-[N-(6-cyclopentyloxy-5-méthoxypyridine-2-yle)-pyridine-3-yleméthylamino]-acide benzoïque,
4-[N-(5-cyclopentyloxy-4-méthoxy-2-méthylephényle) pyridine-3-yleméthylamino]-acide benzoïque,
3-[N-(5-cyclopentyloxy-4-méthoxy-2-méthylephényle)pyridine-3-yleméthylamino]-acide benzoïque, et
et des sels pharmaceutiquement acceptables de ceux-ci,
dans lequel des composés, qui sont optiquement actifs, peuvent être en forme de leurs énantiomères séparés ou de mélanges de ceux-ci, inclusivement des mélanges racémiques.

21. Composé choisi à partir du suivant:
(6-cyclopentyloxy-5-méthoxy-pyridine-2-yle)-pipéridine-4-yle-pyridine-3-yleméthyle-amine hydro-chlorure,
(6-cyclopentyloxy-5-méthoxy-pyridine-2-yle)-pipéridine-4-yle-pyridine-4-yleméthyle-amine,
(6-cyclopropyleméthoxy-5-méthoxy-pyridine-2-yle)-pipéridine-4-yle-pyridine-3-yleméthyle-amine, {4-[(6-cyclopentyloxy-5-méthoxy-pyridine-2-yle)-pyridine-3-yleméthyle-amino]-pipéridine-1-yle}-(4-fluoro-phényle)-méthanone,
(6-cyclopentyloxy-5-méthoxy-pyridine-2-yle)-(1-méthanesulfonyle-pipéridine-4-yle)-pyridine-3-yleméthyle-amine,
6-(cyclopentyloxy)-5-méthoxy-N-pipéridine-3-yle-N-(pyridine-3-yleméthyle) pyridine-2-amine,
6-(cyclopentyloxy)-5-méthoxy-N-pipéridine-3-yle-N-(pyridine-3-yleméthyle)pyridine-2-amine oxalate,
6-isopropoxy-5-méthoxy-N-pipéridine-4-yle-N-(pyridine-3-yleméthyle)pyridine-2-amine,
6-isopropoxy-5-méthoxy-N-pipéridine-4-yle-N-(pyridine-3-yleméthyle)pyxidine-2-amine oxalate,
6-(cyclopropyleméthoxy)-5-(difluorométhoxy)-N-pipéridine-4-yle-N-(pyridine-3-yleméthyle)pyridine-2-amine,
6-(cyclopropyleméthoxy)-5-(difluorométhoxy)-N-pipéridine-4-yle-N-(pyridine-3-yleméthyle)pyridine-2-amine trifluoroacétate,
6-(cyclopentyloxy)-5-méthoxy-N-phényle-N-pipéridine-4-ylepyridine-2-amine,
6-(cyclopentyloxy)-5-méthoxy-N-pipéridine-4-yle-N-(pyrimidin-5-yleméthyle)pyridine-2-amine,
et des sels pharmaceutiquement acceptables de ceux-ci,
dans lequel des composés, qui sont optiquement actifs, peuvent être en forme de leurs énantiomères séparés ou de mélanges de ceux-ci, inclusivement des mélanges racémiques.

22. Composition pharmaceutique comprenant un composé selon une des revendications 1 à 21, et un agent porteur pharmaceutiquement acceptable.

23. Composition selon la revendication 22, dans lequel cette composition en outre comprend un agent pharmaceutique additionnel choisi à partir de bloqueurs de canaux calciques, médicaments cholinergiques, modulateurs récepteurs à l'adénosine, ampakines, modulateurs NMDA-R, modulateurs mGluR, inhibiteurs de cholinestérase ou des combinaisons de ceux-ci.

24. Composition selon la revendication 23, dans lequel cet agent pharmaceutique additionnel est donepezil.

25. Utilisation d'un composé selon une des revendications 1 à 21 pour la production d'un médicament pour augmenter la cognition dans un patient dans lequel une telle augmentation est désirée.

26. Utilisation d'un composé selon une des revendications 1 à 21 pour la production d'un médicament pour le traitement d'un patient souffrant de déficit cognitif ou troubles de cognition.

27. Utilisation selon la revendication 26, dans lequel ce patient est un humain souffrant de déficit de mémoire.

28. Utilisation selon la revendication 27, dans lequel ce patient souffre de déficit de mémoire à cause de maladie d'Alzheimer, schizophrénie, maladie de Parkinson, maladie de Huntington, maladie de Pick, maladie de Creutzfeldt-Jakob, dépression, vieillissement, trauma de la tête, apoplexie, hypoxie CNS, sénilité cérébrale, démence de multi-infarctus, VIH ou maladie cardiovasculaire.

29. Utilisation d'un composé selon une des revendications 1 à 21 pour la production d'un médicament pour le traitement d'un patient ayant une maladie avec des niveaux cAMP réduits.

30. Utilisation d'un composé selon une des revendications 1 à 21 pour la production d'un médicament pour inhiber l'activité d'enzyme PDE4 dans un patient.

31. Utilisation selon la revendication 27, dans laquelle ce patient souffre de déficit de mémoire à cause d'une maladie neurodégénérative ou à cause de troubles neurodégénératifs aigus.

32. Utilisation d'un composé selon une des revendications 1 à 21 pour la production d'un médicament pour le traitement d'un patient souffrant d'une maladie allergique ou inflammatoire.

33. Utilisation d'un composé selon une des revendications 1 à 21 pour la production d'un médicament pour le traitement d'un patient souffrant de schizophrénie, dépression bipolaire ou maniaque, dépression majeure, addiction aux médicaments et/ou dépendance de la morphine.

34. Utilisation d'un composé selon une des revendications 1 à 21 pour la production d'un médicament pour le traitement d'un patient souffrant de psychose **caractérisée par** des niveaux augmentés de PDE-4, dans laquelle cette psychose est une forme de dépression.
